# EUROPEAN PATENT SPECIFICATION

(11) **EP 4 419 181 B1**
(45) Date of publication and mention of the grant of the patent: **25.11.2026**
(21) Application number: 22883089.9
(22) Date of filing: 21.10.2022
(51) Int. Cl.: A61M 16/06, A61M 16/00, A61M 16/08, A61M 16/10, A61M 16/16, A61M 16/20

(54) **PATIENT INTERFACE**
PATIENTENSCHNITTSTELLE
INTERFACE PATIENT

(30) Priority: 22.10.2021 US 202163270955 P; 08.06.2022 US 202263366031 P; 28.09.2022 US 202263377425 P
(43) Date of publication of application: 28.08.2024
(73) Proprietor: Fisher & Paykel Healthcare Limited, Auckland, 2013 (NZ)
(72) Inventor: VAN SCHALKWYK, Andre, Auckland, 2013 (NZ); O'DONNELL, Kevin Peter, Auckland, 2013 (NZ); RADEMEYER, Ivan Justus, Auckland, 2013 (NZ); GARCIA, Enrico Alvarez, Auckland, 2013 (NZ)
(74) Representative: Forresters IP LLP
(86) International application number: PCT/IB2022/060117
(87) International publication number: WO 2023/067558

(56) References cited:
- WO-A1-2015/192186
- WO-A1-2017/042717
- WO-A1-2020/053220
- WO-A1-2020/053220
- WO-A1-2021/012005
- US-A1- 2010 252 037
- US-A1- 2014 158 127
- US-A1- 2016 158 476
- US-A1- 2016 158 476
- US-A1- 2019 328 991
- US-B2- 6 679 265
- US-B2- 8 136 525
- US-B2- 8 136 525
- US-B2- 8 783 257
- US-B2- 8 997 747

## Description

### TECHNICAL FIELD

The present disclosure generally relates to a patient interface for delivering breathing gases to airways of a patient.

### BACKGROUND

Humidifiers are used to provide humidified respiratory gases to a patient. Gases are delivered to the patient via a patient interface. Examples of a patient interface include an oral mask, a nasal mask, a nasal cannula, a combination of oral and nasal mask, and the like.

Patient interfaces comprising nasal interfaces can be used to deliver a flow of gases to a patient. Nasal delivery elements are inserted into the nose of a patient to deliver the required therapy. The nasal delivery elements may be required to seal or semi-seal at the nose, or may not be required to seal at the nose, to deliver the therapy.

US 8,136,525 discloses a mask system for use between a patient and a device to deliver a breathable gas to the patient.

### SUMMARY

The present invention provides a nasal cannula as claimed in claim 1. Preferred features are provided in dependent claims.

Embodiments not falling within the scope of the appended claims are provided to assist in understanding the invention.

A respiratory interface and respiratory therapy system are disclosed that may use nasal flow, e.g. through nasal delivery elements, in a nasal interface to deliver respiratory gases to a patient via an asymmetrical flow. Asymmetrical flow can provide the patient with increased dead space clearance in the upper airways. Due to a decrease in peak expiratory pressure, noise can be reduced.

In accordance with certain features, aspects and advantages of at least one of the embodiments disclosed herein, a nasal interface is disclosed, the nasal interface comprising:
a first nasal delivery element and a second nasal delivery element, wherein the first nasal delivery element and the second nasal delivery element are each configured to seal with a respective naris of a patient,
and a gases manifold comprising a gases inlet for delivery of respiratory gases to the gases manifold,
wherein the first nasal delivery element and the second nasal delivery element are in fluid communication with the gases inlet via the gases manifold, wherein the first nasal delivery element is proximal to the gases inlet and the second nasal delivery element is distal from the gases inlet,
wherein the nasal interface comprises a bypass restriction to provide a pressure drop through the nasal interface between the first nasal delivery element and the second nasal delivery element when gases are delivered from the gases inlet to the first nasal delivery element and the second nasal delivery element such that pressure at the first nasal delivery element is higher than pressure at the second nasal delivery element,
and a bias flow restriction for a flow of gases out of the nasal interface.

In some configurations, the pressure drop through the gases manifold is such that when there is a flow of gases from the gases inlet to the first nasal delivery element and the second nasal delivery element, the flow of gases from the gases inlet to the first nasal delivery element is greater than the flow of gases from the gases inlet to the second nasal delivery element.

In some configurations, the nasal interface comprises a gases flow channel in the gases manifold, wherein the bypass restriction provides a reduced cross-sectional area of a portion of the gases flow channel.

In some configurations, the portion of the gases flow channel is between the first nasal delivery element and the second nasal delivery element and/or is adjacent the second nasal delivery element.

In some configurations, the bypass restriction comprises at least one protrusion extending into the gases flow channel, optionally wherein the bypass restriction comprises a plurality of protrusions extending into the gases flow channel.

In some configurations, the gases manifold comprises a proximal bypass protrusion that is proximal to the nasal delivery elements and/or a distal bypass protrusion that is distal from the nasal delivery elements.

In some configurations, the gases manifold comprises both a proximal bypass protrusion and a distal bypass protrusion which in combination define a predetermined bypass dimension for the restricted flow of gases through the gases manifold between the first nasal delivery element and the second nasal delivery element.

In some configurations, the bypass restriction comprises an angled leading edge and an angled trailing edge that define a converging and diverging bypass restriction in a direction of gases flow through the gases manifold from the first nasal delivery element to the second nasal delivery element.

In some configurations, the bias flow restriction comprises at least one aperture for the flow of gases from the nasal interface to an ambient environment, optionally wherein the bias flow restriction comprises a plurality of apertures for the flow of gases from the nasal interface to an ambient environment.

In some configurations, the bias flow restriction comprises a filter or a diffuser to filter or diffuse gases flowing through the aperture(s).

In some configurations, the nasal interface comprises a filter unit between the gases manifold and the bias flow restriction.

In some configurations, the bias flow restriction is in fluid communication with the gases manifold, optionally wherein the gases manifold comprises the bias flow restriction or is coupled to the bias flow restriction, optionally wherein the bias flow restriction is in fluid communication with the gases manifold but is positioned remotely from the gases manifold.

In some configurations, the gases inlet is in fluid communication with a respiratory conduit.

In some configurations, the respiratory conduit has an internal diameter of between about 12 mm and about 23 mm, optionally more than about 12 mm and up to about 23 mm, optionally more than 12 mm and up to about 22 mm, optionally more than about 12 mm and up to about 21 mm, optionally more than about 12 mm and up to about 20 mm, optionally more than about 12 mm and up to about 19 mm, optionally more than about 12 mm and up to about 18 mm, optionally between about 13 mm and about 17 mm, optionally between about 14 mm and about 16 mm, optionally about 12 mm, optionally about 13 mm, optionally about 14 mm, optionally about 15 mm, optionally about 16 mm, optionally about 17 mm, optionally about 18 mm, optionally about 19 mm, optionally about 20 mm, optionally about 21 mm, optionally about 22 mm, optionally about 23 mm, or optionally any value between any two of those values.

In some configurations, the gases manifold comprises sealing flanges or collars for engagement with the first and second nasal delivery elements.

In some configurations, the bypass restriction comprises an insert for attachment to the gases manifold.

In some configurations, the first and second nasal delivery elements are attached to or integral with a base portion of an interface body.

In some configurations, the base portion is arranged to locate between a patient's face and the gases manifold in use.

In some configurations, the interface body comprises two side arms that extend laterally from either side of the base portion.

In some configurations, the nasal interface comprises headgear with ends that connect to the side arms of the interface body.

In some configurations, the bypass restriction provides a cross-sectional area of a portion of a gases flow channel, and the cross-sectional area of the portion of the gases flow channel is more than 0 to about 1.5 times a combined cross-sectional area of the nasal delivery elements.

In accordance with certain features, aspects and advantages of at least one of the embodiments disclosed herein, a nasal interface is disclosed, the nasal interface comprising:
a first nasal delivery element and a second nasal delivery element, wherein the first nasal delivery element and the second nasal delivery element are each configured to seal with a respective naris of a patient,
and a gases manifold comprising a gases inlet for delivery of respiratory gases to the gases manifold,
wherein the first nasal delivery element and the second nasal delivery element are in fluid communication with the gases inlet via the gases manifold, wherein the first nasal delivery element is proximal to the gases inlet and the second nasal delivery element is distal from the gases inlet,
wherein the nasal interface is configured to create a pressure differential between the first nasal delivery element and the second nasal delivery element when gases are delivered from the gases inlet to both the first nasal delivery element and the second nasal delivery element such that pressure at the first nasal delivery element is higher than pressure at the second nasal delivery element.

In some configurations, the pressure differential is such that when there is a flow of gases from the gases inlet to the first nasal delivery element and the second nasal delivery element, the flow of gases from the gases inlet to the first nasal delivery element is greater than the flow of gases from the gases inlet to the second nasal delivery element.

In some configurations, the gases inlet is in fluid communication with a respiratory conduit.

In some configurations, the respiratory conduit has an internal diameter of between about 12 mm and about 23 mm, optionally between about 12 mm and about 22 mm, optionally between about 12 mm and about 21 mm, optionally between about 12 mm and about 20 mm, optionally between about 12 mm and about 19 mm, optionally between about 12 mm and about 18 mm, optionally between about 13 mm and about 17 mm, optionally between about 14 mm and about 16 mm, optionally about 12 mm, optionally about 13 mm, optionally about 14 mm, optionally about 15 mm, optionally about 16 mm, optionally about 17 mm, optionally about 18 mm, optionally about 19 mm, optionally about 20 mm, optionally about 21 mm, optionally about 22 mm, optionally about 23 mm, or optionally any value between any two of those values.

In some configurations, when gases are delivered from the gases inlet to both the first nasal delivery element and the second nasal delivery element, the pressure of gases flow at the second nasal delivery element is up to about 1 cmH2O less than the pressure of gases flow at the first nasal delivery element.

In some configurations, the nasal interface is configured such that the pressure differential of gases flow between the first nasal delivery element and the second nasal delivery element is higher during an inspiration phase than during an expiration phase.

In some configurations, the nasal interface is configured such that pressure at the first nasal delivery element is higher than pressure at the second nasal delivery element during both the inspiration phase and the expiration phase.

In some configurations, the nasal interface is configured to achieve a patient pressure at the first and second nasal delivery elements of between about 2 cmH2O and about 30 cmH2O in use, optionally between about 2 cmH2O and about 25 cmH2O in use, optionally between about 2 cmH2O and about 20 cmH2O in use, optionally between about 2 cmH2O and about 15 cmH2O in use, optionally between about 2 cmH2O and about 14 cmH2O in use, optionally between about 2 cmH2O and about 13 cmH2O in use, optionally between about 2 cmH2O and about 12 cmH2O in use, optionally between about 2 cmH2O and about 11 cmH2O in use, optionally between about 2 cmH2O and about 10 cmH2O in use.

In some configurations, the pressure differential between the first nasal delivery element and the second nasal delivery element is configured to provide an asymmetric flow through upper airways of a patient of at least about 1 liter per minute (lpm), optionally between about 1 lpm and about 5 lpm.

In some configurations, the asymmetric flow promotes clearing of CO₂ from anatomical dead space of the patient.

In some configurations, the nasal interface comprises a bypass restriction that provides a cross-sectional area of a portion of a gases flow channel, and the cross-sectional area of the portion of the gases flow channel is more than 0 to about 1.5 times a combined cross-sectional area of the nasal delivery elements.

In accordance with certain features, aspects and advantages of at least one of the embodiments disclosed herein, a nasal interface is disclosed, the nasal interface comprising:
an interface body part comprising a first nasal delivery element and a second nasal delivery element, wherein the first nasal delivery element and the second nasal delivery element are each configured to seal with a respective naris of a patient,
and a gases manifold part comprising a gases inlet for delivery of respiratory gases to the gases manifold part,
wherein the interface body part is engageable with the gases manifold part to bring the first nasal delivery element and the second nasal delivery element into fluid communication with the gases inlet such that the first nasal delivery element is proximal to the gases inlet and the second nasal delivery element is distal to the gases inlet, and
wherein the nasal interface comprises at least one gases flow restriction to gases flow through the nasal interface, such that when gases are delivered from the gases inlet to the first nasal delivery element and the second nasal delivery element, pressure at the first nasal delivery element is higher than pressure at the second nasal delivery element.

In some configurations, the at least one gases flow restriction comprises a bypass restriction to provide a pressure drop through the gases manifold part between the first nasal delivery element and the second nasal delivery element when gases are delivered from the gases inlet to the first nasal delivery element and the second nasal delivery element such that pressure at the first nasal delivery element is higher than pressure at the second nasal delivery element.

In some configuration, the bypass restriction provides a cross-sectional area of a portion of a gases flow channel, and the cross-sectional area of the portion of the gases flow channel is more than 0 to about 1.5 times a combined cross-sectional area of the nasal delivery elements.

In some configurations, the nasal interface comprises a gases flow channel in the gases manifold part, wherein the bypass restriction provides a reduced cross-sectional area of a portion of the gases flow channel.

In some configurations, the portion of the gases flow channel is between the first nasal delivery element and the second nasal delivery element and/or is adjacent the second nasal delivery element.

In some configurations, the bypass restriction comprises at least one protrusion extending into the gases flow channel, optionally wherein the bypass restriction comprises a plurality of protrusions extending into the gases flow channel.

In some configurations, the gases manifold part comprises a proximal bypass protrusion that is proximal to the nasal delivery elements and/or a distal bypass protrusion that is distal from the nasal delivery elements.

In some configurations, the gases manifold part comprises both a proximal bypass protrusion and a distal bypass protrusion which in combination define a predetermined bypass dimension for the restricted flow of gases through the gases manifold between the first nasal delivery element and the second nasal delivery element.

In some configurations, the bypass restriction comprises an angled leading edge and an angled trailing edge that define a converging and diverging bypass restriction in a direction of gases flow through the gases manifold from the first nasal delivery element to the second nasal delivery element.

In some configurations, the bypass restriction comprises an insert for attachment to the gases manifold part.

In some configurations, the nasal interface further comprises a bias flow restriction for a flow of gases out of the nasal interface.

In some configurations, the bias flow restriction comprises at least one aperture for the flow of gases from the nasal interface to an ambient environment, optionally wherein the bias flow restriction comprises a plurality of apertures for the flow of gases from the nasal interface to an ambient environment.

In some configurations, the bias flow restriction comprises a filter or a diffuser to filter or diffuse gases flowing through the aperture(s).

In some configurations, the nasal interface comprises a filter unit between the gases manifold part and the bias flow restriction.

In some configurations, the bias flow restriction is in fluid communication with the gases manifold part, optionally wherein the gases manifold comprises the bias flow restriction or is coupled to the bias flow restriction, optionally wherein the bias flow restriction is in fluid communication with the gases manifold part but is positioned remotely from the gases manifold part.

In some configurations, the gases inlet is in fluid communication with a respiratory conduit.

In some configurations, the respiratory conduit has an internal diameter of between about 12 mm and about 23 mm, optionally between about 12 mm and about 22 mm, optionally between about 12 mm and about 21 mm, optionally between about 12 mm and about 20 mm, optionally between about 12 mm and about 19 mm, optionally between about 12 mm and about 18 mm, optionally between about 13 mm and about 17 mm, optionally between about 14 mm and about 16 mm, optionally about 12 mm, optionally about 13 mm, optionally about 14 mm, optionally about 15 mm, optionally about 16 mm, optionally about 17 mm, optionally about 18 mm, optionally about 19 mm, optionally about 20 mm, optionally about 21 mm, optionally about 22 mm, optionally about 23 mm, or optionally any value between any two of those values.

In some configurations, the gases manifold part comprises sealing flanges or collars for engagement with the first and second nasal delivery elements when the interface body part is engaged with the gases manifold part.

In accordance with certain features, aspects and advantages of at least one of the embodiments disclosed herein, a nasal interface is disclosed, the nasal interface comprising:
a first nasal delivery element and a second nasal delivery element, wherein the first nasal delivery element and the second nasal delivery element are each configured to seal with a respective naris of a patient,
and a gases manifold comprising a gases inlet for delivery of respiratory gases to the gases manifold and a gases flow channel,
wherein the first nasal delivery element and the second nasal delivery element are in fluid communication with the gases inlet via the gases flow channel, wherein the first nasal delivery element is proximal to the gases inlet and the second nasal delivery element is distal from the gases inlet,
wherein the nasal interface comprises a bypass restriction that provides a cross-sectional area of a portion of the gases flow channel,
wherein each of the first nasal delivery element and the second nasal delivery element comprises an inner cross-sectional area, wherein the inner cross-sectional areas together provide a combined cross-sectional area of the nasal delivery elements, and wherein the cross-sectional area of the portion of the gases flow channel is more than 0 to about 1.5 times the combined cross-sectional area of the nasal delivery elements.

In some configurations, the cross-sectional area of the portion of the gases flow channel is up to about 1.3 times the combined cross-sectional area of the nasal delivery elements, optionally up to about 1 times the combined cross-sectional area of the nasal delivery elements, optionally up to about 2/3 of the combined cross-sectional area of the nasal delivery elements, optionally up to about 1/2 of the combined cross-sectional area of the nasal delivery elements, optionally up to about 2/5 of the combined cross-sectional area of the nasal delivery elements, optionally up to about 1/3 of the combined cross-sectional area of the nasal delivery elements.

In some configurations, the inner cross-sectional area of each of the first and second nasal delivery elements is at a smallest transverse dimension of the respective nasal delivery element.

In some configurations, the smallest transverse dimension is in a direction that is transverse to a direction of gases flow through the nasal delivery elements.

In some configurations, the inner cross-sectional area of each of the first and second nasal delivery elements is at an outlet of the respective nasal delivery element.

In some configurations, the portion of the gases flow channel is between the first nasal delivery element and the second nasal delivery element and/or is adjacent the second nasal delivery element.

In some configurations, the bypass restriction comprises at least one protrusion extending into the gases flow channel, optionally wherein the bypass restriction comprises a plurality of protrusions extending into the gases flow channel.

In some configurations, the gases manifold comprises a proximal bypass protrusion that is proximal to the nasal delivery elements and/or a distal bypass protrusion that is distal from the nasal delivery elements.

In some configurations, the gases manifold comprises both a proximal bypass protrusion and a distal bypass protrusion which in combination define a predetermined bypass dimension for the restricted flow of gases through the gases manifold between the first nasal delivery element and the second nasal delivery element.

In some configurations, the bypass restriction comprises an angled leading edge and an angled trailing edge that define a converging and diverging bypass restriction in a direction of gases flow through the gases manifold from the first nasal delivery element to the second nasal delivery element.

In some configurations, the nasal interface comprises an interface body and a gases manifold part, wherein the interface body and the gases manifold part together form the gases manifold.

In some configurations, the gases inlet is at a side of the gases manifold.

In some configurations, an open area for gases flow through the bias flow restriction is between about 10 mm² and about 30 mm², optionally between about 25 mm² and about 30 mm², and optionally about 27.5 mm².

In some configurations, an open area for gases flow through the bias flow restriction is more than 0 mm² to about 40 mm², optionally between about 2 mm² and about 40mm², optionally between about 2 mm² and about 5 mm², optionally between about 12 mm² and about 40mm², optionally between about 20 mm² and about 30 mm².

In some configurations, the bias flow restriction is configured such that a flow rate of the flow of gases out of the nasal interface through the bias flow restriction is more than 0 lpm to about 80 Ipm when a pressure of more than 0 cmH2O and up to about 30 cmH2O is provided to the gases inlet in use and the nasal delivery elements are occluded.

In some configurations, the bias flow restriction is configured such that a flow rate of the flow of gases out of the nasal interface through the bias flow restriction is between about 4 lpm and about 15 lpm when a pressure of between about 3 cmH2O and about 10 cmH2O is provided to the gases inlet in use and the nasal delivery elements are occluded.

In some configurations, the bias flow restriction is configured such that a flow rate of the flow of gases out of the nasal interface through the bias flow restriction is between about 15 lpm and about 80 Ipm when a pressure of between about 4 cmH2O and about 30 cmH2O is provided to the gases inlet in use and the nasal delivery elements are occluded.

In some configurations, the bias flow restriction comprises at least one aperture for the flow of gases from the nasal interface to an ambient environment, optionally wherein the bias flow restriction comprises a plurality of apertures for the flow of gases from the nasal interface to an ambient environment.

In some configurations, the bias flow restriction comprises a filter or a diffuser to filter or diffuse gases flowing through the aperture(s).

In some configurations, the nasal interface comprises a filter unit between the gases manifold and the bias flow restriction.

In some configurations, the bias flow restriction is in fluid communication with the gases manifold, optionally wherein the gases manifold comprises the bias flow restriction or is coupled to the bias flow restriction, optionally wherein the bias flow restriction is in fluid communication with the gases manifold but is positioned remotely from the gases manifold.

In some configurations, the cross-sectional area of the portion of the gases flow channel is between about 10% and up to about 100% of a first cross-sectional area of an adjacent part of the gases flow channel, optionally about 10% or more and less than 100% of the first cross-sectional area, optionally up to about 90% of the first cross-sectional area, optionally up to about 80% of the first cross-sectional area, optionally up to about 70% of the first cross-sectional area, optionally up to about 60% of the first cross-sectional area, optionally up to about 55% of the first cross-sectional area, optionally up to about 40% of the first cross-sectional area, optionally up to about 30% of the first cross-sectional area, and optionally up to about 25% of the first cross-sectional area.

In some configurations, the cross-sectional area of the portion of the gases flow channel is up to about 200 mm², optionally up to about 160 mm², optionally up to about 110 mm², optionally up to about 80 mm², optionally up to about 60 mm², and optionally up to about 50 mm².

In some configurations, the combined cross-sectional area of the nasal delivery elements is more than 0 mm² and up to about 250 mm², optionally between about 1 mm² and about 250 mm², optionally between about 1.6 mm² and about 250 mm², optionally between about 50 mm² and about 250 mm², optionally between about 50 mm² and about 200 mm², optionally between about 30 mm² and about 200 mm², optionally between about 30 mm² and about 155 mm², optionally between about 50 mm² and about 155 mm², and optionally between about 70 mm² and about 155 mm².

In some configurations, the cross-sectional area of the portion of the gases flow channel is more than 0 to about 1.5 times the combined cross-sectional area of the nasal delivery elements and the combined cross-sectional area of the nasal delivery elements is between about 1 mm² and about 250 mm².

In some configurations, the cross-sectional area of the portion of the gases flow channel is up to about 1.3 times the combined cross-sectional area of the nasal delivery elements, optionally up to about 1 times the combined cross-sectional area of the nasal delivery elements, optionally up to about 2/3 of the combined cross-sectional area of the nasal delivery elements, optionally up to about 1/2 of the combined cross-sectional area of the nasal delivery elements, optionally up to about 2/5 of the combined cross-sectional area of the nasal delivery elements, optionally up to about 1/3 of the combined cross-sectional area of the nasal delivery elements.

In some configurations, the combined cross-sectional area of the nasal delivery elements is between about 1.6 mm² and about 250 mm², optionally between about 50 mm² and about 250 mm², optionally between about 50 mm² and about 200 mm², optionally between about 30 mm² and about 200 mm², optionally between about 30 mm² and about 155 mm², optionally between about 50 mm² and about 155 mm², and optionally between about 70 mm² and about 155 mm².

In some configurations, the bypass restriction provides a pressure drop through the nasal interface between the first nasal delivery element and the second nasal delivery element when gases are delivered from the gases inlet to the first nasal delivery element and the second nasal delivery element such that pressure at the first nasal delivery element is higher than pressure at the second nasal delivery element.

In accordance with certain features, aspects and advantages of at least one of the embodiments disclosed herein, a nasal interface is disclosed, the nasal interface comprising:
a first nasal delivery element and a second nasal delivery element, wherein the first nasal delivery element and the second nasal delivery element are each configured to seal with a respective naris of a patient,
and a gases manifold comprising a gases inlet for delivery of respiratory gases to the gases manifold and a gases flow channel,
wherein the first nasal delivery element and the second nasal delivery element are in fluid communication with the gases inlet via the gases flow channel, wherein the first nasal delivery element is proximal to the gases inlet and the second nasal delivery element is distal from the gases inlet,
wherein the nasal interface comprises a bypass restriction that provides a cross-sectional area of a portion of the gases flow channel,
wherein each of the first nasal delivery element and the second nasal delivery element comprises an inner cross-sectional area,
and wherein the inner cross-sectional areas of the nasal delivery elements and the cross-sectional area of the portion of the gases flow channel are related so as to create an asymmetrical flow of gases from the nasal delivery elements in use.

In some configurations, the inner cross-sectional areas together provide a combined cross-sectional area of the nasal delivery elements, and wherein the cross-sectional area of the portion of the gases flow channel is more than 0 to about 1.5 times the combined cross-sectional area of the nasal delivery elements.

In some configurations, the cross-sectional area of the portion of the gases flow channel is up to about 1.3 times the combined cross-sectional area of the nasal delivery elements, optionally up to about 1 times the combined cross-sectional area of the nasal delivery elements, optionally up to about 2/3 of the combined cross-sectional area of the nasal delivery elements, optionally up to about 1/2 of the combined cross-sectional area of the nasal delivery elements, optionally up to about 2/5 of the combined cross-sectional area of the nasal delivery elements, optionally up to about 1/3 of the combined cross-sectional area of the nasal delivery elements.

In some configurations, the inner cross-sectional area of each of the first and second nasal delivery elements is at a smallest transverse dimension of the respective nasal delivery element.

In some configurations, the smallest transverse dimension is in a direction that is transverse to a direction of gases flow through the nasal delivery elements.

In some configurations, the inner cross-sectional area of each of the first and second nasal delivery elements is at an outlet of the respective nasal delivery element.

In some configurations, the cross-sectional area of the portion of the gases flow channel is up to about 1 times, optionally up to about 2/3 times, the combined cross-sectional area of the nasal delivery elements, and the nasal interface is configured to provide a bias flow through a bias flow restriction of 20 lpm when a pressure of 4 cmH2O is provided to the gases inlet and the nasal delivery elements are occluded.

In some configurations, the cross-sectional area of the portion of the gases flow channel is up to about 1 times, optionally up to about 2/3 times, the combined cross-sectional area of the nasal delivery elements, and the nasal interface is configured to provide a bias flow through a bias flow restriction of 32 lpm when a pressure of 8 cmH2O is provided to the gases inlet and the nasal delivery elements are occluded.

In some configurations, the cross-sectional area of the portion of the gases flow channel is up to about 2/3 times the combined cross-sectional area of the nasal delivery elements, and the nasal interface is configured to provide a bias flow through the bias flow restriction of 20 Ipm when a pressure of 4 cmH2O is provided to the gases inlet and the nasal delivery elements are occluded, or is configured to provide a bias flow through the bias flow restriction of 32 lpm when a pressure of 8 cmH2O is provided to the gases inlet and the nasal delivery elements are occluded, or is configured to provide a bias flow through the bias flow restriction of 41 lpm when a pressure of 12 cmH2O is applied to the gases inlet and the nasal delivery elements are occluded, or is configured to provide a bias flow of 48 Ipm through the bias flow restriction when a pressure of 16 cmH2O is applied to the gases inlet and the nasal delivery elements are occluded, or is configured to provide a bias flow through the bias flow restriction of 53 lpm when a pressure of 20 cmH2O is applied to the gases inlet and the nasal delivery elements are occluded.

In some configurations, the cross-sectional area of the portion of the gases flow channel is up to about 2/3 times the combined cross-sectional area of the nasal delivery elements, and the nasal interface is configured to provide a bias flow through the bias flow restriction of 32 lpm or higher when a pressure of 8 cmH2O is provided to the gases inlet and the nasal delivery elements are occluded.

In some configurations, the cross-sectional area of the portion of the gases flow channel is up to about 1/3 times the combined cross-sectional area of the nasal delivery elements and the nasal interface is configured to provide a bias flow through the bias flow restriction of 32 lpm or higher when a pressure of 8 cmH2O is provided to the gases inlet and the nasal delivery elements are occluded, or wherein the cross-sectional area of the portion of the gases flow channel is up to about 2/5 times the combined cross-sectional area of the nasal delivery elements and the nasal interface is configured to provide a bias flow through the bias flow restriction of 41 lpm or higher when a pressure of 12 cm H2O is provided to the gases inlet and the nasal delivery elements are occluded, or wherein the cross-sectional area of the portion of the gases flow channel is up to about 2/3 times the combined cross-sectional area of the nasal delivery elements and the nasal interface is configured to provide bias flow through the bias flow restriction of 48 lpm or higher when a pressure of 16 cmH2O is provided to the gases inlet and the nasal delivery elements are occluded.

In accordance with certain features, aspects and advantages of at least one of the embodiments disclosed herein, a nasal interface is disclosed, the nasal interface comprising:
an interface body configured to substantially form a seal with a patient's nasal airways, the interface body configured to deliver gases to a first naris of the patient and to a second naris of the patient,
and a gases inlet for delivery of respiratory gases into the nasal interface, wherein the gases inlet is in fluid communication with the interface body to deliver the respiratory gases from the gases inlet through the interface body to the first naris and second naris of the patient in use, and
wherein the nasal interface is configured to receive incoming gases from the gases inlet and to provide, from the incoming gases, a first flow stream of gases configured to be substantially provided to the first naris of the patient in use and a second flow stream of gases configured to be substantially provided to the second naris of the patient in use, and is configured to direct more of the incoming gases to the first flow stream of gases than to the second flow stream of gases, to create an asymmetric flow of gases at a patient's nasal airways throughout a respiratory cycle of a patient.

In some configurations, the interface body comprises a first outlet or first outlet portion configured to substantially deliver gases to the first naris of the patient, and comprises a second outlet or second outlet portion configured to substantially deliver gases to the second naris of the patient.

In some configurations, the gases inlet is at least partly aligned with the first outlet or first outlet portion and is less aligned or is not aligned with the second outlet or second outlet portion.

In some configurations, the gases inlet is substantially axially aligned with the first outlet or first outlet portion.

In some configurations, at least half of a transverse cross-sectional area of the gases inlet is axially aligned with at least half of a transverse cross-sectional area of the first outlet or first outlet portion.

In some configurations, the gases inlet comprises an outer portion for connecting to a respiratory conduit to provide a flow of gases for a gases source to the interface body, and further comprises an inner portion in fluid communication with the interface body.

In some configurations, the inner portion of the gases inlet is at least partly aligned with the first outlet or first outlet portion.

In some configurations, the gases inlet is angled toward the first outlet or first outlet portion.

In some configurations, the first flow stream of gases has at least one dimension that is larger than a corresponding dimension of the second flow stream of gases.

In some configurations, the at least one dimension comprises a lateral dimension of the first flow stream of gases, and wherein the corresponding dimension comprises a lateral dimension of the second flow stream of gases.

In some configurations, the first flow stream of gases has a larger diameter, cross-sectional area, and/or volume than a corresponding diameter, cross-sectional area, and/or volume of the second flow stream of gases.

In some configurations, a ratio of the cross-sectional area of the first flow stream of gases to the corresponding cross-sectional area of the second flow stream of gases is between about 2:1 and about 5:1, optionally between about 2:1 and about 4:1, optionally between about 2.5:1 and about 3.5: 1, optionally about 3:1.

In some configurations, the first outlet or first outlet portion and the second outlet or second outlet portion comprise substantially the same cross-sectional areas.

In some configurations, the nasal interface is configured to deliver a lower velocity of gases flow through the first outlet or first outlet portion than a velocity of gases flow through the second outlet or second outlet portion during an inhalation phase of a respiratory cycle.

In some configurations, the nasal interface is configured to deliver a higher pressure of gases flow through the first outlet or first outlet portion than a pressure of gases flow through the second outlet or second outlet portion during an inhalation phase of a respiratory cycle.

In some configurations, the nasal interface comprises a single outlet for delivering gases to the first naris and second naris of the patient, wherein the single outlet comprises the first outlet portion and the second outlet portion, and wherein the nasal interface is configured such that the first flow stream of gases is configured to be substantially delivered to the first outlet portion and the second flow stream of gases is configured to be substantially delivered to the second outlet portion.

In some configurations, the interface body comprises a first nasal delivery element comprising the first outlet and a second nasal delivery element comprising the second outlet, wherein the nasal interface is configured such that the first flow stream of gases is configured to be substantially delivered to the first nasal delivery element and the second flow stream of gases is configured to be substantially delivered to the second nasal delivery element, and wherein the first nasal delivery element and the second nasal delivery element are each configured to seal with a respective naris of a patient.

In some configurations, the nasal interface comprises a flow director that is configured to direct more of the incoming gases from the gases inlet to the first flow stream of gases than to the second flow stream of gases.

In some configurations, the nasal interface comprises a connector or elbow for connecting a respiratory conduit to the patient interface.

In some configurations, the connector or elbow comprises or is the flow director.

In some configurations, the flow director comprises a nozzle that is configured to accelerate flow towards the first outlet or first outlet portion.

In some configurations, the nasal interface is configured to direct more of the incoming gases to the first flow stream of gases than to the second flow stream of gases during an inhalation phase of the respiratory cycle.

In some configurations, the interface body is a nasal cushion.

In some configurations, the nasal interface is configured to simultaneously deliver the respiratory gases from the gases inlet through the interface body to both the first naris and second naris of the patient in use.

In accordance with certain features, aspects and advantages of at least one of the embodiments disclosed herein, a nasal interface is disclosed, the nasal interface comprising:
an interface body configured to substantially form a seal with a patient's nasal airways, the interface body configured to deliver gases to a first naris of the patient and to a second naris of the patient,
and a gases inlet for delivery of respiratory gases into the nasal interface, wherein the gases inlet is in fluid communication with the interface body to deliver the respiratory gases from the gases inlet through the interface body to the first naris and second naris of the patient in use, and
wherein the nasal interface is configured to provide a larger dynamic pressure at the first naris of the patient in use and to provide a smaller dynamic pressure at the second naris of the patient in use, to create an asymmetric flow of gases at a patient's nasal airways throughout a respiratory cycle of a patient.

In some configurations, the interface body comprises a first outlet or first outlet portion configured to deliver gases to the first naris of the patient, and comprises a second outlet or second outlet portion configured to deliver gases to the second naris of the patient.

In some configurations, the nasal interface comprises a flow director that is configured to direct more of the incoming gases from the gases inlet to the first outlet or first outlet portion than to the second outlet or second outlet portion.

In some configurations, the flow director comprises a nozzle that is configured to accelerate flow towards the first outlet or first outlet portion.

In some configurations, the nasal interface is configured to receive incoming gases from the gases inlet and provide, from the incoming gases, a first flow stream of gases configured to be substantially provided to the first naris of the patient in use and a second flow stream of gases configured to be substantially provided to the second naris of the patient in use, and is configured to direct more of the incoming gases to the first flow stream of gases than to the second flow stream of gases.

In some configurations, the nasal interface comprises a flow splitter configured to unevenly split the flow from the gases inlet into the first flow stream of gases and the second flow stream of gases.

In some configurations, the nasal interface is configured to simultaneously deliver the respiratory gases from the gases inlet through the interface body to both the first naris and second naris of the patient in use.

The nasal interface of this aspect may have one or more of the features outlined in relation to any of the other aspects.

In accordance with certain features, aspects and advantages of at least one of the embodiments disclosed herein, a nasal interface is disclosed, the nasal interface comprising:
an interface body configured to substantially form a seal with a patient's nasal airways, the interface body configured to deliver gases to a first naris of the patient and to a second naris of the patient,
and a gases inlet for delivery of respiratory gases into the nasal interface, wherein the gases inlet is in fluid communication with the interface body to deliver the respiratory gases from the gases inlet through the interface body to the first naris and second naris of the patient in use, and
a flow splitter configured to unevenly split the flow from the gases inlet into a first flow stream of gases configured to be substantially provided to the first naris of the patient in use and a second flow stream of gases configured to be substantially provided to the second naris of the patient in use, wherein the first flow stream of gases is configured to deliver a greater flow of gases along the first flow stream of gases than a flow of gases along the second flow stream of gases, to create an asymmetric flow of gases at a patient's nasal airways throughout a respiratory cycle of a patient.

In some configurations, the interface body comprises a first outlet or first outlet portion configured to deliver gases to the first naris of the patient, and comprises a second outlet or second outlet portion configured to deliver gases to the second naris of the patient.

In some configurations, the gases inlet is at least partly aligned with the first outlet or first outlet portion and is less aligned or is not aligned with the second outlet or second outlet portion.

In some configurations, the gases inlet is substantially axially aligned with the first outlet or first outlet portion.

In some configurations, at least half of a transverse cross-sectional area of the gases inlet is axially aligned with at least half of a transverse cross-sectional area of the first outlet or first outlet portion.

In some configurations, the gases inlet comprises an outer portion for connecting to a respiratory conduit to provide a flow of gases for a gases source to the interface body, and further comprises an inner portion in fluid communication with the interface body.

In some configurations, the inner portion of the gases inlet is at least partly aligned with the first outlet or first outlet portion.

In some configurations, the gases inlet is angled toward the first outlet or first outlet portion.

In some configurations, the first flow stream of gases has at least one dimension that is larger than a corresponding dimension of the second flow stream of gases.

In some configurations, the at least one dimension comprises a lateral dimension of the first flow stream of gases, and wherein the corresponding dimension comprises a lateral dimension of the second flow stream of gases.

In some configurations, the first flow stream of gases has a larger diameter, cross-sectional area, and/or volume than a corresponding diameter, cross-sectional area, and/or volume of the second flow stream of gases.

In some configurations, a ratio of the cross-sectional area of the first flow stream of gases to the corresponding cross-sectional area of the second flow stream of gases is between about 2:1 and about 5:1, optionally between about 2:1 and about 4:1, optionally between about 2.5:1 and about 3.5:1, optionally about 3:1.

In some configurations, the first outlet or first outlet portion and the second outlet or second outlet portion comprise substantially the same cross-sectional areas.

In some configurations, the nasal interface is configured to deliver a lower velocity of gases flow through the first outlet or first outlet portion than a velocity of gases flow through the second outlet or second outlet portion during an inhalation phase of a respiratory cycle.

In some configurations, the nasal interface is configured to deliver a higher pressure of gases flow through the first outlet or first outlet portion than a pressure of gases flow through the second outlet or second outlet portion during an inhalation phase of a respiratory cycle.

In some configurations, the nasal interface comprises a gases manifold, and the interface body, the gases manifold, and/or the gases inlet comprise(s) the flow splitter.

In some configurations, the flow splitter comprises a wall portion that extends towards or into the gases inlet, wherein the first flow stream of gases is located on one side of the wall portion and the second flow stream of gases is located on an opposite side of the wall portion.

In some configurations, the flow splitter extends into the gases inlet, and splits the gases inlet into a first gases flow stream portion on said one side of the flow splitter and a second gases flow stream portion on an opposite side of the flow splitter.

In some configurations, the flow splitter is substantially rigid.

In some configurations, the interface body is a nasal cushion.

In some configurations, the nasal cushion comprises the flow splitter, and wherein the flow splitter is configured to move and/or deform upon compression of the nasal cushion.

In some configurations, the flow splitter comprises a first wall portion and a second wall portion.

In some configurations, the first wall portion and the second wall portion are hingedly connected to each other, and wherein relative angles of the wall portions are configured to change upon compression of the nasal cushion.

In some configurations, the first wall portion and the second wall portion overlap each other in a relaxed state of the nasal cushion, and wherein an extent of overlap of the wall portions increases upon compression of the nasal cushion.

In some configurations, the nasal interface comprises a single outlet for delivering gases to the first naris and second naris of the patient, wherein the single outlet comprises the first outlet portion and the second outlet portion, and wherein the nasal interface is configured such that the first flow stream of gases is configured to be substantially delivered to the first outlet portion and the second flow stream of gases is configured to be substantially delivered to the second outlet portion.

In some configurations, the interface body comprises a first nasal delivery element comprising the first outlet and a second nasal delivery element comprising the second outlet, wherein the nasal interface is configured such that the first flow stream of gases is configured to be substantially delivered to the first nasal delivery element and the second flow stream of gases is configured to be substantially delivered to the second nasal delivery element, and wherein the first nasal delivery element and the second nasal delivery element are each configured to seal with a respective naris of a patient.

In accordance with certain features, aspects and advantages of at least one of the embodiments disclosed herein, a nasal interface is disclosed, the nasal interface comprising:
an interface body comprising a first nasal delivery element comprising a first outlet configured to deliver gases to a first naris of a patient and a second nasal delivery element comprising a second outlet configured to deliver gases to a second naris of a patient, wherein the first nasal delivery element and the second nasal delivery element are each configured to seal with a respective naris of a patient,
and a gases inlet for delivery of respiratory gases into the nasal interface, wherein the gases inlet is in fluid communication with the interface body to deliver the respiratory gases from the gases inlet through the first nasal delivery element and through the second nasal delivery element, and
a flow splitter to unevenly split the flow from the gases inlet into a first flow stream of gases configured to be substantially provided to the first nasal delivery element and a second flow stream of gases configured to be substantially provided to the second nasal delivery element, wherein the first flow stream of gases is configured to deliver a greater flow of gases along the first flow stream of gases than a flow of gases along the second flow stream of gases, to create an asymmetric flow of gases at a patient's nasal airways throughout a respiratory cycle of a patient.

In some configurations, the nasal interface comprises a flow director that is configured to direct more of the incoming gases from the gases inlet to the first flow stream of gases than to the second flow stream of gases.

In some configurations, the flow director comprises a nozzle that is configured to accelerate flow towards the first outlet or first outlet portion.

In some configurations, the nasal interface is configured to direct more of the incoming gases to the first flow stream of gases than to the second flow stream of gases during an inhalation phase of the respiratory cycle.

In some configurations, the interface body is a nasal cushion.

In some configurations, the nasal interface is configured to simultaneously deliver the respiratory gases from the gases inlet through the interface body to both the first naris and second naris of the patient in use.

In some configurations, the nasal interface comprises a bias flow restriction comprising at least one aperture for the flow of gases from the nasal interface to an ambient environment.

In some configurations, the bias flow restriction comprises a filter or a diffuser to filter or diffuse gases flowing through the aperture(s).

In some configurations, the nasal interface is configured such that a pressure differential of gases flow through the first outlet or first outlet portion and the second outlet or second outlet portion is higher during an expiration phase than during an inspiration phase.

In some configurations, the nasal interface is configured to achieve a patient pressure at the first outlet or first outlet portion and the second outlet or second outlet portion of between about 2 cmH2O and about 30 cmH2O in use, optionally between about 2 cmH2O and about 25 cmH2O in use, optionally between about 2 cmH2O and about 20 cmH2O in use, optionally between about 2 cmH2O and about 15 cmH2O in use, optionally between about 2 cmH2O and about 14 cmH2O in use, optionally between about 2 cmH2O and about 13 cmH2O in use, optionally between about 2 cmH2O and about 12 cmH2O in use, optionally between about 2 cmH2O and about 11 cmH2O in use, optionally between about 2 cmH2O and about 10 cmH2O in use.

In some configurations, a pressure differential between the first outlet or first outlet portion and the second outlet or second outlet portion is configured to provide an asymmetric flow through upper airways of a patient of at least about 1 liter per minute (lpm), optionally between about 1 lpm and about 5 lpm.

In some configurations, the asymmetric flow promotes clearing of CO₂ from anatomical dead space of the patient.

In accordance with certain features, aspects and advantages of at least one of the embodiments disclosed herein, a respiratory therapy system is disclosed, the respiratory therapy system comprising:
a gases source for respiratory gases and configured to provide pressure controlled respiratory gases;
a breathing tube to receive the pressure controlled respiratory gases; and
the nasal interface outlined above or herein in fluid communication with the breathing tube to deliver the respiratory gases to a patient.

In some configurations, the interface body comprises a first outlet or first outlet portion configured to deliver gases to the first naris of the patient, and comprises a second outlet or second outlet portion configured to deliver gases to the second naris of the patient, and wherein the nasal interface is configured to create a pressure differential between the first outlet or first outlet portion and the second outlet or second outlet portion when gases are delivered from the gases inlet to both the first outlet or first outlet portion and the second outlet or second outlet portion such that pressure at the first outlet or first outlet portion is higher than pressure at the second outlet or second outlet portion.

In accordance with certain features, aspects and advantages of at least one of the embodiments disclosed herein, a respiratory therapy system is disclosed, the respiratory therapy system comprising:
a gases source for respiratory gases and configured to provide pressure controlled respiratory gases;
a breathing tube to receive the pressure controlled respiratory gases; and
a nasal interface having a gases inlet in fluid communication with the breathing tube to deliver the respiratory gases to a patient, the nasal interface comprising a first nasal delivery element and a second nasal delivery element, wherein the first nasal delivery element and the second nasal delivery element are each configured to seal with a respective naris of a patient, wherein the nasal interface is configured to create a pressure differential between the first nasal delivery element and the second nasal delivery element when gases are delivered from the gases inlet to both the first nasal delivery element and the second nasal delivery element such that pressure at the first nasal delivery element is higher than pressure at the second nasal delivery element.

In some configurations, the respiratory therapy system comprises a respiratory conduit to receive the pressure controlled respiratory gases from the breathing tube, wherein the respiratory conduit is in fluid communication with the breathing tube and the gases inlet of the nasal interface.

In some configurations, the respiratory therapy system further comprises a respiratory gases filter.

In some configurations, the respiratory gases filter is located between the breathing tube and the respiratory conduit.

In some configurations, the respiratory gases filter is located between the gases manifold and a bias flow restriction.

In some configurations, the respiratory therapy system further comprises a humidifier configured to humidify said pressure controlled respiratory gases prior to their delivery to the nasal interface.

In some configurations, the breathing tube is a heated breathing tube, and is configured to receive the pressure controlled respiratory gases from the humidifier.

In some configurations, a temperature of gases flow exiting the first and second nasal delivery elements is between about 31°C and about 41°C, optionally more than 31°C and up to about 41°C, optionally between about 36°C and about 39°C, optionally about 37°C.

In accordance with certain features, aspects and advantages of at least one of the embodiments disclosed herein, a method of providing respiratory support to a patient is provided, the method comprising:
providing a respiratory therapy system comprising:
   a gases source for respiratory gases and configured to provide pressure controlled respiratory gases;
   a breathing tube to receive the pressure controlled respiratory gases; and
   a nasal interface having a gases inlet in fluid communication with the breathing tube to deliver the respiratory gases to a patient, the nasal interface comprising a first nasal delivery element and a second nasal delivery element;
sealing each of the first nasal delivery element and the second nasal delivery element with a respective naris of a patient;
operating the respiratory therapy apparatus to provide a flow of gases to the nasal interface; and
delivering an asymmetrical flow of gases from the respiratory therapy apparatus through the first nasal delivery element and the second nasal delivery element at a patient's nares.

In some configurations, the nasal delivery elements are in fluid communication with the gases inlet via a gases flow channel, wherein the first nasal delivery element is proximal to the gases inlet and the second nasal delivery element is distal from the gases inlet, and wherein the nasal interface comprises a bypass restriction that provides a cross-sectional area of a portion of the gases flow channel, wherein each of the first nasal delivery element and the second nasal delivery element comprises an inner cross-sectional area, wherein the inner cross-sectional areas together provide a combined cross-sectional area of the nasal delivery elements, and wherein the cross-sectional area of the portion of the gases flow channel is more than 0 to about 1.5 times the combined cross-sectional area of the nasal delivery elements.

In some configurations, the cross-sectional area of the portion of the gases flow channel is up to about 1 times, optionally up to about 2/3 times, the combined cross-sectional area of the nasal delivery elements, and wherein the method comprises providing a pressure of 4 cmH2O to the gases inlet such that there is a bias flow through a bias flow restriction of 20 Ipm.

In some configurations, the cross-sectional area of the portion of the gases flow channel is up to about 1 times, optionally up to about 2/3 times, the combined cross-sectional area of the nasal delivery elements, and wherein the method comprises providing a pressure of 8 cmH2O to the gases inlet such that there is a bias flow through a bias flow restriction of 32 Ipm.

In some configurations, the cross-sectional area of the portion of the gases flow channel is up to about 2/3 times the combined cross-sectional area of the nasal delivery elements, and wherein the method comprises providing a pressure of 4 cmH2O to the gases inlet such that there is a bias flow through the bias flow restriction of 20 Ipm, or wherein the method comprises providing a pressure of 8 cmH2O to the gases inlet such that there is a bias flow through the bias flow restriction of 32 Ipm, or wherein the method comprises providing a pressure of 12 cmH2O to the gases inlet such that there is a bias flow through the bias flow restriction of 41 Ipm, or wherein the method comprises providing a pressure of 16 cmH2O to the gases inlet such that there is a bias flow through the bias flow restriction of 48 Ipm, or wherein the method comprises providing a pressure of 20 cmH2O to the gases inlet such that there is a bias flow through the bias flow restriction of 53 lpm.

In some configurations, the cross-sectional area of the portion of the gases flow channel is up to about 2/3 times the combined cross-sectional area of the nasal delivery elements, and wherein the method comprises providing a pressure of 8 cmH2O to the gases inlet such that there is a bias flow through the bias flow restriction of 32 lpm or higher.

In some configurations, the cross-sectional area of the portion of the gases flow channel is up to about 1/3 times the combined cross-sectional area of the nasal delivery elements and wherein the method comprises providing a pressure of 8 cmH2O to the gases inlet such that there is a bias flow through the bias flow restriction of 32 lpm or higher, or wherein the cross-sectional area of the portion of the gases flow channel is up to about 2/5 times the combined cross-sectional area of the nasal delivery elements and wherein the method comprises providing a pressure of 12 cmH2O to the gases inlet such that there is a bias flow through the bias flow restriction of 41 lpm or higher, or wherein the cross-sectional area of the portion of the gases flow channel is up to about 2/3 times the combined cross-sectional area of the nasal delivery elements and wherein the method comprises providing a pressure of 16 cmH2O to the gases inlet such that there is a bias flow through the bias flow restriction of 48 lpm or higher.

In some configurations, a temperature of gases flow exiting the first and second nasal delivery elements is between about 31°C and about 41°C, optionally more than 31°C and up to about 41°C, optionally between about 36°C and about 39°C, optionally about 37°C.

In some configurations, the nasal interface is as outlined above or herein.

In some configurations, the respiratory therapy system is as outlined above or herein.

In accordance with certain features, aspects and advantages of at least one of the embodiments disclosed herein, a method of providing respiratory support to a patient is provided, the method comprising:
providing a respiratory therapy system comprising:
a gases source for respiratory gases and configured to provide pressure controlled respiratory gases;
a breathing tube to receive the pressure controlled respiratory gases; and
a nasal interface in fluid communication with the breathing tube 16 to deliver the respiratory gases to a patient;
sealing the nasal interface with a patient's nasal airways;
operating the respiratory therapy apparatus to provide a flow of gases to the nasal interface; and
receiving incoming gases at a gases inlet of the nasal interface and creating an asymmetric flow of gases at a patient's nasal airways.

In some configurations, the method comprises creating an asymmetric flow of gases at the patient's nasal airways throughout a respiratory cycle of the patient.

In some configurations, the nasal interface is as outlined above or herein.

In some configurations, the respiratory therapy system is as outlined above or herein.

Features from one or more embodiments or configurations may be combined with features of one or more other embodiments or configurations. Additionally, more than one embodiment or configuration may be used together in a respiratory support system during a process of respiratory support of a patient.

As used herein the term "(s)" following a noun means the plural and/or singular form of that noun.

As used herein the term "and/or" means "and" or "or", or where the context allows both.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

This disclosure may also be said broadly to consist in the parts, elements and features referred to or indicated in the specification of the application, individually or collectively, and any or all combinations of any two or more said parts, elements or features, and where specific integers are mentioned herein which have known equivalents in the art to which this disclosure relates, such known equivalents are deemed to be incorporated herein as if individually set forth.

The disclosure consists in the foregoing and also envisages constructions of which the following gives examples only.

### BRIEF DESCRIPTION OF THE DRAWINGS

Specific embodiments and modifications thereof will become apparent to those skilled in the art from the detailed description herein having reference to the figures that follow, of which:
Figure 1 is a front perspective view of an exemplary configuration patent interface of the present disclosure comprising a nasal interface.
Figure 2 is a close-up perspective view of the nasal interface.
Figure 3 is a rear perspective view of the patient interface.
Figure 4 is a close-up perspective view of the nasal interface.
Figure 5 is a front perspective view of the patient interface, showing the gases manifold part separated from the interface body part that comprises the nasal delivery elements, a bias flow restriction part separated from the gases manifold part, and a respiratory conduit separated from the gases manifold part.
Figure 6(a) is a front perspective view of the bias flow restriction part.
Figure 6(b) is an exploded front perspective view of components of the bias flow restriction part.
Figure 7(a) is a perspective sectional view of the bias flow restriction part.
Figure 7(b) is an orthogonal sectional view of the bias flow restriction part.
Figure 8 is an orthogonal view towards the front of the bias flow restriction part.
Figure 9 is a front partial sectional view of the nasal interface showing a gases flow restriction in the gases manifold.
Figure 10(a) is a front perspective sectional view of the nasal interface schematically showing gases flow directions through the nasal interface.
Figure 10(b) is an orthogonal front sectional view of the nasal interface schematically showing gases flow directions through the nasal interface.
Figure 11 shows views of the gases manifold, where Figure 11(a) is a front perspective view, Figure 11(b) is a front perspective view sectioned through a horizontal plane, and Figure 11(c) is a front perspective view sectioned through a vertical plane.
Figure 12 shows views of the gases manifold, where Figure 12(a) is a top view, Figure 12(b) is a sectional view along line b-b of Figure 12(d), Figure 12(c) is a front view, Figure 12(d) is an end view, and Figure 12(e) is a sectional view along line e-e of Figure 12(d).
Figure 13 shows views of the face mount part or interface body part of the nasal interface, where Figure 13(a) is a rear view, Figure 13(b) is a front view, and Figure 13(c) is a sectional view along line c-c of Figure 13(b).
Figure 14 is a sectional view through the gases manifold and one of the nasal delivery elements.
Figure 15 is a schematic diagram of the functionality and effect of use of the patient interface.
Figure 16 shows side-swapping functionality where the respiratory conduit is coupled to the right side of the gases manifold in Figure 16(a) and the respiratory conduit is coupled to the left side of the gases manifold in Figure 16(b).
Figure 17(a) shows remote positioning of the bias flow restriction part.
Figure 17(b) shows remote positioning of the bias flow restriction part with a filter between the gases manifold and the bias flow restriction part.
Figure 18 is an exploded view of components of the headgear of the patient interface.
Figure 19 schematically shows the configuration of the nasal interface of Figures 1 to 18.
Figure 20 schematically shows an alternative configuration of the nasal interface.
Figure 21 schematically shows another alternative configuration of the nasal interface.
Figure 22 shows a respiratory therapy system incorporating the patient interface and nasal interface of the present disclosure.
Figure 23 shows the results of testing different ratios of bypass restriction to combined nasal delivery element area for 15 breaths per minute 10i:20e 500Vt (tidal volume) breath pattern, at pressures of 4 cmH2O and 8 cmH2O.
Figure 24 shows the results of testing different ratios of bypass restriction to combined nasal delivery element area for 25 breaths per minute ARDS (acute respiratory distress syndrome) breath pattern, at pressures of 4 cmH2O, 8 cmH2O, 12 cmH2O, 16 cmH2O, and 20 cmH2O.
Figure 25 shows the results of testing different ratios of bypass restriction to combined nasal delivery element area for 45 breaths per minute 350 Vt (tidal volume) sinusoidal breath pattern, at pressures of 4 cmH2O, 8 cmH2O, 12 cmH2O, 16 cmH2O, and 20 cmH2O.
Figure 26 shows modelled effects of different nasal delivery element sizes, different bypass restriction cross-sectional areas, different set pressures, and different bias flow restriction openness on rebreathing with the nasal interface for 15 breaths per minute.
Figure 27 shows modelled effects of different nasal delivery element sizes, different bypass restriction cross-sectional areas, different set pressures, and different bias flow restriction openness on rebreathing with the nasal interface for 25 breaths per minute.
Figure 28 shows modelled effects of different nasal delivery element sizes, different bypass restriction cross-sectional areas, different set pressures, and different bias flow restriction openness on rebreathing with the nasal interface with for 45 breaths per minute.
Figure 29 schematically shows an alternative configuration nasal interface for use in the patient interface.
Figure 30 shows a front perspective view of an exemplary configuration of the nasal interface.
Figure 31 shows a front sectional view of the nasal interface showing gases flow paths.
Figure 32 shows a front sectional view of the nasal interface showing an exhaust gas flow path.
Figure 33 shows an overhead part sectional view of the nasal interface.
Figure 34 shows another overhead part sectional view of the nasal interface.
Figure 35 shows an overhead front perspective view of an interface body/nasal cushion of the nasal interface.
Figure 36 shows an underside front perspective view of an interface body/nasal cushion of the nasal interface.
Figure 37 shows a front sectional view of an alternative exemplary configuration nasal interface.
Figure 38 shows a front perspective sectional view of another alternative exemplary configuration nasal interface.
Figure 39 shows a front perspective view of another alternative exemplary configuration nasal interface.
Figure 40 shows a part sectional view of the nasal interface.
Figure 41 shows a front perspective sectional view of an alternative exemplary configuration nasal interface.
Figure 42 shows a front perspective sectional view of an alternative exemplary configuration nasal interface.
Figure 43 shows a top view of an alternative exemplary configuration nasal interface.
Figure 44 shows front section views of an alternative exemplary configuration nasal interface, where Figure 44(a) shows the nasal cushion in an at-rest state and Figure 44(b) shows the nasal cushion in a compressed state.
Figure 45 shows a front perspective view of an alternative exemplary configuration nasal cushion for use in the nasal interfaces.
Figure 46 shows a rear perspective view of the nasal cushion.
Figure 47 shows deformation or movement of a flow director or flow splitter of the nasal cushion.
Figure 48 shows alternative deformation or movement of a flow director or flow splitter of the nasal cushion.
Figure 49 shows an alternative exemplary configuration nasal cushion for use in the nasal interfaces, where Figure 49(a) is a first front perspective view and Figure 49(b) is a second front perspective view.
Figures 50(a) - 50(c) show three alternative exemplary configuration nasal cushions for use in the nasal interfaces.
Figure 51 shows an alternative exemplary configuration nasal cushion for use in the nasal interfaces, where Figure 51(a) is a top perspective view and Figure 51(b) is a front view.
Figure 52 shows an alternative exemplary configuration nasal cushion for use in the nasal interfaces, where Figure 52(a) is a rear view and Figure 52(b) is a top perspective view.
Figure 53 is a front perspective view of an alternative exemplary configuration nasal interface.
Figure 54 is an exploded front perspective view of the nasal interface.
Figure 55 is an exploded rear perspective view of the nasal interface.
Figure 56 is an overhead sectional view of the nasal interface.
Figure 57 is a front perspective view of a patient interface comprising an alternative exemplary configuration nasal interface.
Figure 58 is an overhead sectional view of the nasal interface.
Figure 59 is a front perspective view of the nasal cushion of the nasal interface.

### DETAILED DESCRIPTION

Patient interfaces can be used for delivering breathing gases to airways of a patient. The patient interfaces may comprise nasal interfaces that can be used to deliver a flow of gases to a patient. In some configurations, nasal delivery elements, such as nasal prongs or pillows, are inserted into the nose of a patient to deliver the required therapy. The nasal delivery elements may be desired to seal at the nose to deliver the therapy. One or more of the nasal delivery elements may comprise a nasal pillow to seal at the nose.

Disclosed is a system to deliver gases to a patient through a nasal interface.

The system provides a pressure differential at first and second nasal delivery elements of the nasal interface, with a resulting differential gases flow at the first and second nasal delivery elements. This allows an asymmetrical flow to be delivered through the nasal interface to both nares. Asymmetrical flow as described herein refers to a flow that differs within the nasal interface or within the nose. In this way, a different flow may be delivered by each nasal delivery element. An asymmetrical flow may also include partial unidirectional flow.

Delivery of asymmetrical flow may improve clearance of dead space in the upper airways. A nasal interface as described is configured to produce such asymmetrical flow through nasal delivery elements.

Flow generated by respiratory therapy depends on flow through the nasal interface, which depends on the pressure at each nasal delivery element. If the pressure is different at each nasal delivery element, an asymmetric flow of gases will be generated.

If flow, leak, or a combination of flow and leak, is asymmetrical through the nasal interface, the flow through the nose may become asymmetrical during breathing. Partial unidirectional flow may be a type of asymmetrical flow. Partial unidirectional flow may provide improved clearance of anatomical dead space as the air is flushed from the upper airways. Partial unidirectional flow may be more comfortable than total unidirectional flow. Total unidirectional flow herein includes all flow entering one naris by a nasal delivery element and exiting via the other naris via a nasal delivery element, venting to the atmosphere, due to the absence of a nasal delivery element, or the like. Partial unidirectional flow as described herein includes flow that may enter the nose via both nares and leave the nose from one naris, flow that may enter the nose through one naris and leave the nose via both nares, or different proportions of flow that may enter the nose through both nares and/or different proportions of flow that may leave the nose through both nares, and may be flow that may enter the nose via both nares and leave the nose from one or both nares and optionally via the mouth. If there is a pressure differential between the first and second nasal delivery elements, during inspiration the first nasal delivery element will receive more gases flow from a gases inlet than the second nasal delivery element. During expiration, the second nostril associated with the second nasal delivery element will expel more gases flow than the first nostril associated with the first nasal delivery element. The pressure differential between the first and second nasal delivery elements can change depending on whether the patient's breathing cycle is in an inspiration phase or expiration phase.

The asymmetrical flow assessment may be applied over a suitable period. For example, the asymmetrical flow assessment may be applied over one breath cycle of the patient or alternatively over a different number of breath cycles of the patient.

The partially unidirectional flow may reduce turbulence in the patient's nasal cavity, which could improve comfort.

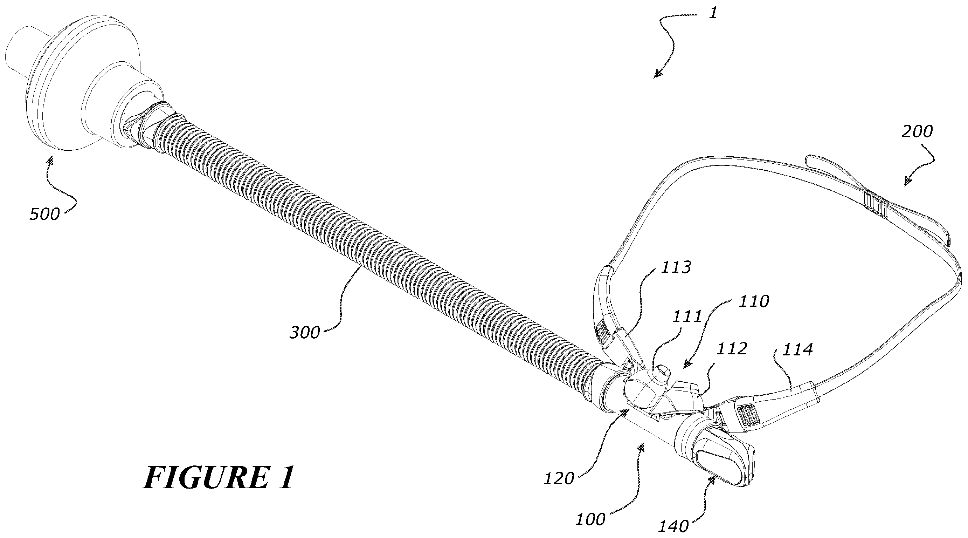

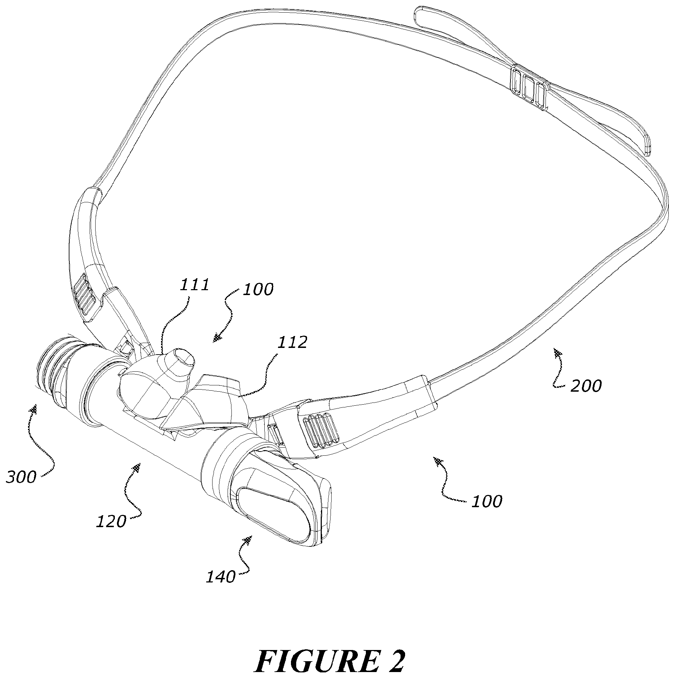

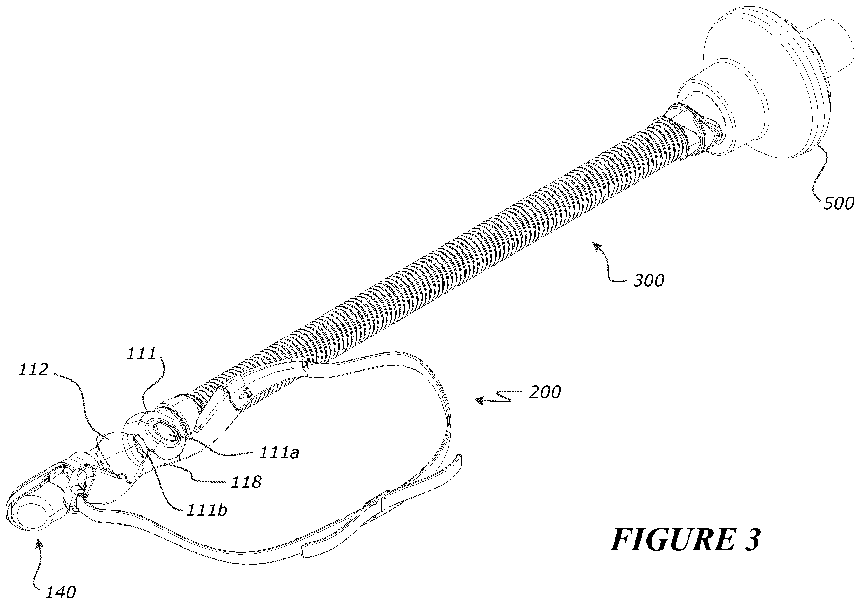

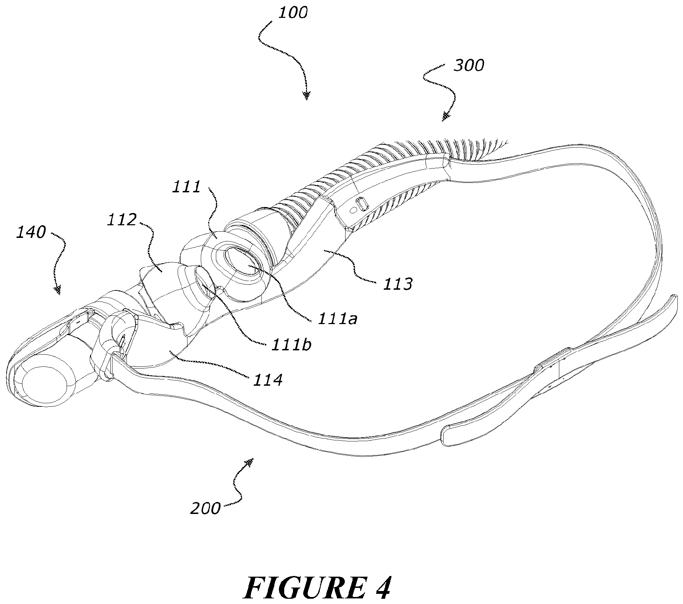

Figures 1-5 show an exemplary patient interface 1 that comprises a nasal interface 100 with nasal delivery elements comprising a first nasal delivery element 111 and a second nasal delivery element 112.

The nasal interface 100 provides a patient with a patient interface suitable for the delivery of pressure-controlled, optionally high humidity, gas flow to the patient's nasal cavity/nares. In some configurations, the nasal interface 100 is adapted to deliver a high flow of gases over a wide flow range (e.g. about 8 Ipm, or higher depending on other therapy applications, perhaps such as 10 - 50 Ipm, 20 - 40 Ipm, or higher). The flow rates may be bias flows averaged over time. In some configurations, the nasal interface 100 is adapted to deliver a lower flow of gases. The flow is dependent on pressure so it can fluctuate depending on different breathing pressures and set pressures. Wherein set pressure(s) relates to the therapy and/or patient pressure(s) which are maintained by an ancillary respiratory therapy apparatus when used in conjunction with the nasal interface of the disclosure.

The nasal interface 100 comprises a face mount part or interface body 110 part including a pair of hollow nasal delivery elements 111 and 112, integrally moulded with or removably attached to the interface body 110. The nasal interface 100 comprises a gases manifold 120 part that comprises a gases inlet 121. The gases manifold 120 may be removably attached or integrally moulded to the respiratory conduit 300.

The interface body 110 part may be connectable to or engageable with the gases manifold 120 part, or may be integrally formed or permanently engaged with the gases manifold 120 part. If the interface body 110 part is engageable with the gases manifold part 120, that engagement brings the first nasal delivery element 111 and the second nasal delivery element 112 into fluid communication with the gases inlet 121 such that the first nasal delivery element 111 is more proximal the gases inlet 121 and the second nasal delivery 112 element is more distal the gases inlet 121.

The interface body 110 may be formed from a soft, flexible material such as silicone, thermoplastic elastomers, or other polymers known in the art. The nasal delivery elements 111 and 112 may be supple and may be formed from a sufficiently thin layer of silicone or other suitable material to achieve this property. The interface body 110 and nasal delivery elements 111, 112 may, for example, be formed from an elastomeric material that is able to confirm to the geometry of a patient's nostril and/or cheek and provide an effective pneumatic seal.

The gases manifold 120 may be formed from a relatively harder material such as Polycarbonate, a High-Density Polyethylene (HDPE) or any other suitable plastics material known in the art. The interface body 110 provides a soft interfacing component to the patient for comfortably delivering the flow of gases through the nasal delivery elements 111 and 112, while the gases manifold 120 fluidly couples the respiratory conduit 300 to the nasal delivery elements 111 and 112 of the interface body 110.

The nasal delivery elements 111 and 112 are substantially hollow.

The first and second nasal delivery elements 111, 112 may have the same shape and configuration as each other, i.e. may be symmetrical. In other configurations, the first and second nasal delivery elements may have a different shape and/or configuration from each other, i.e. may be asymmetrical.

The interface body 110 is shaped to generally follow the contours of a patient's face around the upper lip area. The interface body 110 is moulded or pre-formed to be able to conform to and/or is pliable to adapt, accommodate and/or correspond with the contours of the user's face, in the region of the face where the nasal interface is to be located.

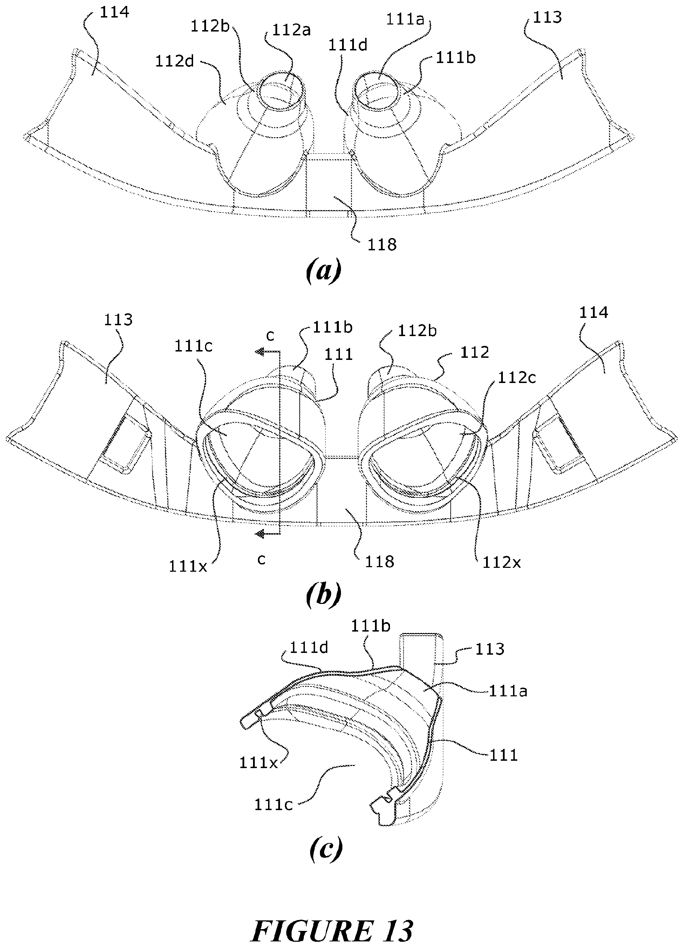

Referring to Figures 13(a)-(c), the interface body 110 comprises a base portion 118 from which the nasal delivery elements 111 and 112 extend.

The base portion 118 is arranged to locate between a patient's face and the gases manifold 120 in use. The base portion 118 may act as a cushion to avoid the gases manifold 120 from touching the patient's face.

In the configuration shown, the interface body 110 comprises two side arms that extend laterally from either side of the base portion 118.

In the configuration shown, the side arms comprise wing portions 113 and 114 extending laterally from either side of the base portion 118. The wing portions 113 and 114 are integrally formed with the base portion 118 but may alternatively be separate parts.

In some configurations, the nasal delivery elements 111, 112 extend generally upwardly and rearwardly from the base portion 118 of the interface body 110.

Adhesive pads (not shown) may be provided on each wing portion 113, 114 to facilitate coupling of the nasal interface 100 to the patient.

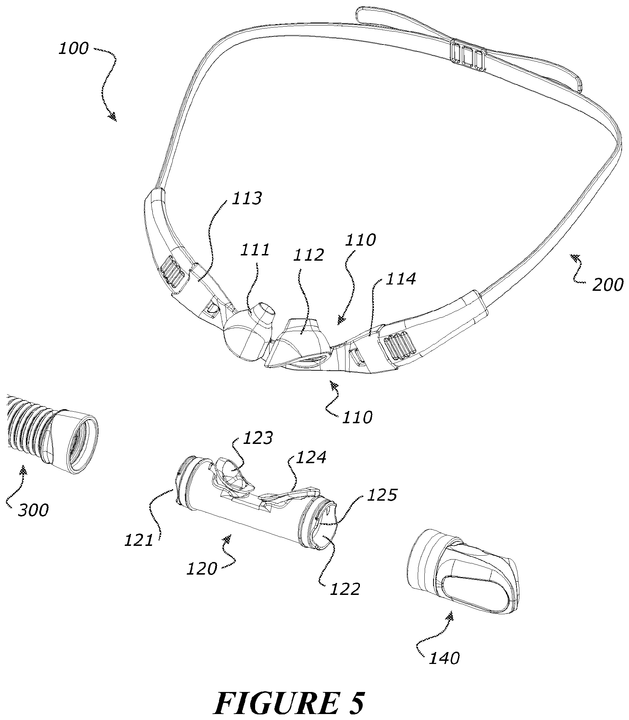

The gases manifold 120 is generally tubular in shape having a gases port 121, 122 at at least one side thereof, and optionally at either side thereof (Figures 5, 11, and 12). At least one of the gases ports 121, 122 may be removably attachable to a respiratory conduit 300, such as via a threaded engagement but alternatively via a snap-fit or any other type of coupling known in the art. That enables the at least one of the gases ports 121, 122 to act as a gases inlet for the gases manifold 120 and thereby for the nasal interface 100. Alternatively, the port 121, 122 may be fixedly coupled or integrally formed with a respiratory conduit 300.

By having the respiratory conduit 300 extending from a side of the gases manifold 120 and thereby from a side of the nasal interface 100, a patient's mouth may be readily accessible while wearing the nasal interface for feeding/eating, drinking, or verbal communication for example.

Flow enters the nasal interface 100 through the gases inlet and travels through the gases manifold 120 in a direction that a substantially transverse to the direction the flow is intended to travel into the first and second nasal delivery elements 111, 112.

The gases inlet is in fluid communication with the respiratory conduit 300.

In some configurations, the respiratory conduit 300 has an internal diameter of between about 12 mm and about 23 mm, optionally more than about 12 mm and up to about 23 mm, optionally more than about 12 mm and up to about 22 mm, optionally more than about 12 mm and up to about 21 mm, optionally more than about 12 mm and up to about 20 mm, optionally more than about 12 mm and up to about 19 mm, optionally more than about 12 mm and up to about 18 mm, optionally between about 13 mm and about 17 mm, optionally between about 14 mm and about 16 mm, optionally about 12 mm, optionally about 13 mm, optionally about 14 mm, optionally about 15 mm, optionally about 16 mm, optionally about 17 mm, optionally about 18 mm, optionally about 19 mm, optionally about 20 mm, optionally about 21 mm, optionally about 22 mm, optionally about 23 mm, or optionally any value between any two of those values.

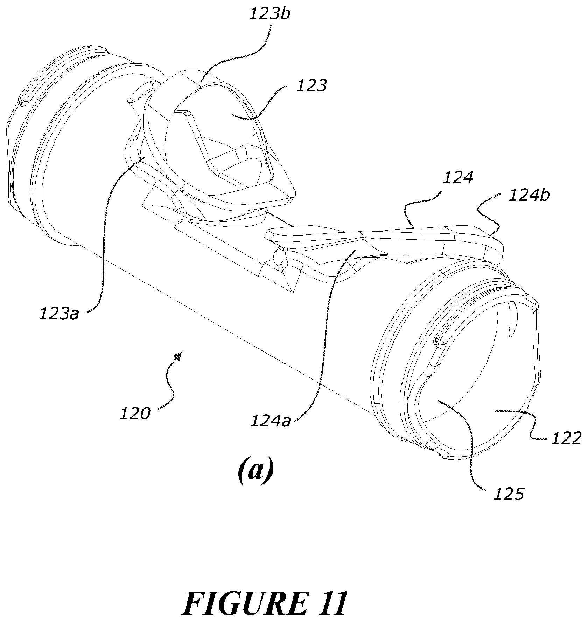

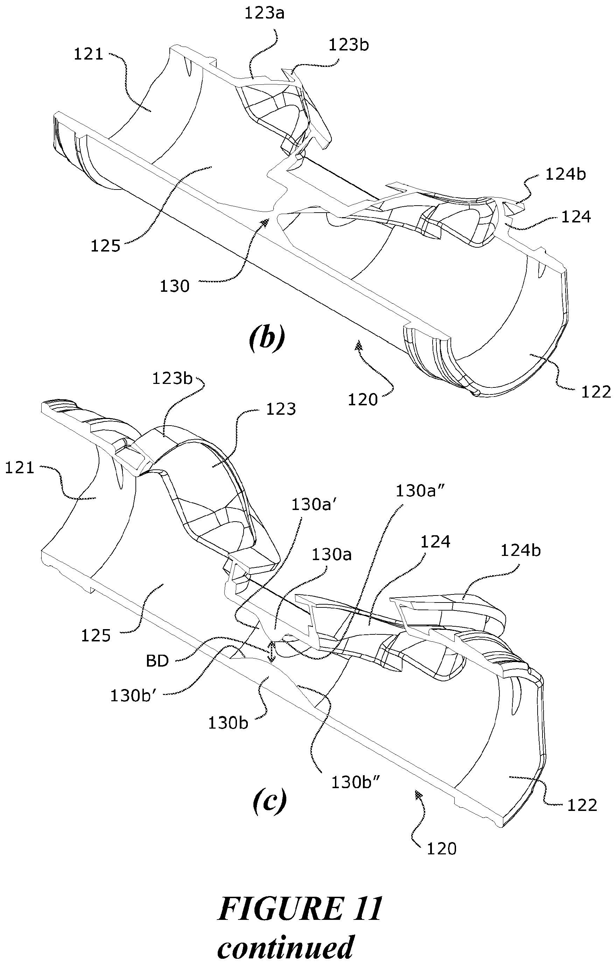

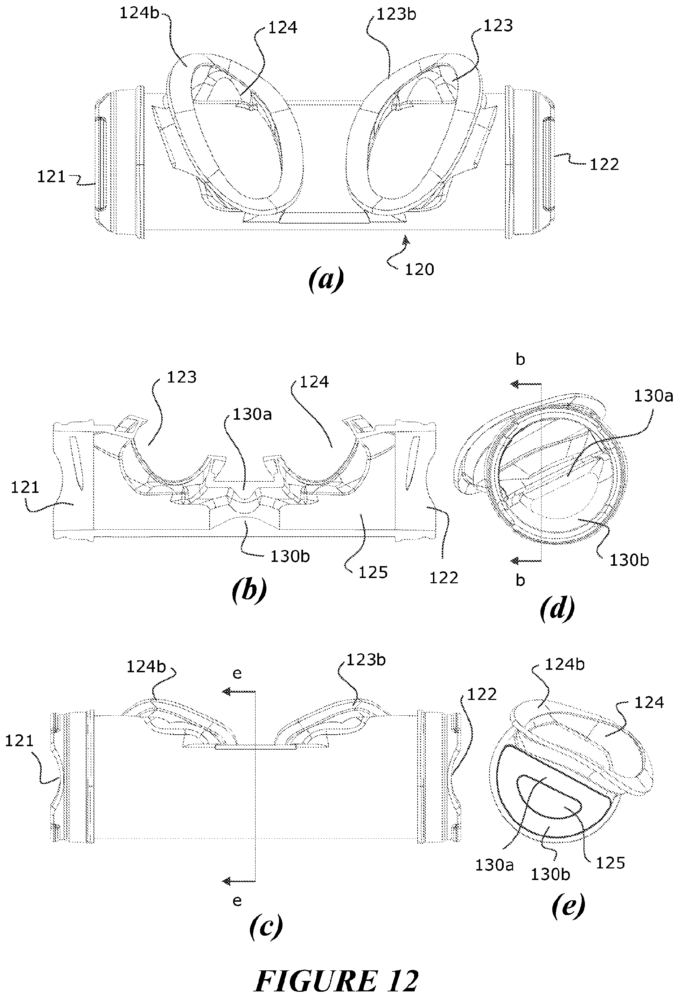

Referring to Figures 11 and 12, a gases flow path is defined by a lumen or flow channel 125 in the gases manifold 120.

The flow channel 125 extends from the gases port 121 at one side of the gases manifold 120, through the gases manifold, to the gases port 122 at the other side of the gases manifold 120.

The flow channel 125 is in fluid communication with a first gases outlet 123 and a second gases outlet 124. The first gases outlet 123 is configured to deliver gases to the first nasal delivery element 111 and the second gases outlet 124 is configured to deliver gases to the second nasal delivery element 112.

The shape of the gases outlets 123, 124 corresponds with and fits with the interface body 110 e.g. with a friction fit or snap fit engagement, such that substantial force, or at least a deliberate force applied by a user or a carer, is required to separate the manifold 120 from the interface body 110.

An effective seal is formed between the gases outlets 123, 124 and the interface body 110 upon engagement of the gases manifold 120 with the interface body 110.

In the configuration shown, each of the gases outlets is provided in a respective outlet portion 123a, 124a of the gases manifold 120.

Each outlet portion 123a, 124a comprises a sealing flange 123b, 124b for engagement with the first and second nasal delivery elements 111, 112.

The sealing flanges 123b, 124b extend transversely outward from an adjacent section of the respective outlet portion 123a, 123b. The sealing flanges 123b, 124b are received in a respective portion 111x, 112x of the nasal delivery elements 111, 112.

In the configuration shown, the sealing flanges 123b, 124b are generally annular in shape, and the respective portions 111x, 112x of the nasal delivery elements comprise annular channels in an inner surface of the nasal delivery elements 111, 112.

In alternative configurations, the sealing flanges 123b, 124b and the respective portions 111x, 112x could have different shapes. For example, they could each comprise one or more discrete members that do not extend around the entire periphery of the outlet portions 123a, 123b and the nasal delivery elements 111, 112.

In the configuration shown, the outlet portions 123a, 124a and the sealing flanges 123b, 124b are received in the interior of the nasal delivery elements 111, 112. In an alternative configuration, that could be reversed so that bases of the nasal delivery elements 111, 112 are received in the interior of the body portions 123a, 124a. In that configuration, the outlet portions 123a, 124a may comprise sealing collars for engagement with the first and second nasal delivery elements. The sealing collars may engage with the exterior of the nasal delivery elements to provide a seal therebetween.

The nasal delivery elements 111, 112 may comprise projections that are received in respective recesses in the sealing collars. The projections and recesses may be generally annular in shape, or could have a different configuration as described above for the sealing flanges 123b, 124b and complementary portions 111x, 112x.

In some configurations, the sealing flanges or collars and complementary portions on the nasal delivery elements additionally act as retention features to maintain the interface body 110 and gases manifold 120 in engagement with each other. In alternative configurations, the interface body 110 and gases manifold 120 may comprise one or more other retention features such as clips or fasteners or the like for example, to maintain the interface body 110 and gases manifold 120 in engagement with each other.

The gases manifold 120 may consist of a single part or may comprise a plurality of components that assemble together. For example, the gases manifold 120 may have a first body portion that provides the gases flow channel 125, and that optionally provides the gases ports 121, 122. The gases manifold 120 may have a second body portion that provides the gases outlets 123, 124. Alternatively, the gases manifold 120 may be a single component. In an alternative configuration, the gases manifold 120 may comprise a single outlet, and the interface body 110 may comprise a single complementary gases entry that couples with the single outlet of the gases manifold 120 and that is in fluid communication with the first and second nasal delivery elements 111, 112 to deliver the gases to the first and second nasal delivery elements 111, 112.

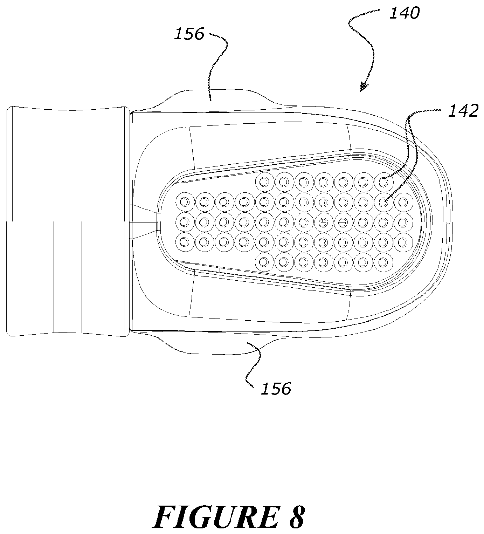

Referring to Figures 1 to 14 and 18, in some configurations a nasal interface 100 of the present disclosure comprises a first nasal delivery element 111 and a second nasal delivery element 112. The first nasal delivery element 111 and the second nasal delivery element 112 are each configured to seal with a respective naris of a patient. The first nasal delivery element is configured to seal with a first naris of the patient and the second nasal delivery element is configured to seal with a second naris of the patient.

In some configurations, the first nasal delivery element 111 and the second nasal delivery element 112 are configured to seal with an entrance to the nares of the patient. In some configurations, the first nasal delivery element 111 and the second nasal delivery element 112 are configured to seal with an interior of the nares of the patient. In some configurations, the first nasal delivery element 111 and the second nasal delivery element 112 are configured to seal with both the entrance to the nares and the interior of the nares of the patient.

The nasal interface comprises a gases manifold 120 comprising a gases inlet 121 for delivery of respiratory gases to the gases manifold. The first nasal delivery element 111 and the second nasal delivery element 112 are in fluid communication with the gases inlet 121 via the gases manifold 120.

The gases inlet 121 is in communication with a single gases entry portion of a gases flow channel 125 of the gases manifold. With this configuration, respiratory gases enter the gases manifold 120 from a single region, for example from a single side, of the gases manifold, and are delivered to the first and second nasal delivery elements 111, 112 from that single region.

The gases flow generally in one direction from the single side of the gases manifold to the opposite side of the gases manifold, in addition to passing through the first and second nasal delivery elements 111, 112.

The gases manifold 120 may comprise a single gases inlet 121.

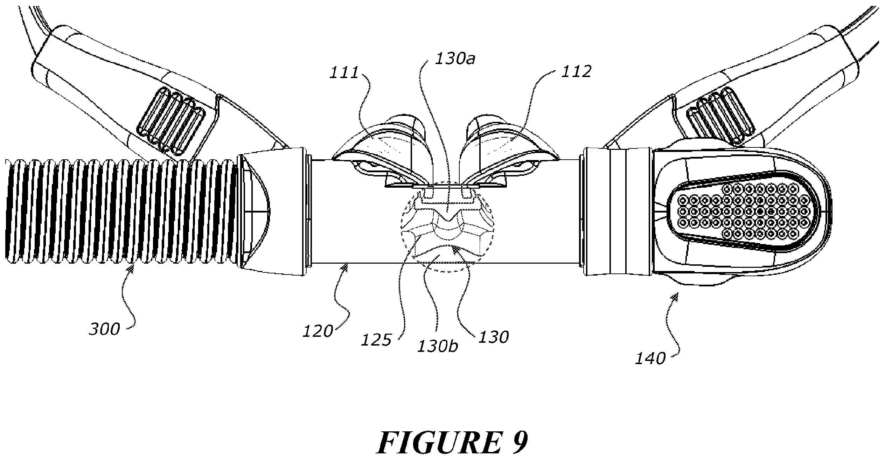

With reference to Figures 9, 10, and 11, the nasal interface comprises a bypass restriction 130 to provide a pressure drop through the nasal interface 100 between the first nasal delivery element 111 and the second nasal delivery element 112 when gases are delivered from the gases inlet 121 to the first nasal delivery element 111 and the second nasal delivery element 112 such that pressure at the first nasal delivery element 111 is higher than pressure at the second nasal delivery element 112.

As used herein, a bypass restriction 130 may be any feature or geometry that provides a pressure drop through the nasal interface 100 between the first nasal delivery element 111 and the second nasal delivery element when gases are delivered from the gases inlet 121 to the first nasal delivery element 111 and the second nasal delivery element 112 such that pressure at the first nasal delivery element 111 is higher than pressure at the second nasal delivery element 112. In some configurations, the bypass restriction 130 may be a physical restriction relative to an adjacent part of the gases flow channel 125, relative to the gases inlet 121, relative to the combined cross-sectional area A₃+ A₄ of the first and second nasal delivery elements 111, 112, and/or relative to any other part of the nasal interface 100.

In some configurations, the bypass restriction 130 may be a flow splitter or flow director.

The pressure drop is such that gases pressure upstream of the bypass restriction will be higher than gases pressure downstream of the bypass restriction.

The pressure at the first nasal delivery element 111 may be at an outlet of the first nasal delivery element and/or along the first nasal delivery element and/or adjacent the first nasal delivery element. The pressure at the second nasal delivery element 112 may be at an outlet of the second nasal delivery element and/or along the second nasal delivery element and/or adjacent the second nasal delivery element.

The pressure drop through the gases manifold 120 may be such that when there is a flow of gases from the gases inlet 121 to the first nasal delivery element 111 and the second nasal delivery element 112, the flow of gases from the gases inlet 121 to the first nasal delivery element 111 is greater than the flow of gases from the gases inlet 121 to the second nasal delivery element 112.

The bypass restriction 130 may restrict the flow of gases through the gases manifold 120 between the first nasal delivery element 111 and the second nasal delivery element 112.

In some configurations, when gases are delivered from the gases inlet 121 to both the first nasal delivery element 111 and the second nasal delivery element 112, the pressure of gases flow at the second nasal delivery element 112 is up to about 1 cmH2O less than the pressure of gases flow at the first nasal delivery element.

The pressure drop caused by the bypass restriction 130 and thereby the pressure differential of gases flow between the first nasal delivery element 111 and the second nasal delivery element 112 will typically be higher during an inspiration phase than during an expiration phase. That is because, when a patient expires gases, more of the expiratory gases will pass through the second nasal delivery element 112 than through the first nasal delivery element 111. For example, during an inspiration phase the pressure of gases flow at the second nasal delivery element 112 may be about 0.6 cmH2O less than the pressure pf gases flow at the first nasal delivery element 111, and during an expiration phase the pressure of gases flow at the second nasal delivery element 112 may be about 0.3 cmH2O less than the pressure of gases flow at the first nasal delivery element 111. The magnitude of the difference between the pressure of gases flow at the first nasal delivery element 111 and the pressure of the gases flow at the second nasal delivery element 112, for a given bypass restriction 130, will be dependent on the set pressure as well as the phase of the breathing cycle.

In some configurations, the nasal interface 100 is configured to achieve a patient pressure at the first and second nasal delivery elements 111, 112 of between about 2 cmH2O and about 30 cmH2O in use, optionally between about 2 cmH2O and about 25 cmH2O in use, optionally between about 2 cmH2O and about 20 cmH2O in use, optionally between about 2 cmH2O and about 15 cmH2O in use, optionally between about 2 cmH2O and about 14 cmH2O in use, optionally between about 2 cmH2O and about 13 cmH2O in use, optionally between about 2 cmH2O and about 12 cmH2O in use, optionally between about 2 cmH2O and about 11 cmH2O in use, optionally between about 2 cmH2O and about 10 cmH2O in use.

The nasal interface 100 may be configured such that pressure at the first nasal delivery element 111 is higher than pressure at the second nasal delivery element 112 during both the inspiration phase and the expiration phase.

A set pressure may be delivered to the second nasal delivery element 112 and a higher pressure may be delivered to the first nasal delivery element 111.

As the set pressure increases, the pressure differential between the first nasal delivery element and the second nasal delivery element increases, with that increase providing increased dead space clearance or washout.

In some configurations, the pressure differential between the first nasal delivery element 111 and the second nasal delivery element 112 is configured to provide an asymmetric flow through upper airways of a patient of at least about 1 liter per minute (lpm), optionally between about 1 Ipm and about 2 Ipm, optionally between about 1 lpm and about 5 lpm. In some configurations, the asymmetric flow may be less than 1 lpm.

The nasal interface 100 is configured to cause an asymmetrical flow of gases at a patient's nares through the first nasal delivery element 111 and the second nasal delivery element 112, due to the pressure drop through the gases manifold and the resulting pressure differential between the first nasal delivery element 111 and the second nasal delivery element 112. The resulting asymmetrical flow of gases can provide improved dead space clearance.

In some configurations, the gases manifold 120 comprises a gases flow channel 125 in the gases manifold 120, and the bypass restriction 130 provides a reduced cross-sectional area of a portion of the gases flow channel 125.

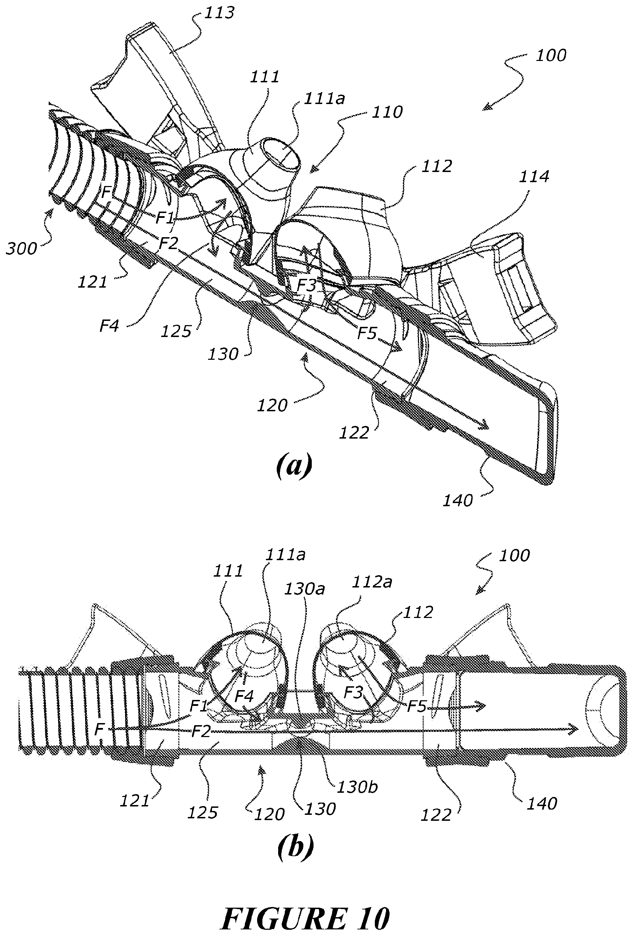

This is illustrated in Figure 10 for example, where it can be seen that the spacing through the gases flow channel 125 in the region of the bypass restriction 130 is significantly reduced compared to the spacing through the gases flow channel 125 on either side of the bypass restriction 130.

The portion of the gases flow channel 125 that is restricted may be between the first nasal delivery element 111 and the second nasal delivery element 112 and/or may be adjacent the second nasal delivery element 112. In particular, the portion of the gases flow channel that is restricted may be between the first gases outlet 123 and a second gases outlet 124 of the gases manifold.

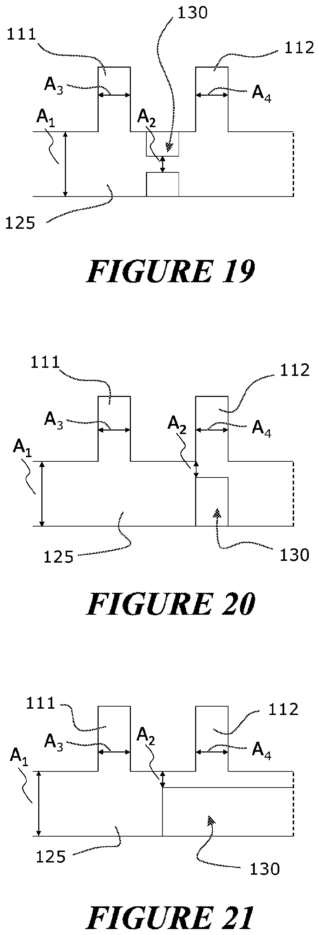

Figure 19 schematically shows the configuration of the nasal interface of Figures 1 to 18, but additionally showing relative cross-sectional areas of the bypass restriction region (area A₂) and an adjacent or main part of the gases flow channel 125 (area A₁). The bypass restriction 130 is shown in this configuration as being between the first nasal delivery element 111 and the second nasal delivery element 112.

In some configurations, the volume of the plenums at the base of the first and second nasal delivery elements 111, 112 are substantially the same. The bypass restriction 130 may be a localised restriction.

Figure 20 schematically shows an alternative configuration of the nasal interface where the bypass restriction 130 is adjacent to the second nasal delivery element 112. The bypass restriction 130 is positioned opposite the base of the second nasal delivery element 112.

Figure 21 schematically shows an alternative configuration of the nasal interface where the bypass restriction 130 is both between the first and second nasal delivery element 111 and the second nasal delivery element 112, but is also adjacent to the second nasal delivery element. The bypass restriction 130 is partly opposite the base of the second nasal delivery element.

The volume in the gases flow channel 125 at the base of the second nasal delivery element 112 is less than the volume in the gases flow channel at the base of the first nasal delivery element 111.

The bypass restriction 130 may extend into the gases flow channel in one or more directions (i.e. from one or more wall portions of the gases flow channel 125). In some configurations, the bypass restriction 130 may extend into the gases flow channel in one direction - e.g. in an upward direction, a downward direction, a forward direction, or a rearward direction. In some configurations, the bypass restriction 130 may extend into the gases flow channel in more than one direction - e.g. in more than one of an upward direction, a downward direction, a forward direction, or a rearward direction.

The bypass restriction 130 may comprise at least one protrusion 130a, 130b extending into the gases flow channel 135. In some configurations, the bypass restriction 130 may comprise a plurality of protrusions extending into the gases flow channel 125.

For example, the bypass restriction 130 may comprise diametrically opposed protrusions that extend into the flow channel.

In some configurations, the gases manifold 120 comprises a proximal bypass protrusion 130a that is proximal to the first and second nasal delivery elements 111, 112 and/or a distal bypass protrusion 130b that is distal from the first and second nasal delivery elements 111, 112.

In the configuration shown, the gases manifold 120 comprises both a proximal bypass protrusion 130a and a distal bypass protrusion 130b which in combination define a predetermined bypass dimension BD for the restricted flow of gases through the gases manifold 120 between the first nasal delivery element 111 and the second nasal delivery element 112.

The predetermined bypass dimension BD will generally be substantially smaller than a dimension of an adjacent or main part of the gases flow channel 125.

The predetermined bypass dimension BD may relate to the cross-sectional area A₂ outlined below.

When a plurality of protrusions are provided, they may be discrete protrusions, semi-continuous, or continuous. Figures 11(a) and 11(b) for example show that a portion of the bypass restriction extends around substantially the entire periphery of the gases flow channel 125 to form the upper and lower bypass protrusions 130a, 130b.

With reference to Figure 11(c), the bypass restriction 130 comprises an angled leading edge 130a', 130b' and an angled trailing edge 130a", 130b" that define a converging and diverging bypass restriction in a direction of gases flow through the gases manifold from the first nasal delivery element 111 to the second nasal delivery element 112.

The angled leading edge 130a', 130b' and/or the angled trailing edge 130a", 130b" may be substantially straight or planar, or alternatively may be curved. If curved, the curved surfaces may be convex so as to be bowed in a direction toward a centre of the gases flow channel 125 or may be concave so as to be bowed in a direction away from a centre of the gases flow channel 125.

Any suitable combination of shapes could be provided. For example, at least one of the leading edges 130a', 130b' may be one of straight, concave, or convex, and at least one of the trailing edges 130a", 130b" may be another one of straight, concave, or convex.

The leading edge 130a', 130b' and the trailing edge 130a", 130b" may have the same configuration as each other or may have different configurations from each other. For example, the gradient and/or curvature of the upstream side may differ from the gradient and/or curvature of the downstream side.

When a plurality of projections are provided for the bypass restriction 130, the projections may be the same shape and configuration as each other or may have a different shape and configuration from each other.

In the configuration shown, the upper projection 130a has a shorter width in a direction along the gases flow channel than the lower projection 130b. In alternative configurations, the upper projection 130a may be the same width as the lower projection 130b or may have a shorter width than the lower projection.

In the configuration shown, the upper projection 130a extends substantially the same distance into the gases flow channel 125 as the lower projection 130b. In alternative configurations, the upper projection 130a may extend further into the gases flow channel 125 than the lower projection 130b or the lower projection 130b may extend further into the gases flow channel 125 than the upper projection 130a.

The bypass restriction 130 may be integrally formed with the gases manifold 120. Alternatively, the bypass restriction 130 may comprise an insert for attachment to the gases manifold 120. For example, the bypass restriction may be formed as a sleeve or plug. The sleeve or plug may be attached to the gases manifold in any suitable manner. For example, the sleeve or plug may be press-fit, screwed, fastened, or the like into the gases flow channel 125 of the gases manifold.

The bypass restriction 130 may be provide by the gases manifold 120, by the base portion 118 of the interface body, or by both the gases manifold 120 and the base portion 118 of the interface body.

The bypass restriction 130 is configured to provide the reduced second cross-sectional area A₂ in the gases flow channel 125 compared to the first cross-sectional area A₁ of an adjacent or main part of the gases flow channel 125.

In some configurations, the second cross-sectional area A₂ may be between about 10% and about 40% of the first cross-sectional area A₁. In some configurations, the second cross-sectional area A₂ may be between about 10% and about 35% of the first cross-sectional area A₁., optionally between about 10% and about 30% of the first cross-sectional area A₁, optionally between about 10% and about 25% of the first cross-sectional area A₁, and optionally about 17.5% of the first cross-sectional area A₁. In some configurations, the second cross-sectional area A₂ may be about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, or about 40% of the first cross-sectional area A₁, or may be any percentage between any two of those percentages.

In one exemplary configuration, the first cross-sectional area may be about 200 mm² (corresponding to a radius of about 8 mm), and the second cross sectional area may be between about 20 mm² and about 80 mm², optionally between about 20 mm² and about 70 mm², optionally between about 20 mm² and about 60 mm², optionally between about 20 mm² and about 50 mm², optionally between about 30 mm² and about 40 mm², and optionally about 35 mm².

The predetermined bypass dimension BD may, for example, be between about 5 mm and about 10 mm, optionally between about 5 mm and about 9.5 mm, optionally between about 5 mm and about 8.75 mm, optionally between about 5 mm and about 8 mm², optionally between about 6 mm and about 7 mm, optionally between about 6.5 mm and about 7 mm, and optionally about 6.7 mm.

In some configurations, the nasal interface 100 comprises an interface body 110 comprising the first nasal delivery element 111 and the second nasal delivery element 112.

In some configurations, the gases manifold 120 is integral with the interface body 110 or is separate from and couplable with the interface body 110.

The first nasal delivery element 111 has a first outlet 111a defined by an opening at its tip or terminal end 111b for delivery of gases from the first nasal delivery element 111. Gases delivered through the first nasal delivery element 111 exit the first nasal delivery element 111 via the first outlet 111a.

The second nasal delivery element 112 has a second outlet 112a defined by an opening at its tip or terminal end 112b for delivery of gases from the second nasal delivery element 112. Gases delivered through the second nasal delivery element 112 exit the second nasal delivery element via the second outlet 112a.

The first and second nasal delivery elements 111, 112 may have any suitable shape to seal with the nares of the patient. For example, in one configuration, the first and second nasal delivery elements 111, 112 may be substantially tubular and may be sized to be larger than the nares of a patient, but may be supple or flexible to deform and seal with the nares upon insertion into the nares. In some configurations, the nasal delivery elements 111, 112 are more supple or flexible than the body portion 118.

As another example, and as shown, the first and second nasal delivery elements 111, 112 may comprise nostril locators or pillows to seal with the nares of the patient.

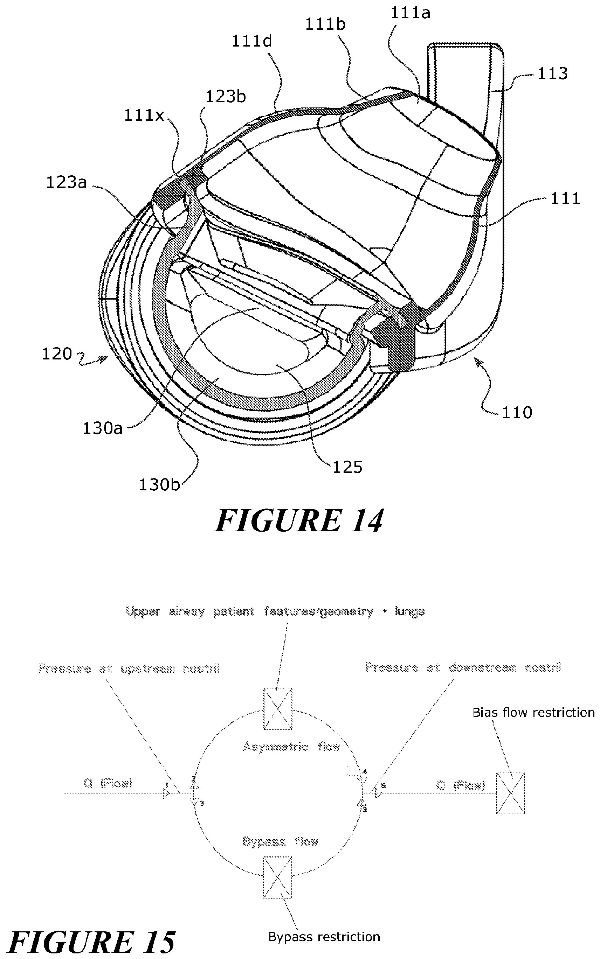

In the illustrated configuration, as shown in Figures 13 and 14 for example, each pillow can generally taper such that it narrows toward its respective outlet 111a, 112a at the tip or terminal end 111b, 112b thereof. As such, the proximal opening 111a, 112a may have a smaller diameter or transverse dimension than a distal opening 111c, 112c at a base of the pillow. Generally speaking, the pillows can taper in a proximal direction toward their tip or terminal ends 111b, 112b.

In the configuration shown, the tip or terminal ends 111b, 112b of the pillows configured to be received in the nares of a patient, while enlarged regions 111d, 112d of the pillows adjacent to the tip or terminal ends 111b, 112b are configured to seal against the entrance to the nares. In other configurations, the tip or terminal ends 111b, 112b and part of the enlarged regions 111d, 112d may be configured to be received in the nares to seal therewith.

The pillows may be supple or flexible to deform and seal with the nares upon insertion into the nares or contact with the nares. In some configurations, the pillows are more supple or flexible than the body portion 118.

The pillows also desirably are sufficiently stiff to reduce the likelihood of ballooning or being insufficiently self-supporting to provide an indication to the user of correct location and orientation of the nasal interface 100 relative to the face. The pillows may have sufficient stiffness to inhibit or prevent significant collapse in response to positioning of the pillows relative to the patient's nares. In some configurations, the pillows can have a thickness of about 0.7 mm with some variation being possible slightly higher and lower keeping in mind a desire to reduce user discomfort while still assisting with nasal interface positioning.

The pillows may comprise one or more stiffening elements or features to inhibit collapse of the pillows.

The first and second nasal delivery elements 111, 112 may be movable relative to the body portion 118 to enable the angle and positioning of the nasal delivery elements 111, 112 to be adjusted in response to contact with the patient's nares.

The pillows and nasal interface may have any one or more of the features described in relation to the nostril locators of US patent no. 10,918,818.

If any leakage occurs between the nasal delivery elements 111, 112 and the patient's nares, that leakage will be minimal and can be compensated for or controlled by adjusting the therapy gases flow rate.

The nasal interface 100 is configured to cause an asymmetrical flow of gases at a patient's nares, due to the pressure drop through the gases flow channel 125 of the nasal interface 100.

The nasal interface 100 may be configured such that such that about 10 Ipm to about 50 lpm is delivered out of the nasal interface 100 through the nasal delivery elements 111, 112. The proportion that is delivered through each nasal delivery element will vary depending on the patient, pressure differential, and stage of the breath cycle.

Having a differential of flow rates between the nasal delivery element 111, 112 can provide the benefits of asymmetrical flow described below.

In some configurations, there is a relatively constant pressure differential between the nasal delivery elements 111, 112 and a resulting relatively constant asymmetric flow through the nasal delivery elements 111, 112. In some configurations, the pressure differential and the resulting asymmetric flow may vary. As long as there is a pressure drop through the gases manifold 120 for at least some portion of the breath cycle, asymmetric flow will occur.

The proportion of the total volumetric flow rate being delivered through each prong 111, 112 can be determined by delivering gases with a known volumetric flow rate to the gases inlet 121 of the nasal interface 100 while the nasal interface is not applied to a patient's nares. The volumetric flow rate exiting each outlet 111a, 112a can be measured by a suitable flow meter or sensor to determine the proportion of the total volumetric flow rate of gases flow into the gases inlet 121 that is exiting the outlet 111a, 112a of each nasal delivery element 111, 112.

The nasal interface 100 comprises a bias flow restriction 140 for a flow of gases out of the nasal interface 100, and optionally for a flow of gases out of the gases manifold 120.

With reference to Figures 1-5, 16, and 18, the bias flow restriction 140 is in fluid communication with the gases manifold 120 and, more particularly, with the gases port 122 of the gases manifold 120.

The bias flow restriction 140 is positioned downstream in the patient interface 100 from the first and second nasal delivery elements 111, 112 and opposite to the gases port 121, so gases may pass from the first and second nasal delivery elements 111, 112 and out of the nasal interface via the bias flow restriction 140. Some of the gases that enter the gases inlet port 121 may travel out of the bias flow restriction 140 without passing through the first and second nasal delivery elements 111, 112. The gases that travel out of the nasal interface via the bias flow restriction may comprise expiratory gases and may further comprise some inlet gases that have not passed through the first and second nasal delivery elements 111, 112.

The bias flow restriction 140 allows for the provision of a pressure therapy to the nares of a patient. The bias flow restriction 140 enables a restricted flow of gases through the bias flow restriction out of the nasal interface 100. If there was no bias flow restriction 140 and the gases port 122 was closed, all exhaled gases would be rebreathed. If there was no bias flow restriction and the gases port 122 was open, the respiratory therapy apparatus would not be able to apply a pressure through the nasal interface.

The open area for gases flow through the bias flow restriction may be selected to provide sufficient area for bias flow while minimizing noise from the bias flow. In one exemplary configuration, when a patient pressure about 10 cmH2O is provided, gases flow through the nasal interface 100 may be about 25-45 Ipm, and the open area for gases flow through the bias flow restriction may be between about 10 mm² and about 15 mm². However, this is one example only, and these values may vary depending on system parameters and patient requirements. In another example, the open area for gases flow through the bias flow restriction may be between about 10 mm² and about 30 mm², optionally between about 25 mm² and about 30 mm², and optionally about 27.5 mm².

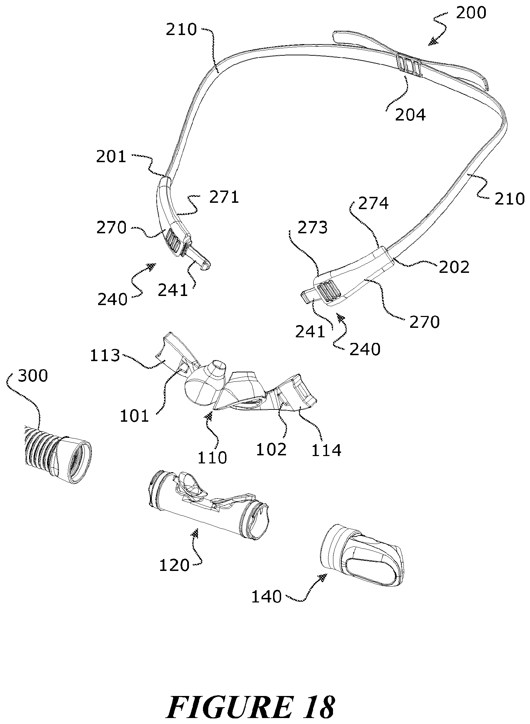

The gases manifold 120 may comprise the bias flow restriction 140 or may be coupled to the bias flow restriction 140. In an alternative configuration illustrated in Figure 18, the bias flow restriction 140 may be in fluid communication with the gases manifold 120 but positioned remotely from the gases manifold 120. In this alternative configuration, an expiratory gases conduit 160 is coupled to the gases port 122 of the gases manifold 120 and to the bias flow restriction 140. The expiratory gases conduit 160 can have any suitable length. This configuration enables expiratory gases and any inlet gases that bypass the first and second nasal delivery elements 111, 112 to be vented through the bias flow restriction 140 at a location spaced apart from the patient.

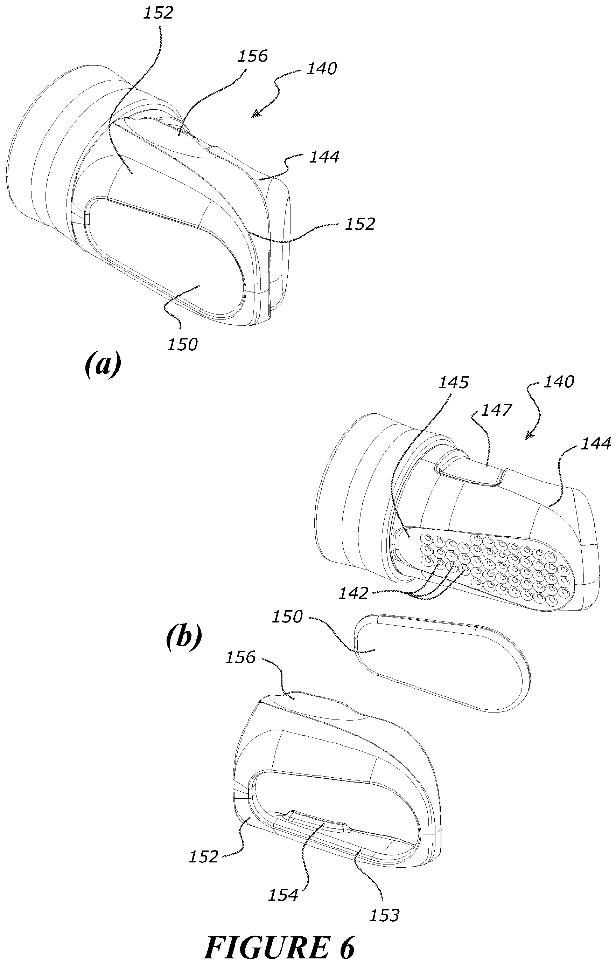

Referring to Figures 6-9, the bias flow restriction 140 comprises one or more gases outlets for the flow of gases from the nasal interface 100, and optionally from the gases manifold 120, to an ambient environment.

The one or more gases outlets may comprise one or more apertures. In the configuration shown, the one or more gases outlets comprises a plurality of apertures 142 for the flow of gases from the nasal interface 100, and optionally from the gases manifold 120, to the ambient environment.

The plurality of apertures 142 may be provided in any suitable arrangement or array. For example, in the configuration shown, the plurality of apertures 142 are provided in an array of four long rows and two outer short rows. However, any other suitable arrangement could be provided, such as a larger or smaller number of rows of apertures, a larger or smaller number of apertures in each row, or a random arrangement of apertures.

The bias flow restriction 140 may comprise 1, 2, 3, 4, 5, 10, 15, 20, 25, 30, 35, 40, 45, 50, or more apertures.

Additionally, or alternatively, the one or more gases outlets may comprise one or more slots which may be straight, curved, wavy, sinuous, or any other suitable shape.

The one or more gases outlets will typically have outlet dimension(s) that is/are substantially smaller than the size of a gases inlet into the bias flow restriction 140, to create a pressure drop or resistance to flow out of the one or more gases outlets. The pressure drop is such that gases pressure upstream of the one or more gases outlets will be higher than gases pressure downstream of the one or more gases outlets.

However, when a plurality of outlets are provided, the sum of the outlet dimensions may approach the size of the gases inlet.

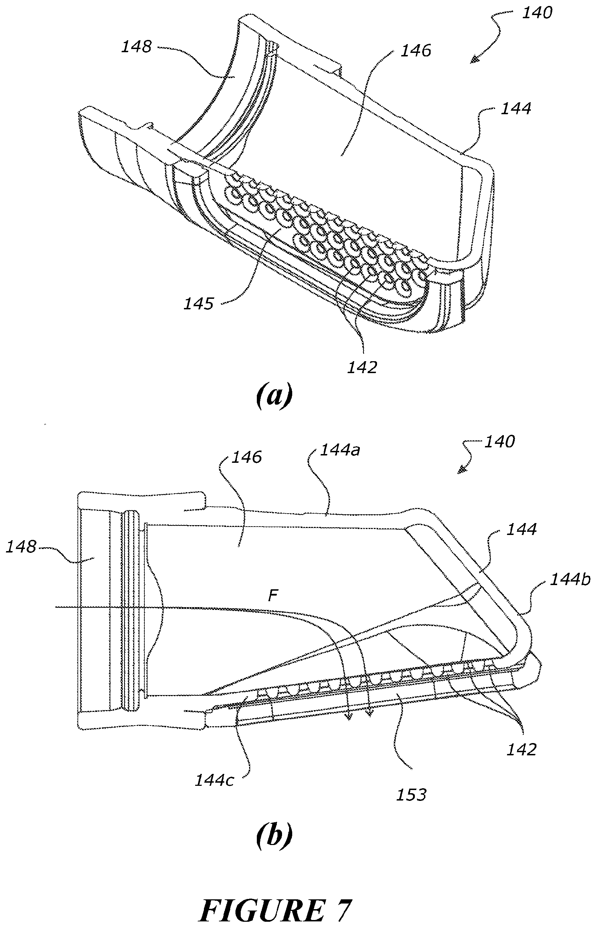

In some configurations, the gases inlet 148 and the one or more gases outlets are arranged in the bias flow restriction 140 so that gases flow F needs to undertake a change in direction between entering the bias flow restriction and exiting the bias flow restriction. This is represented by arrow F in Figure 7 for example.

In some configurations, the one or more gases outlets is/are provided in a restriction component body 144. The restriction component body 144 defines a body gases flow passage 146 that is in fluid communication with a body gases inlet 148. The one or more gases outlets is/are in fluid communication with the body gases flow passage 146 such that gases pass from the gases port 122 of the gases manifold, into the body gases flow passage 146, and out of the one or more gases outlets (e.g. the apertures 142).

As shown in Figure 7(b) for example, the restriction component body 144 may have a tapered configuration in which the body gases flow passage 146 becomes smaller more distal from the body gases inlet 148 than proximal to the body gases inlet 148. A ceiling, an end wall 144b, and/or a wall 144c of the body that contains the one or more gases outlets may be angled so as to be non-parallel and non-perpendicular relative to each other, to encourage flow from the body gases inlet 148 to pass through the one or more gases outlets.

In some configurations, the bias flow restriction 140 is configured to direct the flow of gases out of the bias flow restriction away from a patent's face. In the configuration shown, the bias flow restriction 140 is configured to direct the flow of gases at least partly in a forward direction and in some configurations entirely in a forward direction away from the patient's face.

The bias flow restriction 140 enables the venting of carbon dioxide (CO₂) via the use of the one or more gases outlets. In the embodiment illustrated, the nasal interface 100 has aperture(s) 142 for expelling gases from inside the nasal interface 100 to the environment. The aperture(s) 142 or other openings can help expel carbon dioxide gases from the user to reduce the rebreathing of the carbon dioxide gases.

The one or more gases outlets create a controlled or known leak to enable the exhausting of the user's exhaled carbon dioxide gases. There may be a performance trade-off between the location of the one or more openings (relative to the patient's nose) and the amount of bias flow required. As used herein, bias flow refers to the flow of gases to the environment through the bias flow restriction 140. The flow rate of the bias flow and the design geometry of the one or more openings can have an effect on the noise level and draft that the bias flow produces, as well as the amount of entrainment that the exiting gas flow may cause.

The one or more gases outlets may comprise a plurality of through holes 142 that expel gases from the nasal interface. In other configurations, the gases outlets can be slits or large openings instead of or in addition to small through holes. In some configurations, the gases outlets can be disposed on other portions of the interface. Generally, relatively smaller hole sizes produce less airflow noises compared to a larger hole size given the same flow velocity through both hole sizes. The plurality of holes helps reduce airflow noises compared to having one or a few holes with the same vent area when expelling a given volume of gas.

The one or more gases outlets may have any one or more of the features or functionality described for the vents in US patent no. 10,898,866.

The bias flow restriction 140 may comprise an optional filter or diffuser to filter or diffuse gases flowing through the one or more gases outlets, e.g. through the aperture(s).

The filter may mitigate respiratory contaminants being released through the bias flow restriction.

The diffuser may diffuse gases existing the bias flow restriction to reduce noise.

Figure 6 shows a filter or diffuser member 150 that is configured to cover the at least one or more gases outlets to filter or diffuse gases as they exit the one or more gases outlets. The filter or diffuser member 150 may comprise any suitable material, such as one or more of non-woven fibrous material (including polymer fibres), open cell foam, sintered polymer.

In some configurations, the restriction component body 144 comprises a filter or diffuser recess 145 to receive the filter or diffuser member 150.

The bias flow restriction 140 may comprise a shroud 152 that is configured to attach to the restriction component body 144 and to maintain the filter or diffuser member 150 in place over the one or more gases outlets.

The shroud 152 comprises an aperture 153 that is at least the size of the at least one opening of the restriction component body 144.

The shroud 152 may carry the filter or diffuser member 150 in the aperture 153 or the filter or diffuser member 150 may be sandwiched between the shroud 152 and the recess 145.

The shroud 152 may be removably attachable to the restriction component body 144 to enable the filter or diffuser member 150 to be cleaned or replaced.

The shroud 152 may attach to the restriction component body 144 by any suitable arrangement, such as clip(s), fastener(s) or the like. In the configuration shown, the shroud 152 comprises two inwardly directed engagement components 154 that are a snap fit into complementary engagement recess(es) 147 on the restriction component body 144.

The shroud may comprise one or more gripping portions 156 to enable the engagement components 154 to be released from the recess(es) 147 to remove the shroud 152 from the restriction component body 144. In the configuration shown, the gripping portion(s) 156 comprise an outward projection to enable the user to apply force in an outward and downward direction to force the engagement component(s) out of engagement from the restriction component body 152, but any other suitable configuration could be used.

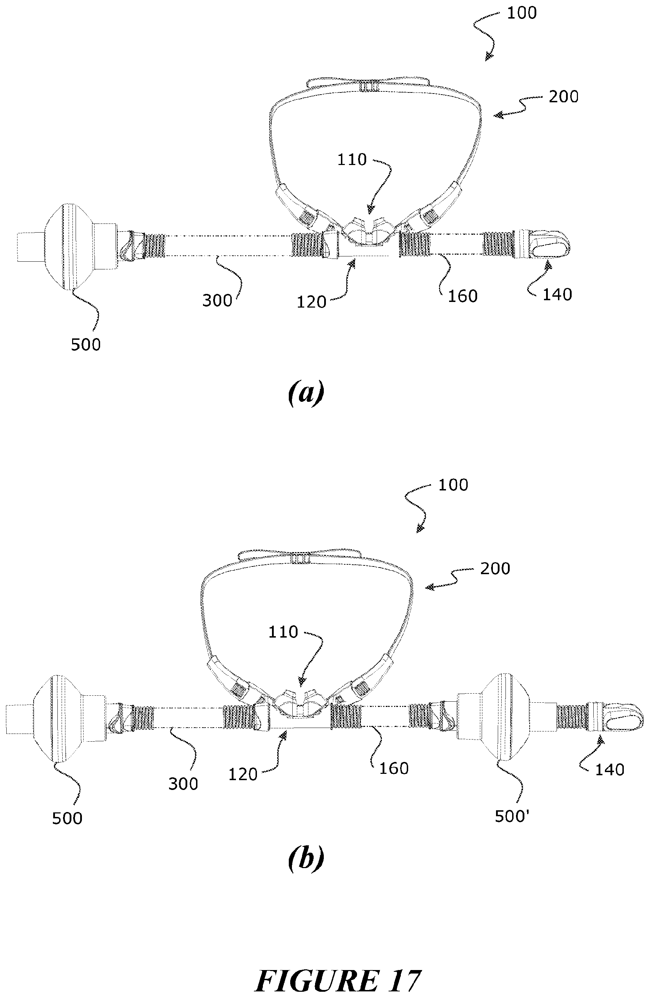

In some configurations and as shown in Figure 17(b), a filter unit 500' may be provided between the gases manifold 120 and the bias flow restriction 140. The filter unit 500' may have any one of more of the features described herein for the filter unit 500.

In some configurations a nasal interface 100 of the present disclosure comprises a first nasal delivery element 111 and a second nasal delivery element 112, wherein the first nasal delivery element 111 and the second nasal delivery element 112 are each configured to seal with a respective naris of a patient, and a gases manifold 120 comprising a gases inlet 121 for delivery of respiratory gases to the gases manifold, wherein the first nasal delivery element 111 and the second nasal delivery element 112 are in fluid communication with the gases inlet 121 via the gases manifold 120, wherein the first nasal delivery element 111 is proximal to the gases inlet 121 and the second nasal delivery element 112 is distal from the gases inlet 121, wherein the nasal interface 100 is configured to create a pressure differential between the first nasal delivery element 111 and the second nasal delivery element 112 when gases are delivered from the gases inlet 121 to both the first nasal delivery element 111 and the second nasal delivery element 112 such that pressure at the first nasal delivery element 111 is higher than pressure at the second nasal delivery element 112.

In some configurations, the pressure differential is such that when there is a flow of gases from the gases inlet 121 to the first nasal delivery element 111 and the second nasal delivery element 112, the flow of gases from the gases inlet 121 to the first nasal delivery element 111 is greater than the flow of gases from the gases inlet 121 to the second nasal delivery element 112.

In some configurations, the gases inlet 121 is in fluid communication with the respiratory conduit 300.

In some configurations, when gases are delivered from the gases inlet to both the first nasal delivery element and the second nasal delivery element, the pressure of gases flow at the second nasal delivery element 112 is up to about 1 cmH2O less than the pressure of gases flow at the first nasal delivery element 111.

For example, the pressure of gases flow at the second nasal delivery element 112 may be about 0.1 cmH2O, about 0.2 cmH2O, about 0.3 cmH2O, about 0.4 cmH2O, about 0.5 cmH2O, about 0.6 cmH2O, about 0.7 cmH2O, about 0.8 cm H2O, about 0.9 cmH2O, or about 1 cmH2O less than the pressure of gases flow at the first nasal delivery element 111, or the difference may be any value between any two of those values.

The pressure differential of gases flow between the first nasal delivery element and the second nasal delivery element may be higher during an inspiration phase than during an expiration phase.

The nasal interface may be configured to achieve a patient pressure at the first and second nasal delivery elements of between about 2 cmH2O and about 30 cmH2O in use, optionally between about 2 cmH2O and about 25 cmH2O in use, optionally between about 2 cmH2O and about 20 cmH2O in use, optionally between about 2 cmH2O and about 15 cmH2O in use, optionally between about 2 cmH2O and about 14 cmH2O in use, optionally between about 2 cmH2O and about 13 cmH2O in use, optionally between about 2 cmH2O and about 12 cmH2O in use, optionally between about 2 cmH2O and about 11 cmH2O in use, optionally between about 2 cmH2O and about 10 cmH2O in use.

In some configurations, the pressure differential between the first nasal delivery element 111 and the second nasal delivery element 112 is configured to provide an asymmetric flow through upper airways of a patient of between about 1 liter per minute (lpm) and about 5 lpm.

For example, the asymmetric flow through the upper airways of the patient may be about 1 lpm, about 1.25 Ipm, about 1.5 Ipm, about 1.75 Ipm, about 2 Ipm, about 2.25 Ipm, about 2.5 Ipm, about 2.75 Ipm, about 3 Ipm, about 3.25 Ipm, about 3.5 Ipm, about 3.75 Ipm, about 4 Ipm, about 4.25 Ipm, about 4.5 Ipm, about 4.75 Ipm, about 5 Ipm, or may be any value between any two of those values.

The asymmetric flow promotes clearing of CO₂ from anatomical dead space of the patient.

As outlined above, the interface body 110 may be engageable with the gases manifold 120. Accordingly, in some configurations a nasal interface 100 of the present disclosure comprises an interface body 110 part comprising a first nasal delivery element 111 and a second nasal delivery element 112, wherein the first nasal delivery element and the second nasal delivery element are each configured to seal with a respective naris of a patient. The nasal interface 100 of the present disclosure further comprises a gases manifold 120 part comprising a gases inlet 121 for delivery of respiratory gases to the gases manifold part. The interface body 110 part is engageable with the gases manifold 120 part to bring the first nasal delivery element 111 and the second nasal delivery element 112 into fluid communication with the gases inlet 121 such that the first nasal delivery element 111 is more proximal the gases inlet 121 and the second nasal delivery element 112 is more distal the gases inlet 121. The nasal interface 100 comprises at least one gases flow restriction 130 to gases flow through the nasal interface, such that when gases are delivered from the gases inlet 121 to the first nasal delivery element 111 and the second nasal delivery element 112, pressure at the first nasal delivery element is higher than pressure at the second nasal delivery element.

The at least one flow restriction may comprise a bypass restriction. The bypass restriction may have any one or more of the features and functionality described herein for the bypass restriction 130.

In some configurations, the nasal interface further comprises a bias flow restriction. The bias flow restriction may have any one or more of the features and functionality described herein for the bias flow restriction 140.

Therefore, the nasal interface described herein may comprise a bypass restriction, a bias flow restriction, or may comprise both a bypass restriction and a bias flow restriction.

By providing asymmetrical flow, use of the nasal interfaces 100 of the present disclosure may provide a reduction of dead space (i.e. the volume of air not involved in the gases exchange within the lungs) compared to conventional continuous positive airway pressure (CPAP) therapy. It is understood that within the upper airway of the patient, some proportion of the gas moves in a unidirectional manner, flowing in one nostril and out the other, reducing the upper airway dead space. This may be most notable at higher set pressures which lead to an increase in the asymmetrical flow and hence an increase in the dead space clearance.

The bypass restriction 130 promotes asymmetrical flow. The bias restriction 140, in combination with the sealing nasal elements 111, 112, allows for provision of a CPAP-style therapy. The nasal interface 100 enables CPAP with increased dead space clearance. The bypass restriction 130 enables the dead space clearance. The sealing nasal delivery elements 111, 112 enable the CPAP therapy.

Inspiratory and expiratory flows will be present in both nostrils. However, the flow is a partial unidirectional flow in which a greater proportion of the inspiratory flow will be through the nostril that is closes to the gases inlet 121 and thereby to the flow source.

The nasal interface 100 may be used for a pressure-controlled therapy, but with higher humidity than traditional CPAP therapy. The higher humidity is believed to beneficially work in conjunction with the increased dead space clearance.

In some configurations, the nasal interface 100 may be suitable for use in, or may be used in, pressure-controlled therapy with a therapy pressure of between about 2 cmH2O and about 10 cmH2O, depending on patient and therapy requirements.

For example, the nasal interface may be suitable for use in, or may be used in, pressure-controlled therapy with a therapy pressure of about 2 cmH2O, 2.5 cmH2O, 3 cmH2O, 3.5 cmH2O, 4 cmH2O, about 4.5 cmH2O, about 5 cmH2O, about 5.5 cmH2O, about 6 cmH2O, about 6.5 cmH2O, about 7 cmH2O, about 7.5 cmH2O, about 8 cmH2O, about 8.5 cmH2O, about 9 cmH2O, about 9.5 cmH2O, or about 10 cmH2O.

The pressure may be set or controlled by the respiratory therapy system, an example of which is described below.

To maintain a desired pressure, a flow of gases is provided and the flow rate will depend on the phase of the breath cycle and the geometry of the bias flow restriction 140, amongst other factors.

Figures 10(a) and (b) show the gases flow through the nasal interface 100. As the flow of gases F enters the flow channel 125 of the gases manifold 120, a portion F1 of the flow will proceed through the upstream first nasal delivery element 111 and through the patient's upper airway. A portion F2 of the flow will proceed past the bypass restriction 130. The portion F2 of the flow that proceeds past the bypass restriction 130 enables flow F3 through the downstream second nasal delivery element 111 so inspiration can occur through both nasal delivery elements 111, 112 (presuming neither nostril is blocked).

There is a preference for the flow F to enter the upstream nostril on inspiration, and slightly more flow will enter the upstream nostril than the downstream nostril. On expiration, the flow will have a preference to exit the downstream nostril (flow F5) and more flow will exit that nostril than the upstream nostril (flow F4), due to lesser flow F3 than F1.

When breath holding, some portion of the flow will travel into the upstream nostril and out of the downstream nostril, due to the higher flow F1 into the upstream nostril than flow F3 into the downstream nostril.

The geometry of the bias flow restriction 140 will define the volume of positive flow through the nasal interface 100. A larger area for bias flow will result in higher flow rate required for the gas source to reach the desired therapeutic pressure.

The bypass restriction 130 results in a pressure drop between the gases delivered to the upstream and downstream nostrils F1, F3, with a resulting pressure differential at the first and second nasal delivery elements 111, 112.

The pressure drop results in asymmetric flow which leads to "flushing" or "clearance" of the dead space, the dead space being the volume of the gas which is not involved in gases exchange within the alveoli and consists largely of CO₂.

In some configurations, the configuration of the asymmetric flow may be between about 1 and about 5 lpm.

In some configurations, the pressure at downstream nostril may be about 1 cmH2O less than the pressure delivered to the upstream nostril, e.g. about 6 cmH2O at the upstream nostril and about 5 cmH2O at the downstream nostril.

The bias flow restriction 140 may be configured to avoid negative flows during the provision of respiratory therapy.

It is recognised that negative flows contribute to dead space or rebreathing. Therefore, the bias flow restriction 140 should be large enough to enable a bias flow that is high enough so that the occurrence of negative flows and the amount of rebreathing are either reduced or eliminated.

Asymmetric flow will reduce the amount of gas that is rebreathed throughout a respiratory cycle as the upper airway volume is ventilated.

This may alternatively be described as reducing the dead space or reducing the amount of gas that does not take part in the gas exchange during a breath cycle.

The effects of reducing dead space are also seen in for example high flow therapy.

This reduces CO₂ rebreathing and increases the amount of oxygen available for gas exchange.

The delivered gas may require additional humidity than is typically used for non-invasive ventilation (NIV) or continuous positive airway pressure (CPAP) therapy. This additional humidity is to prevent drying of the upper airways as gas within the dead space is replaced by the gas provided by the therapy.

The anatomical dead space volume for a given patient may typically be between 100 ml and 150 ml.

The bypass flow caused by the bypass restriction 130 is such that there is a pressure difference between the two nostrils in response to the apparatus delivering flow in combination with respiration, or during an apnoea. Some possible configurations of the bypass restriction may be as described, however, in alternative configurations of the bypass restriction this may be achieved by one or a combination of two or more of the following:
- Having the bypass flow restricted for example by having a reduced cross-section or some element to create this restriction such as one or a plurality of projections or nozzles or some other reduction in cross-section.
- The bypass restriction geometry may be designed so that there is a preference for it to travel one way rather than the other, for example by making use of geometry that has a higher pressure drop in one direction than the other such as a bell-mouth shaped nozzle or restriction, a low-pressure ejector, or a non-return valve.
- A flexible element or valve that creates a preferential (but not exclusive) flow from the upstream to downstream direction.
- A user-adjustable valve by means of a screw or some other mechanism to alter the cross-sectional area for the bypass flow to pass through.
- Flow enters the first nasal delivery element either axially, radially, tangentially at an angle or some combination of these flows, to preferentially direct the gases flow at the first nasal delivery element rather than the second nasal delivery element.
- The bypass restriction comprises a sparse network of material to create a pressure drop such as a filter, nonwoven polypropylene, foamed plastic, sintered material, or any other material that creates a pressure drop across it when flow is present.

The bypass restriction may comprise any one or more of the features described in US 2016/0228665.

The overall bias flow is mostly controlled by selection of the geometry across the bias flow restriction 140. Some possible configurations of the bias flow restriction by may be as described, however, in alternative configurations the pressure drop of the bias flow restriction may be achieved by one or a combination of two or more of the following:
- Having the bias flow restricted for example by having a reduced cross section or some element to create this restriction such as one or multiple members or nozzles or some other reduction in cross-section.
- A flexible element or valve that creates a preferential, and possibly exclusive, flow out of the nasal interface to reduce or prevent entrainment of ambient air.
- A non-return valve may be used to reduce/prevent entrainment of ambient air.
- A flexible element may be used to create a pressure drop that is less likely to occlude in the presence of water or sputum than rigid holes/nozzles.
- A sparse network of material may be present that would create a pressure drop such as a filter, nonwoven polypropylene, foamed plastic, sintered material, or any other material that create a pressure drop across it when flow is present
- A user-adjustable valve by means of a screw or some other mechanism to alter the cross-sectional area of the flow.
- Altering the direction of the flow multiple times.

The pressure drop across the nasal interface 100 may be relatively constant across the patient's breath cycle, or alternatively may vary across the patient's breath cycle.

Table 1 summarises different gases flows that may be encountered during use of the nasal interface 100, with reference to Figure 15.

**Table 1 - Summary of flows**

| **Arro w** | **Inspiratio n** | **Expiratio n** | **Apnoea** / **breath holding** | **Inspiratio n with upstream nostril blocked** | **Expiratio n with upstream nostril blocked** | **Inspiration with downstrea m nostril blocked** | **Expiration with downstrea m nostril blocked** |
|---|---|---|---|---|---|---|---|
| 1 | Positive | Either | Positive | Positive | Either | Positive | Either |
| 2 | Positive | Either | Positive | Zero | Zero | Positive | Negative |
| 3 | Positive | Either | Positive | Positive | Either | Either | Positive |
| 4 | Either | Positive | Positive | Negative | Positive | Zero | Zero |
| 5 | Either | Positive | Positive | Either | Positive | Either | Positive |

In Figure 15, positive flow is in the direction of the arrow, negative flow is opposite to the direction of the arrow. Either means that flow may be moving in either direction based on a number of factors. Zero means there is no nett flow in this scenario.

If the upstream nostril is completely blocked then the patient will receive flow to the downstream nostril.

If the downstream nostril is completely blocked then the patient will receive flow through the upstream nostril.

In both of these cases the patient will not receive any asymmetric flow but will continue to be provided therapy without this component.

If either nostril is substantially but not completely blocked, a reduced amount of asymmetric flow will be present.

The device may be used on a similar group of patients that are suitable for non-invasive ventilation (NIV).

Nasal cycling may introduce fluctuations in the asymmetric flow provided by the nasal interface 100.

As indicated schematically in Figure 15, the nasal interface 100 forms a circuit with a patient's upper airways and lungs. A first portion of the circuit comprises the first nasal delivery element 111, the patient's upstream nostril associated with that first nasal delivery element 111, the patient's upper airway and lungs, the second nasal delivery element 112, and the patient's downstream nostril associated with that second nasal delivery element 112. A second portion of the circuit comprise the first nasal delivery element 111, the bypass restriction 130, and the second nasal delivery element 112. The bypass restriction provides a pressure drop through the gases manifold between the first nasal delivery element 111 and the second nasal delivery element 112, which results in an asymmetric flow through the first nasal delivery element 111 and the second nasal delivery element 112.

The nasal interface 100 creates a pressure differential between the two nostrils such that the upstream nostril is at a higher pressure than the downstream nostril for at least some portion of the breath cycle.

This pressure difference creates a flow within the upper airway where after a full breath cycle more flow has entered the upstream nostril than the downstream nostril and more flow has left the downstream nostril than the upstream nostril. This additional flow into the upstream nostril and out of the downstream nostril is asymmetric flow.

The asymmetric flow dilutes the gasses in the airways of the patient which is referred to in the art as washout or dead space clearance.

There are a number of ways of achieving this pressure differential between the entrance to the two nostrils including having a bypass flow between the nostrils that is tuned to provide some pressure drop.

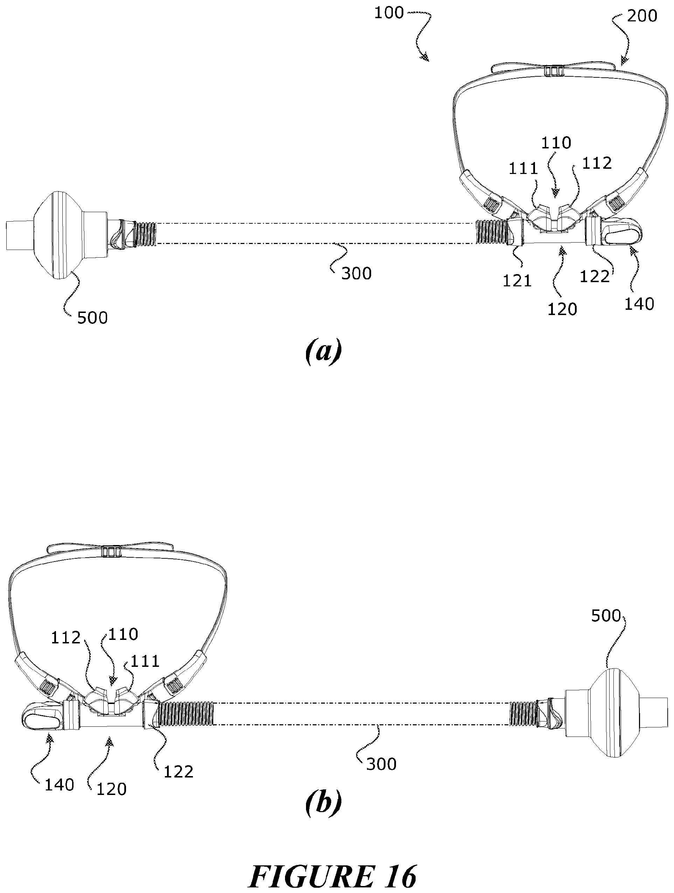

In some configurations of the nasal interface 100, the gases manifold 120 may be a configuration that allows the respiratory conduit 300 to connect to either the right side of the gases manifold (Figure 16(a)) or the left side of the gases manifold (Figure 16(b)). That is, the respiratory conduit 300, and optionally the bias flow restriction 140, may be side-swappable relative to the gases manifold 120. That enables the respiratory conduit 300 to be positioned on the right side or left side of the patient in use.

In some configurations, the gases ports 121, 122 may have the same configuration as each other, so that the respiratory conduit 300 can be selectively coupled with either of the gases ports 121, 122. The gases port that the patient breathing conduit is connected to will form the gases inlet for the gases manifold 120, and the opposite gases port will form the gases outlet for the gases manifold. For example, in the configuration of Figure 16(a), the gases port 121 will form the gases inlet, and the first nasal delivery element 111 will form the upstream nasal delivery element that is more proximal to the gases inlet. In the configuration of Figure 16(b), the gases port 122 will form the gases inlet, and the second nasal delivery element 112 will form the upstream nasal delivery element that is more proximal to the gases inlet.

The internal features of the gases manifold 120 may be symmetrical, so that the performance of the nasal interface 100 doesn't change depending on which side of the gases manifold the respiratory conduit 300 is connected to.

When a bias flow restriction 140 is provided, that may be able to be selectively coupled with either of the gases ports 121, 122, opposite to the respiratory conduit 300. The respiratory conduit 300 and the bias flow restriction 140 may have the same coupling features as each other.

Although the respiratory conduit 300 may be selectively connected to either side of the gases manifold 120, at any stage during use of the nasal interface one of the ports 121, 122 will act as a single gases inlet into the gases manifold 120. The other one of the ports 121, 122 will typically act as a gases outlet from the gases manifold to deliver gases to the bias flow restriction 140.

The nasal interface 100 may be provided with one or more pressure ports to allow pressure measurement for control of a respiratory therapy apparatus or for reporting purposes. The pressure port(s) may be provided upstream and/or downstream and/or within the nasal interface 100.

Gases entering and/or exiting the nasal interface 100 may be filtered. An upstream and/or downstream filter may be provided for that purpose.

In the configuration shown, the patient interface 1 comprises a filter 500 that is in fluid communication with the respiratory conduit 300 to filter gases entering the respiratory conduit 300.

The filter(s) may have any one or more of the features and functionality of the filter of US patent no. 6,619,287.

It may be desirable to configure the cross-sectional area through the bypass restriction 130 to be as wide as possible to increase patient comfort. However, increasing the cross-sectional area through the bypass restriction 130 risks decreasing the amount of asymmetric flow. That is, increasing the cross-sectional area through the bypass restriction 130 decreases the degree of restriction, which in turn decreases the pressure difference between the upstream and downstream nasal delivery elements 111, 112 that drives asymmetric flow and washout.

As outlined above, the bypass restriction 130 may be any feature or geometry that provides a pressure drop through the nasal interface 100 between the first nasal delivery element 111 and the second nasal delivery element when gases are delivered from the gases inlet 121 to the first nasal delivery element 111 and the second nasal delivery element 112 such that pressure at the first nasal delivery element 111 is higher than pressure at the second nasal delivery element 112. In some configurations, the bypass restriction 130 may be a physical restriction relative to an adjacent part of the gases flow channel 125, relative to the gases inlet 121, relative to the combined cross-sectional area A₃+ A₄ of the first and second nasal delivery elements 111, 112, and/or relative to any other part of the nasal interface 100.

The inventors have discovered that effective asymmetric flow may be maintained at a wide range of ratios of bypass restriction cross-sectional area to combined nasal delivery element cross-sectional area. This may allow patient comfort to be optimised while retaining therapeutically effective asymmetric flow.

The therapeutically effective asymmetric flow may be provided by having sufficient washout from the patient's upper airway dead space. The washout level may be at least about 10% of the volume of the patient's upper airway, optionally at least about 20% of that volume, optionally at least about 30% of that volume, optionally at least about 40% of that volume, optionally at least about 50% of that volume, optionally at least about 60% of that volume, optionally at least about 70% of that volume, optionally at least about 80% of that volume, optionally at least about 90% of that volume, optionally about 100% of that volume. In some configurations, the washout level may be determined over a single breath cycle.

The ratio of bypass restriction 130 cross-sectional area A₂ to combined nasal delivery element 111, 112 cross-sectional area A₃+ A₄ contributes to achieving asymmetric flow and thus effective washout. The bypass restriction 130 cross-sectional area A₂ and combined nasal delivery element 111, 112 cross-sectional area A₃+ A₄ are cross-sectional areas for the flow of gases or inner cross-sectional areas. The combined nasal delivery element 111, 112 cross-sectional area A₃+ A₄ may be at a smallest transverse dimension of the respective nasal delivery element 111, 112.

The bypass restriction 130 drives asymmetric flow by restricting gas flow to the downstream nasal delivery element 112 relative to the upstream nasal delivery element 111. The bypass restriction 130 cross-sectional area A₂ should therefore be sufficiently narrow (or in other words, sufficiently restrictive) relative to the combined nasal delivery element 111, 112 cross-sectional area A₃+ A₄ such that a restriction, and thus a pressure difference, is achieved.

It is, however, also desirable for the bypass restriction 130 cross-sectional area A₂ to be as wide as possible in order to increase patient comfort and therapeutic versatility. In particular, it is desirable for the bypass restriction 130 cross-sectional area A₂ to be sufficiently wide so that if the upstream nasal delivery element 111 or naris becomes blocked during therapy, the patient may still receive CPAP therapy through the downstream nasal delivery element 112. Increasing the effort of inspiration could potentially make a patient feel starved of air. Making the bypass restriction 130 cross-sectional area A₂ larger means that a greater fraction of the flow on inspiration goes to the downstream naris via the downstream nasal delivery element 112. This reduces the pressure drop experienced by the patient and decreases this discomfort. However, making the bypass restriction 130 cross-sectional area A₂ larger would cause the therapy to move towards traditional CPAP therapy where there is no therapeutically effective asymmetrical flow. Patients undergoing traditional CPAP therapy may feel more comfortable with less restriction because the apparatus can control flows more easily, reducing flow velocities and decreasing noise and the feeling of jetting in the nostrils.

In some configurations, a nasal interface 100 of the present disclosure comprises a first nasal delivery element 111 and a second nasal delivery element 112, wherein the first nasal delivery element 111 and the second nasal delivery element 112 are each configured to seal with a respective naris of a patient, and a gases manifold 120 comprising a gases inlet 121 for delivery of respiratory gases to the gases manifold 120 and a gases flow channel 125, wherein the first nasal delivery element 111 and the second nasal delivery element 112 are in fluid communication with the gases inlet 121 via the gases flow channel 125, wherein the first nasal delivery element 111 is proximal to the gases inlet 121 and the second nasal delivery element 112 is distal from the gases inlet 121, wherein the nasal interface comprises a bypass restriction 130 that provides a cross-sectional area A₂ of a portion of the gases flow channel 125, wherein each of the first nasal delivery element 111 and the second nasal delivery element 112 comprises an inner cross-sectional area A₃, A₄, wherein the inner cross-sectional areas A₃, A₄ together provide a combined cross-sectional area A₃+ A₄ of the nasal delivery elements 111, 112, and wherein the cross-sectional area A₂ of the portion of the gases flow channel 125 is more than 0 to about 1.5 times the combined cross-sectional area A₃+ A₄ of the nasal delivery elements.

Such a configuration with the recited relative cross-sectional areas could be used in any of the nasal interfaces 100 disclosed herein.

In some configurations, the cross-sectional area A₂ of the portion of the gases flow channel 0.25 times to about 1.5 times the combined cross-sectional area A₃+ A₄ of the nasal delivery elements.

In some configurations, the cross-sectional area A₂ of the portion of the gases flow channel is up to about 1.3 times the combined cross-sectional area A₃+ A₄ of the nasal delivery elements, optionally up to about 1 times the combined cross-sectional area A₃+ A₄ of the nasal delivery elements, optionally up to about 2/3 of the combined cross-sectional area A₃+ A₄ of the nasal delivery elements, optionally up to about 1/2 of the combined cross-sectional area A₃+ A₄ of the nasal delivery elements, optionally up to about 2/5 of the combined cross-sectional area A₃+ A₄ of the nasal delivery elements, optionally up to about 1/3 of the combined cross-sectional area A₃+ A₄ of the nasal delivery elements.

In some configurations, the cross-sectional area A₂ of the portion of the gases flow channel is more than 0 mm² and up to about 375 mm², optionally between about 1 mm² and about 375 mm², optionally between about 1 mm² and about 250 mm², optionally between about 1 mm² and about 200 mm², optionally between about 1 mm² and about 167 mm², optionally between about 50 mm² and about 167 mm², optionally between about 50 mm² and about 103 mm², optionally between about 35 mm² and about 100 mm². The bypass restriction 130 cross-sectional area A₂ may be any other value or range of values related to the combined cross-sectional area A₃+ A₄ of the nasal delivery elements 111, 112 recited herein

In some configurations, the inner cross-sectional area A₃, A₄ of each of the first and second nasal delivery elements 111, 112 is at a smallest transverse dimension of the respective nasal delivery element.

In some configurations, the inner cross-sectional area of each of the first and second nasal delivery elements 111, 112 is at an outlet 111a, 112a of the respective nasal delivery element 111, 112. Alternatively, the inner cross-sectional area may be elsewhere; for example, part-way along the nasal delivery element 111, 112 or at an inlet or base of the nasal delivery element.

The inner cross-sectional area A₃, A₄ of each of the nasal delivery elements 111, 112 may be in a direction that is transverse to a direction of gases flow through the nasal delivery elements 111, 112.

In some configurations, the bypass restriction 130 comprises at least one protrusion 130a, 130b extending into the gases flow channel 125. In some configurations, the bypass restriction 130 comprises a plurality of protrusions extending into the gases flow channel 125.

In some configurations, the gases manifold 120 comprises a proximal bypass protrusion 130a that is proximal to the nasal delivery elements 111, 112 and/or a distal bypass protrusion 130b that is distal from the nasal delivery elements 111, 112.

In some configurations, the gases manifold 120 comprises both a proximal bypass protrusion 130a and a distal bypass protrusion 130b which in combination define a predetermined bypass dimension BD for the restricted flow of gases through the gases manifold 120 between the first nasal delivery element 111 and the second nasal delivery element 112. In some configurations, the predetermined bypass dimension BD may be restricted relative to an adjacent part of the gases flow channel 125, relative to the gases inlet 121, relative to the combined cross-sectional area A3+ A4 of the first and second nasal delivery elements 111, 112, and/or relative to any other part of the nasal interface 100.

The predetermined bypass dimension BD will generally be substantially smaller than a dimension of an adjacent or main part of the gases flow channel 125.

In some configurations, the bypass restriction 130 comprises an angled leading edge 130a', 130b' and an angled trailing edge 130a", 130b" that define a converging and diverging bypass restriction in a direction of gases flow through the gases manifold from the first nasal delivery element 111 to the second nasal delivery element 112.

In some configurations, the gases manifold 120 comprises a single inlet and a single outlet.

In some configurations, the nasal interface 100 comprises an interface body 110 and a gases manifold part, and the interface body 110 and the gases manifold part together form the gases manifold 120.

In some configurations, the portion of the gases flow channel that provides the cross-sectional area A₂ is provided by the interface body 110 and the gases manifold part.

As outlined above, the interface body 110 may be formed from a soft, flexible material.

In some configurations, the cross-sectional area A₂ of the portion of the gases flow channel may be variable. For example, when the patient wears the nasal interface 100, a portion of the patient's face may impinge on the base of the nasal delivery elements 111, 112 or the interface body 110 to narrow the bypass restriction 130 and thereby the cross-sectional area A₂ of the portion of the gases flow channel. This could be affected by the distance of the base of the nasal delivery elements 111, 112 from the patient's septum. In some configurations, the interface body 110 or a portion thereof may be configured to limit the variability of the cross-sectional area A₂ of the portion of the gases flow channel when the patient wears the nasal interface 100. For example, a portion of the interface body 110 may be stiffened with another more rigid material, using a more rigid material and/or designed with a specific geometry.

In some configurations, the gases manifold 120 or the gases manifold part is separable from the interface body 110.

In some configurations, the gases inlet 121 is at a side of the gases manifold 120.

In some configurations, the nasal interface 100 comprises a bias flow restriction 140 for a flow of gases out of the nasal interface 100 through the bias flow restriction 140.

In some configurations, the bias flow restriction 140 comprises at least one aperture 142 for the flow of gases from the nasal interface 100 to an ambient environment. In some configurations, the bias flow restriction 140 comprises a plurality of apertures 142 for the flow of gases from the nasal interface 100 to an ambient environment.

In some configurations, the bias flow restriction 140 comprises a filter or a diffuser to filter or diffuse gases flowing through the aperture(s) 142.

In some configurations, the nasal interface comprises a filter unit 500' between the gases manifold 120 and the bias flow restriction 140.

In some configurations, the bias flow restriction 140 is in fluid communication with the gases manifold 120. In some configurations, the gases manifold 120 comprises the bias flow restriction 140 or is coupled to the bias flow restriction 140. In some configurations, the bias flow restriction 140 is in fluid communication with the gases manifold 120 but is positioned remotely from the gases manifold.

In some configurations, the bias flow restriction 140 comprises an open area for gases flow out of the nasal interface 100 through the bias flow restriction 140. In some configurations, the open area is more than 0 mm² to about 40 mm², optionally between about 2 mm² and about 40mm², optionally between about 2 mm² and about 5 mm², optionally between about 12 mm² and about 40mm², optionally between about 20 mm² and about 30 mm².

In some configurations, the open area for gases flow out of the nasal interface 100 through the bias flow restriction is about 1 mm², about 2 mm², about 3 mm², about 4 mm², about 5 mm², about 6 mm², about 7 mm², about 8 mm², about 9 mm², about 10 mm², about 11 mm², about 12 mm², about 13 mm², about 14 mm², about 15 mm², about 16 mm², about 17 mm², about 18 mm², about 19 mm², about 20 mm², about 21 mm², about 22 mm², about 23 mm², about 24 mm², about 25 mm², about 26 mm², about 27 mm², about 28 mm², about 29 mm², about 30 mm², about 31 mm², about 32 mm², about 33 mm², about 34 mm², about 35 mm², about 36 mm², about 37 mm², about 38 mm², about 39 mm², or about 40 mm², or is any value between any two of those values.

The flow through the bias flow restriction 140 for a given pressure difference between the body gases flow passage 146 and the outside of the bias flow restriction 140 is largely determined by the cross-sectional area of the apertures 142 and their geometry. The geometric factor may be known as the discharge coefficient. For example, cylindrical outlet apertures 142 with sharp edges will let through less flow than smooth apertures that are shaped like venturi nozzles or apertures with a substantial radius, chamfer, or other expansion and contraction features on either the inlet or outlet side. Viscous effects such as in long thin channels may also reduce the overall flow rate through the apertures, depending on their shape.

The size of the apertures 140 could be increased, but the design of the filter or diffuser could additionally or alternatively be adjusted to add resistance.

Therefore, the upper end of the range of sizes of open area for gases flow out of the nasal interface 100 through the bias flow restriction 140 could be increased by up to 25% (e.g. 50 mm² rather than 40 mm²) if a suitably configured filter or diffuser is used.

Similarly, if apertures 142 with a high discharge coefficient are used, the lower end of the range of sizes of open area for gases flow could be reduced by up to 50% (e.g. 6 mm² rather than 12 mm²).

In some configurations, the bias flow restriction 140 is configured such that a flow rate of the flow of gases out of the nasal interface 100 through the bias flow restriction 140 is more than 0 lpm to about 80 Ipm when a pressure of more than 0 cmH2O and up to about 30 cmH2O is provided to the gases inlet 121 in use.

In some configurations, the bias flow restriction 140 is configured such that a flow rate of the flow of gases out of the nasal interface 100 through the bias flow restriction 140 is about 5 Ipm, about 10 Ipm, about 15 Ipm, about 20 Ipm, about 25 Ipm, about 30 Ipm, about 35 Ipm, about 40 Ipm, about 45 Ipm, about 50 Ipm, about 55 Ipm, about 60 Ipm, about 65 Ipm, about 70 Ipm, about 75 Ipm, about 80 Ipm, or is any value between any two of those values when a pressure of about 5 cmH2O, about 10 cmH2O, about 15 cmH2O, about 20 cmH2O, about 25 cmH2O, about 30 cmH2O, or of any value between any two of those values is provided to the gases inlet 121 in use.

In some configurations, the bias flow restriction 140 is configured such that a flow rate of the flow of gases out of the nasal interface 100 through the bias flow restriction 140 is between about 35 lpm and about 55 lpm when a pressure of between about 5 cmH2O and about 10 cmH2O is applied to the gases inlet 121 in use and the nasal delivery elements 111, 112 are occluded.

In some configurations, the bias flow restriction 140 is configured such that a flow rate of the flow of gases out of the nasal interface 100 through the bias flow restriction 140 is between about 4 lpm and about 15 lpm when a pressure of between about 3 cmH2O and about 10 cmH2O is provided to the gases inlet 121 in use and the nasal delivery elements 111, 112 are occluded.

In some configurations, the bias flow restriction 140 is configured such that a flow rate of the flow of gases out of the nasal interface 100 through the bias flow restriction 140 is about 5 Ipm, about 6 Ipm, about 7 Ipm, about 8 Ipm, about 10 Ipm, about 11 Ipm, about 12 Ipm, about 13 Ipm, about 14 Ipm, about 15 Ipm, or is any value between any two of those values when a pressure of about 3 cmH2O, about 4 cmH2O, about 5 cmH2O, about 6 cmH2O, about 7 cmH2O, about 8 cmH2O, about 9 cmH2O, about 10 cmH2O, or of any value between any two of those values is provided to the gases inlet 121 in use and the nasal delivery elements 111, 112 are occluded.

In some configurations, the bias flow restriction 140 is configured such that a flow rate of the flow of gases out of the nasal interface 100 through the bias flow restriction 140 is between about 15 lpm and about 80 Ipm when a pressure of between about 4 cmH2O and about 30 cmH2O is provided to the gases inlet 121 in use and the nasal delivery elements 111, 112 are occluded.

In some configurations, the bias flow restriction 140 is configured such that a flow rate of the flow of gases out of the nasal interface 100 through the bias flow restriction 140 is about 15 Ipm, about 20 Ipm, about 25 Ipm, about 30 Ipm, about 35 Ipm, about 40 Ipm, about 45 Ipm, about 50 Ipm, about 55 Ipm, about 60 Ipm, about 65 Ipm, about 70 Ipm, about 75 Ipm, about 80 Ipm, or is any value between any two of those values when a pressure of about 5 cmH2O, about 10 cmH2O, about 15 cmH2O, about 20 cmH2O, about 25 cmH2O, about 30 cmH2O, or of any value between any two of those values is provided to the gases inlet 121 in use and the nasal delivery elements 111, 112 are occluded.

In some configurations, the nasal interface 100 could, as an addition to or as an alternative to having a bias flow restriction, be connected to an expiratory limb of a ventilator or have a positive end-expiratory pressure (PEEP) valve to control the amount of bias flow out of the nasal interface 100, which affects pressure and washout at the nasal interface 100.

In some configurations, the cross-sectional area A₂ of the portion of the gases flow channel 125 is transverse to a direction of gases flow through the portion of the gases flow channel 125.

The inner cross-sectional area A₃+ A₄ of each nasal delivery element 111, 112 may be the cross-sectional area bounded by the inner wall of the nasal delivery element 111, 112. For non-circular cross-sections, the references herein to a diameter may be interpreted as a transverse dimension. In some configurations, references herein to a diameter include but are not limited to a hydraulic diameter.

In some configurations, the cross-sectional area A₂ of the portion of the gases flow channel 125 is reduced in comparison to a cross-sectional area A₁ of an adjacent portion of the gases flow channel 125.

In some configurations, the cross-sectional area A₂ of the portion of the gases flow channel 125 is between about 10% and up to about 100% of a first cross-sectional area A₁ of an adjacent part of the gases flow channel, optionally about 10% or more and less than 100% of the first cross-sectional area, optionally up to about 90% of the first cross-sectional area A₁, optionally up to about 80% of the first cross-sectional area A₁, optionally up to about 70% of the first cross-sectional area A₁, optionally up to about 60% of the first cross-sectional area A₁, optionally up to about 55% of the first cross-sectional area A₁, optionally up to about 40% of the first cross-sectional area A₁, optionally up to about 30% of the first cross-sectional area A₁, and optionally up to about 25% of the first cross-sectional area A₁.

In some configurations, the cross-sectional area A₂ of the portion of the gases flow channel is up to about 200 mm², optionally up to about 160 mm², optionally up to about 110 mm² , optionally up to about 80 mm², optionally up to about 60 mm², and optionally up to about 50 mm².

In some configurations, the combined cross-sectional area A₃+ A₄ of the nasal delivery elements 111, 112 is more than 0 mm² and up to about 250 mm², optionally between about 1 mm² and about 250 mm², optionally between about 1.6 mm² and about 250 mm², optionally between about 50 mm² and about 250 mm², optionally between about 50 mm² and about 200 mm², optionally between about 30 mm² and about 200 mm², optionally between about 30 mm² and up to about 155 mm², and optionally between about 50 mm² and up to about 155 mm².

In some configurations, the combined cross-sectional area A₃+ A₄ of the nasal delivery elements 111, 112 is about 1 mm², about 1.6 mm², about 5 mm², about 10 mm², about 15 mm², about 20 mm², about 25 mm², about 30 mm², about 35 mm², about 40 mm², about 45 mm², about 50 mm², about 55 mm², about 60 mm², about 65 mm², about 70 mm², about 75 mm², about 80 mm², about 85 mm², about 90 mm², about 95 mm², about 100 mm², about 105 mm², about 110 mm², about 115 mm², about 120 mm², about 125 mm², about 130 mm², about 135 mm², about 140 mm², about 145 mm², about 150 mm², about 155 mm², about 160 mm², about 165 mm², about 170 mm², about 175 mm², about 180 mm², about 185 mm², about 190 mm², about 195 mm², about 200 mm², about 205 mm², about 210 mm², about 215 mm², about 220 mm², about 225 mm², about 230 mm², about 235 mm², about 240 mm², about 245 mm², or about 250 mm², or is any value between any two of those values.

In some configurations, the bypass restriction 130 provides a pressure drop through the nasal interface 100 between the first nasal delivery element 111 and the second nasal delivery element 112 when gases are delivered from the gases inlet 121 to the first nasal delivery element 111 and the second nasal delivery element 112 such that pressure at the first nasal delivery element 111 is higher than pressure at the second nasal delivery element 112.

In some configurations, a nasal interface 100 of the present disclosure comprises a first nasal delivery element 111 and a second nasal delivery element 112, wherein the first nasal delivery element 111 and the second nasal delivery element 112 are each configured to seal with a respective naris of a patient, and a gases manifold 120 comprising a gases inlet 121 for delivery of respiratory gases to the gases manifold 120 and a gases flow channel, wherein the first nasal delivery element 111 and the second nasal delivery element 112 are in fluid communication with the gases inlet 121 via the gases flow channel 125, wherein the first nasal delivery element 111 is proximal to the gases inlet 121 and the second nasal delivery element 112 is distal from the gases inlet 121, wherein the nasal interface comprises a bypass restriction 130 that provides a cross-sectional area A₂ of a portion of the gases flow channel, wherein each of the first nasal delivery element 111 and the second nasal delivery element 112 comprises an inner cross-sectional area A₃, A₄, and wherein the inner cross-sectional areas A₃, A₄ of the nasal delivery elements and the cross-sectional area A₂ of the portion of the gases flow channel are related so as to create an asymmetrical flow of gases from the nasal delivery elements 111, 112 in use.

In some configurations, the inner cross-sectional areas A₃, A₄ together provide a combined cross-sectional area A₃+ A₄ of the nasal delivery elements 111, 112, and wherein the cross-sectional area A₂ of the portion of the gases flow channel 125 is more than 0 to about 1.5 times the combined cross-sectional area A₃+ A₄ of the nasal delivery elements 111, 112.

In some configurations, the cross-sectional area A₂ of the portion of the gases flow channel is up to about 1.3 times the combined cross-sectional area A₃+ A₄ of the nasal delivery elements 111, 112, optionally up to about 1 times the combined cross-sectional area A₃+ A₄ of the nasal delivery elements 111, 112, optionally up to about 2/3 of the combined cross-sectional area A₃+ A₄ of the nasal delivery elements 111, 112, optionally up to about 1/2 of the combined cross-sectional area A₃+ A₄ of the nasal delivery elements 111, 112, optionally up to about 2/5 of the combined cross-sectional area A₃+ A₄ of the nasal delivery elements 111, 112, optionally up to about 1/3 of the combined cross-sectional area A₃+ A₄ of the nasal delivery elements 111, 112.

In some configurations, the inner cross-sectional area A₃, A₄ of each of the first and second nasal delivery elements 111, 112 is at a smallest transverse dimension of the respective nasal delivery element 111, 112.

In some configurations, the inner cross-sectional area A₃, A₄ of each of the first and second nasal delivery elements 111, 112 is at an outlet 111a, 112a of the respective nasal delivery element 111, 112. Alternatively, the inner cross-sectional area may be elsewhere; for example, part-way along the nasal delivery element or at an inlet or base of the nasal delivery element 111, 112.

In some configurations, the cross-sectional area A₂ of the portion of the gases flow channel 125 is up to about 1 times, optionally up to about 2/3 times, the combined cross-sectional area A₃+ A₄ of the nasal delivery elements 111, 112, and the nasal interface is configured to provide a bias flow through a bias flow restriction 140 of 20 lpm when a pressure of 4 cmH2O is provided to the gases inlet 121 and the nasal delivery elements 111, 112 are occluded. This may be in a patient that has an adult breath pattern of 15 breaths per minute (BPM) of 10i:20e 500 Vt for example.

In some configurations, the cross-sectional area A₂ of the portion of the gases flow channel 125 is up to about 1 times, optionally up to about 2/3 times, the combined cross-sectional area A₃+ A₄ of the nasal delivery elements 111, 112, and the nasal interface is configured to provide a bias flow through a bias flow restriction 140 of 32 lpm when a pressure of 8 cmH2O is provided to the gases inlet 121 and the nasal delivery elements 111, 112 are occluded. This may be in a patient that has an adult breath pattern of 15 BPM of 10i:20e 500 Vt, for example.

In some configurations, the cross-sectional area A₂ of the portion of the gases flow channel 125 is up to about 2/3 times the combined cross-sectional area A₃+ A₄ of the nasal delivery elements 111, 112, and the nasal interface is configured to provide a bias flow through the bias flow restriction 140 of 20 lpm when a pressure of 4 cmH2O is provided to the gases inlet 121 and the nasal delivery elements 111, 112 are occluded (this may be in a patient that has an adult breath pattern of 15 BPM of 10i:20e 500 Vt or in patent that has ARDS and has an adult breath pattern of 25 BPM for example), or is configured to provide a bias flow through the bias flow restriction 140 of 32 lpm when a pressure of 8 cmH2O is provided to the gases inlet 121 and the nasal delivery elements 111, 112 are occluded, or is configured to provide a bias flow through the bias flow restriction 140 of 41 lpm when a pressure of 12 cmH2O is applied to the gases inlet 121 and the nasal delivery elements 111, 112 are occluded, or is configured to provide a bias flow of 48 Ipm through the bias flow restriction 140 when a pressure of 16 cmH2O is applied to the gases inlet 121 and the nasal delivery elements 111, 112 are occluded, or is configured to provide a bias flow through the bias flow restriction 140 of 53 lpm when a pressure of 20 cmH2O is applied to the gases inlet 121 and the nasal delivery elements 111, 112 are occluded. This may be in a patent that has ARDS and has an adult breath pattern of 25 BPM, for example.

In some configurations, the cross-sectional area A₂ of the portion of the gases flow channel 125 is up to about 2/3 times the combined cross-sectional area A₃+ A₄ of the nasal delivery elements 111, 112, and the nasal interface is configured to provide a bias flow through the bias flow restriction 140 of 32 lpm or higher when a pressure of 8 cmH2O is provided to the gases inlet 121 and the nasal delivery elements 111, 112 are occluded. This may be in a patent that has an adult breath pattern of 15 BPM of 10i:20e 500 Vt or in patent that has ARDS and has an adult breath pattern of 25 BPM or in a patent that has an adult breath pattern of 25 BPM of 350 sinusoidal breath pattern, for example.

In some configurations, the cross-sectional area A₂ of the portion of the gases flow channel 125 is up to about 1/3 times the combined cross-sectional area A₃+ A₄ of the nasal delivery elements 111, 112 and the nasal interface is configured to provide a bias flow through the bias flow restriction 140 of 32 lpm or higher when a pressure of 8 cmH2O is provided to the gases inlet 121 and the nasal delivery elements 111, 112 are occluded, or wherein the cross-sectional area A₂ of the portion of the gases flow channel is up to about 2/5 times the combined cross-sectional area A₃+ A₄ of the nasal delivery elements 111, 112 and the nasal interface is configured to provide a bias flow through the bias flow restriction 140 of 41 lpm or higher when a pressure of 12 cm H2O is provided to the gases inlet 121 and the nasal delivery elements 111, 112 are occluded, or wherein the cross-sectional area A₂ of the portion of the gases flow channel 125 is up to about 2/3 times the combined cross-sectional area A₃+ A₄ of the nasal delivery elements 111, 112 and the nasal interface is configured to provide bias flow through the bias flow restriction 140 of 48 lpm or higher when a pressure of 16 cm H2O is provided to the gases inlet 121 and the nasal delivery elements 111, 112 are occluded.

The effectiveness of asymmetric flow (and the resulting washout) of the nasal interface 100 was assessed for six different ratios across three different tests.

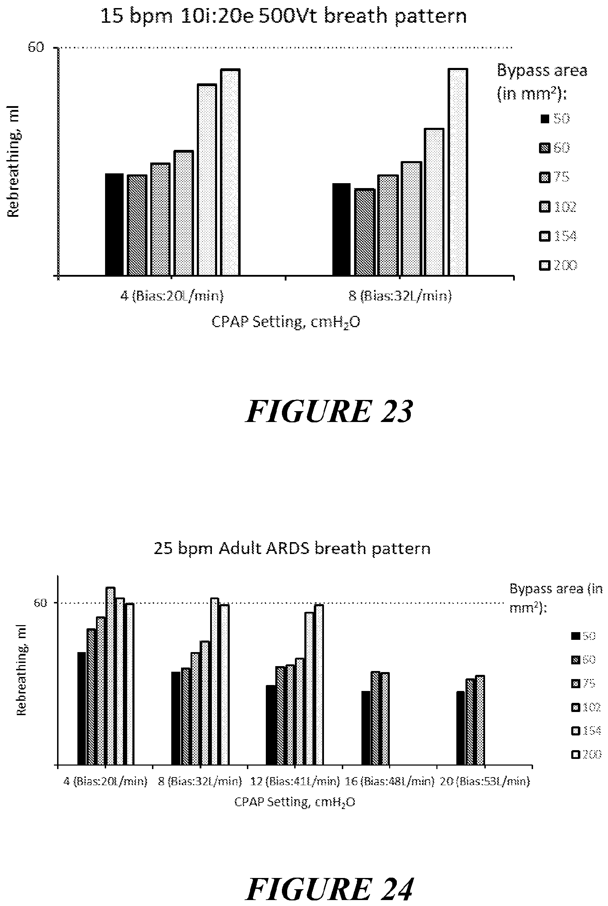

Each test comprised a set breathing pattern, upon which the CPAP settings were varied. Tables 2 and 3 show the constant settings and varied settings respectively of the tests. The results of the tests are shown in Figures 23 to 25.

**Table 2**

| **Bypass restriction cross-sectional area A₂ (BRA) - mm²** | **Nasal delivery element cross-sectional area (CNDEA) - mm²** | **BRA:CNDEA** | **Cross-sectional area through aperture(s) of bias flow restriction - mm²** |
|---|---|---|---|
| 50 | | 50:154 (about 1/3) | |
| 60 | | 60:154 (about 2/5) | |
| 75 | 154 (combined A₃+ A₄) | 75:154 (about 1/2) | 27.5 |
| 102 | 77 (individual A₃, A₄) | 102:154 (about 2/3) | |
| 154 | | 154:154 (about 1) | |
| 200 | | 200:154 (about 1.5) | |

**Table 3**

| | **CPAP settings (cmH2O)** | **Breath patterns** |
|---|---|---|
| Test 1 (Figure 23) | 4, 8 | 15 breaths/min (bpm) 10i:20e 500 Vt |
| Test 2 (Figure 24) | 4, 8, 12, 16, 20 | 25 breaths/min (bpm) adult ARDS - approximately 10i:15e and 400 Vt |
| Test 3 (Figure 25) | 4, 8, 12, 16, 20 | 45 breaths/min (bpm) 10i:10e 350 Vt |

In the tables above *x*i:*y*e is a ratio of *x* inspiratory time to y expiratory time and Vt is the tidal volume and a measure (in ml) of the amount of air that moves in or out of the lungs with each respiratory cycle.

In each test, asymmetric flow and washout was measured by rebreathing (the lower the rebreathing, the greater the washout). The typical level of rebreathing in CPAP without asymmetric flow is approximately 60 ml. Therefore, for the purposes of these tests, washout may be understood to be equal to '60 ml - x', wherein x = the volume of rebreathing in ml. 60 ml is an exemplary figure for an upper airway model, excluding the interface itself, and presumes no dead space in the interface.

As shown in Figures 23 to 25, effective washout was generally achieved at ratios of 102:154 (about 2/3) and below (bypass restriction cross-sectional area (BRA) : combined nasal delivery element cross-sectional area (CNDEA)).

Tests at ratios of 1:1 (BRA:CNDEA) and above showed inconsistent and/or minimal washout. However, some washout is shown/expected with ratios as high as 1.5:1 (BRA:CNEDA). In practice, a selected ratio may be below 1:1 (BRA:CNDEA).

Test 1 (15 bpm; 4, 8 cmH2O) showed significant washout at ratios of 102:154 (about 2/3) (BRA:CNEDA) and below, inconsistent washout at 1:1 (BRA:CNDEA), and minimal washout at 200:154 (about 1.5) (BRA:CNDEA).

Test 2 (25 bpm; 4, 8, 12, 16, 20 cmH2O) showed significant washout at ratios of 102:154 (about 2/3) (BRA:CNDEA) and below, but minimal washout at 1:1 (BRA:CNDEA) and above.

Test 3 (45 bpm; 4, 8, 12, 16, 20 cmH2O) showed washout at ratios of 102:154 (about 2/3) (BRA:CNDEA) and below at higher cmH2O levels, otherwise no significant washout was observed.

More particularly, and with reference to Figure 23, in Test 1 significant washout was achieved at ratios of 102:154 (about 2/3) (BRA:CNDEA) and below. A ratio of 1:1 (BRA:CNEDA) showed effective washout at 8 cmH2O, but minimal washout at 4 cmH2O. Minimal washout was achieved at 200:154 (about 1.5) (BRA:CNDEA).

With reference to Figure 24, in Test 2 significant washout was achieved at ratios of 102:154 (about 2/3) (BRA:CNDEA) and below (with the exception of 102:154 BRA:CNDEA at 4 cmH2O). Minimal washout was shown at 4, 8, and 12 cmH2O levels for ratios of 1:1 (BRA:CNDEA) and above. Those ratios were not tested at 16 cmH2O and 20 cmH2O levels.

At low pressures and high breath rates, rebreathing can occur because of insufficient bias flow. That may affect the results particularly at low pressures since at higher pressures the bias flow increases.

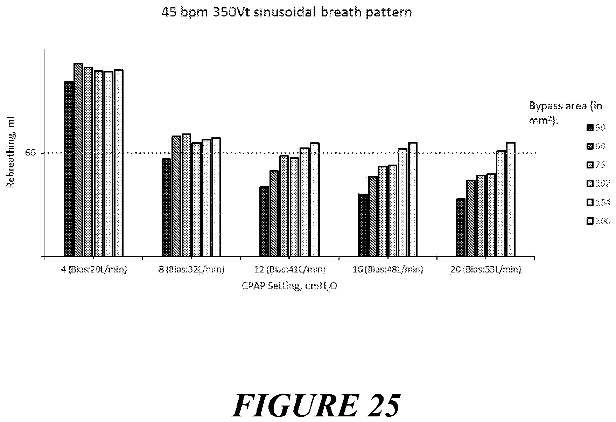

With reference to Figure 25, in Test 3 significant washout was achieved at ratios 102:154 (about 2/3) (BRA:CNDEA) and below for higher cmH2O levels, but not at lower cmH2O levels. Better than baseline washout was achieved at: 8 cmH2O and above for 50:154 (about 1/3) (BRA:CNDEA), 12 cmH2O and above for 60:154 (about 2/5) (BRA:CNDEA), 16 cmH2O and above for 75:154 (about 1/2) and 102:154 (about 2/3) (BRA:CNDEA). Washout was not achieved at ratios of 1:1 (BRA:CNDEA) or higher in this test.

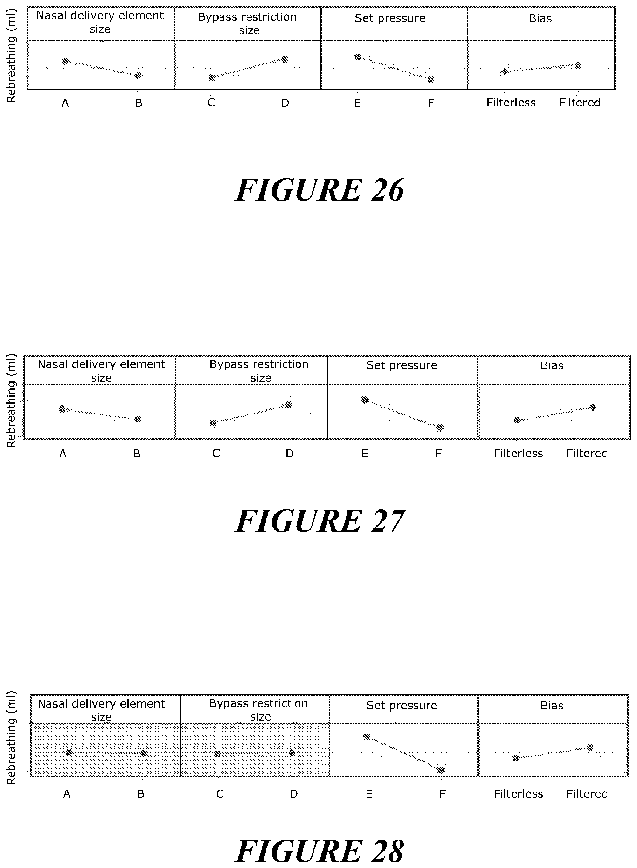

Figures 26 to 28 show modelled effects of different nasal delivery element 111, 112 sizes, different bypass restriction cross-sectional areas, different set pressures, and different bias flow restriction openness on rebreathing with the nasal interface for 15 breaths per minute, 25 breaths per minute, and 45 breaths per minute respectively.

The Y-axis (dependent axis) on the charts shows rebreathing, where a lower amount is better and shows more washout.

The 'nasal delivery element size' of each chart shows the combined cross-sectional area A₃+ A₄ of the nasal delivery elements 111, 112.

A is smaller than B. A-B provides a possible range for the combined cross-sectional area A₃+ A₄.

As outlined herein, in some configurations, the combined cross-sectional area A₃+ A₄ of the nasal delivery elements 111, 112 is more than 0 mm² (A) and up to about 250 mm² (B), optionally between about 1 mm² and about 250 mm², optionally between about 1.6 mm² and about 250 mm², optionally between about 50 mm² and about 250 mm², optionally between about 50 mm² and about 200 mm², optionally between about 30 mm² and about 200 mm², optionally between about 30 mm² and about 155 mm², optionally between about 50 mm² and about 155 mm², and optionally between about 70 mm² and about 155 mm².

The 'bypass restriction size' portion of each chart shows the bypass restriction 130 cross-sectional area A₂, i.e. the cross-sectional area A₂ of the portion of the gases flow channel.

C is smaller than D. C - D provides a possible range for the bypass restriction cross-sectional area A₂.

As outlined herein, in some configurations, the bypass restriction 130 cross-sectional area A₂ is more than 0 to about 1.5 times the combined cross-sectional area A₃+ A₄ of the nasal delivery elements 111, 112, optionally about 0.25 times to about 1.5 times the combined cross-sectional area A₃+ A₄ of the nasal delivery elements 111, 112, optionally about 1 times the combined cross-sectional area A₃+ A₄ of the nasal delivery elements 111, 112 or less, optionally about 2/3 times the combined cross-sectional area A₃+ A₄ of the nasal delivery elements 111, 112 or less.

As outlined herein, in some configurations, the bypass restriction 130 cross-sectional area A₂ is more than 0 mm² (C) and up to about 375 mm² (D), optionally between about 1 mm² and about 375 mm², optionally between about 1 mm² and about 250 mm², optionally between about 1 mm² and about 200 mm², optionally between about 1 mm² and about 167 mm², optionally between about 50 mm² and about 167 mm², optionally between about 50 mm² and about 103 mm², optionally between about 35 mm² and about 100 mm². The bypass restriction 130 cross-sectional area A₂ may be any other value or range of values related to the combined cross-sectional area A₃+ A₄ of the nasal delivery elements 111, 112 recited herein.

In some configurations, the cross-sectional area A₂ of the portion of the gases flow channel is more than 0 to about 1.5 times the combined cross-sectional area A₃ + A₄ of the nasal delivery elements 111, 112 and the combined cross-sectional area A₃ + A₄ of the nasal delivery elements 111, 112 is between about 1 mm² and about 250 mm².

The 'set pressure' portion of each chart shows the pressure applied to the gases inlet 121 of the nasal interface 100.

E is smaller than F. E - F provides a possible range of pressure applied to the gases inlet 121.

As outlined herein, in some configurations, a pressure of more than 0 cmH2O (E) and up to about 30 cmH2O (F) is provided to the gases inlet 121 in use.

In some configurations, a pressure of between about 3 cmH2O and about 10 cmH2O is provided to the gases inlet 121 in use.

In some configurations, a pressure of between about 4 cm H2O and about 30 cmH2O is applied to the gases inlet 121 in use.

The 'Bias' portion of each chart shows the effects of the openness of the open area for gases flow out of the nasal interface 100 through the bias flow restriction 140 on rebreathing and washout. 'Filterless' indicates a more open bypass restriction, where no filter or diffuser is in place. 'Filtered' shows a more closed bypass restriction, where a filter or diffuser is in place on the bypass restriction.

As outlined above, in some configurations, the bias flow restriction 140 comprises an open area for gases flow out of the nasal interface 100 through the bias flow restriction 140. In some configurations, the open area is more than 0 mm² to about 40 mm², optionally between about 2 mm² and about 40mm², optionally between about 2 mm² and about 5 mm², optionally between about 12 mm² and about 40mm², optionally between about 20 mm² and about 30 mm².

In some configurations, the bias flow restriction 140 is configured such that a flow rate of the flow of gases out of the nasal interface through the bias flow restriction is more than 0 lpm to about 80 Ipm when a pressure of more than 0 cmH2O and up to about 30 cmH2O is provided to the gases inlet 121 in use.

In some configurations, the bias flow restriction 140 is configured such that a flow rate of the flow of gases out of the nasal interface through the bias flow restriction is between about 4 lpm and about 15 lpm when a pressure of between about 3 cmH2O and about 10 cmH2O is provided to the gases inlet 121 in use.

In some configurations, the bias flow restriction 140 is configured such that a flow rate of the flow of gases out of the nasal interface through the bias flow restriction is between about 15 lpm and about 80 Ipm when a pressure of between about 4 cmH2O and about 30 cmH2O is provided to the gases inlet 121 in use.

The plots show that at lower breath rates such as 15 BPM and 25 BPM, increasing the nasal delivery element combined cross-sectional area, reducing the bypass restriction cross-sectional area, increasing the pressure applied to the gases inlet, and/or increasing the open area for gases flow through the bias flow restriction 140 reduces the amount of rebreathing and increases the amount of washout.

The combined cross-sectional area A₃+ A₄ of the nasal delivery elements 111, 112 may be maximised to increase washout, up to a size that comfortably fits within a patient's nares.

The bypass restriction 130 cross-sectional area A₂ may be minimised to increase washout, although increasing that cross-sectional area may enhance patient comfort.

The plots show that at higher breath rates such as 45 BPM, varying the nasal delivery element cross-sectional area or the bypass restriction may have negligible effect, due to rebreathing in the gases conduit (hence the left two boxes in the 45 BPM plot are shaded grey as they are not statistically significant to results). Increasing the pressure applied to the gases inlet 121, and/or increasing the open area for gases flow through the bias flow restriction 140 reduces the amount of rebreathing and increases the amount of washout.

A headgear may be used to retain the nasal interface 100 against the patient's face. The headgear comprises a head strap 200. The head strap 200 may be a single continuous length and adapted to extend in use along the patient's cheeks, above the ears and about the back of the head, may be adjustable, and/or may extend around other portions of the patient's head.

The headgear has ends that connect to the side arms of the interface body 110.

In the exemplary configuration shown (Figure 18), primary end portions 201 and 202 of the strap 200 are adapted to releasably connect respective formations 101 and 102 on either side of the nasal interface 100 to hold the nasal interface 100 in position during use.

In one configuration, a clip component is provided at each end portion 201, 202 capable of being received and retained within the corresponding formation 101, 102. The clip component may be coupled to the strap at the respective primary end portion. Furthermore, the head strap 200 is adjustable in length to help customise the strap to the wearer's head. The strap 200 may be formed from a soft and stretchable/elastic material such as an elastic, textile material/fabric that is comfortable to the wearer. Alternatively, the strap 200 may be formed from a substantially more rigid, or less flexible, material such as a hard plastics material.

The headgear may further comprise an additional strap or other headgear component that couples the strap 200 to extend over the patient's crown in use. A crown strap or crown component can have the benefit of pulling the strap 200 up and above the patient's ears in use to improve fit and comfort.

Rear portions of the strap 200 may extend through a receiver 204. The receiver 204 may allow the rear portions of the strap 200 to be adjusted to adjust the size of the headgear to fit a patient's head.

Strap segments of a fixed length can be releasably connected to the main strap to extend its length.

A number of strap segments of varying predetermined lengths may be provided to provide alternative adjustment lengths. For example, one or more strap segments may be provided having a length within the range of about 1cm to about 10cm, or within the range of about 2cm to about 6cm. The strap segments 220 have lengths of, for example, about 2cm, about 4cm or about 6cm. It will be appreciated that these examples are not intended to be limiting and the length of each strap segments can be of any size as it is dependent on the user and/or application.

Furthermore, each end of each strap segment may be connectable to a respective end of another strap segment and/or to a respective secondary end portion of the main strap 210 to thereby enable a user to combine one or more strap segments of the same or varying lengths to customise the overall length of the extension as desired.

The additional strap segments may be formed from a soft and stretchable/elastic material such as an elastic, textile material/fabric that are comfortable to the wearer. For example, a tubular knitted type head strap or sections of the head straps 210 may be utilised, particular for comfort over a user's ears.

It will be appreciated that particular comfort may be achieved from a head strap which is able to provide suitable locating of the nasal interface 100 in a relatively stable position on a user's face, yet simultaneously provide for a relatively loose fit or low tension fit about the user's head.

Alternatively, the additional strap segments may be formed from a substantially rigid material such as a hard plastics material.

Interface connectors 240 are provided at the primary end portions 201 and 202 of the main strap 210. These connectors 240 have a strap connection mechanism to connect the primary end portions 201, 202, but include a clip member, such as a push fit clip 241, at an end of the connector 240 opposing the strap ends. The clip 241 is configured to releasably couple the respective formation 101, 102 at the side of the nasal interface 100. The clip member 241 may be a bendable part, such as a plastic part, that forms a hinged portion relative to the strap. The clip 241 may be preformed to have a curved shape along its length, such as one with an angle between flat and 20 degrees for example. This curve allows the clip 241 to fit the contour of the patient's face in the region of the clip 241.

The nasal interface may comprise sleeves 270. Each sleeve 270 may be pre-formed to have a curved shape along its length, such as one with an angle between flat and 20 degrees for example. The curve allows the sleeve to fit the contour of the patient's face or cheek in the region of the sleeve in use. Alternatively, the sleeve 270 may take on the shape of a curved sleeve upon engagement with the primary end portion 201, 202 or connector 240 of the head strap 200.

The sleeve 270 provides a surface region of relatively higher frictional surface material for frictionally engaging with the user's face or facial skin. This surface region is to be positioned for frictional engagement with the facial cheek skin of a user. The surface region is at least localised to the strap or the section of strap which is to be positioned upon the cheeks of a user. The surface region provided with the relatively higher frictional surface material may be of a material that is smooth and comfortable on the skin of the patient. The sleeve 270 or at least the surface region 271 is therefore formed from a relatively softer material than the connector 240.

In one configuration, the surface region 271 or the sleeve 270 is formed from a soft Thermoplastic Elastomer (TPE), but may alternatively be formed from another plastics material such as silicone, or any other biocompatible materials.

The surface region 271 may be a surface of wider surface area more adjacent to the patient interface than the surface area more distant from the patient interface. In one configuration, the sleeve 270 tapers from a relatively wider surface area 273 to a relatively lesser surface area 274 in a direction extending away from a connection point between the connector 240 and the nasal interface 100. The width of the sleeve at the end 273 may be the same or similar to the width of the tapered distal end of the corresponding wing portion 113, 114 of the face mount part 110. This provides a smooth transition between the nasal interface 100 and the headgear for improving aesthetics and achieving a visually appealing effect.

The sleeves 270 may be coloured to provide an identification of the nasal interface 100. As described herein, the nasal interfaces may be provided in different sizes such as small, medium, and large, for example. The sleeves 270 of each of those sizes may comprise different colours to represent the different sizes. Alternatively, or additionally, the sleeves may be coloured in a specific way to represent that the nasal interfaces have asymmetrical nasal delivery elements rather than symmetrical.

The headgear may comprise cheek supports 270 as described or similar, at or adjacent either side end of straps of headgear of the interface, which connect to the nasal interface, for frictionally engaging with the user's face to stabilise the mask on the face at the cheeks. Such headgear may again comprise a single head strap adapted to extend in use along the patient's cheeks, above the ears and about the back of the head, with ends comprising clips in any suitable form which couple to the nasal interface on either side (or are permanently attached to the nasal interface).

The patient interface 1 may comprise a tube retention clip (not shown). The tube retention clip can support the respiratory conduit 300 or other gases supply tube 16 from part of the patient interface 1. By supporting the respiratory conduit 300 or other gases supply tube from or near the nasal interface 100, bending moment applied to the respiratory conduit 300 or other gases supply tube 16 as a result of asymmetrical flow through the first and second nasal delivery elements 111, 112 and/or movement of the patient's head will be resisted by the tube retention clip, thereby enhancing patient comfort.

The patient interface 1 may have any one or more of the features and functionality described in PCT publication no. WO 2014/182179 or US patent no. 10,406,311.

As an alternative to a headgear, the patient interface may comprise a securement system of the type described in PCT publication number WO 2012/053910 or US patent no. 10,238,828.

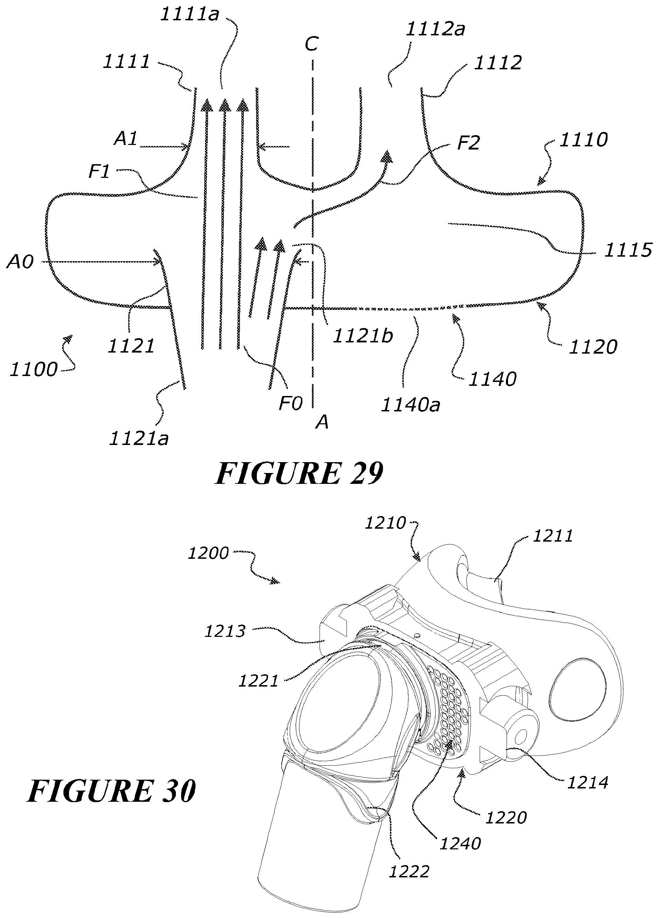

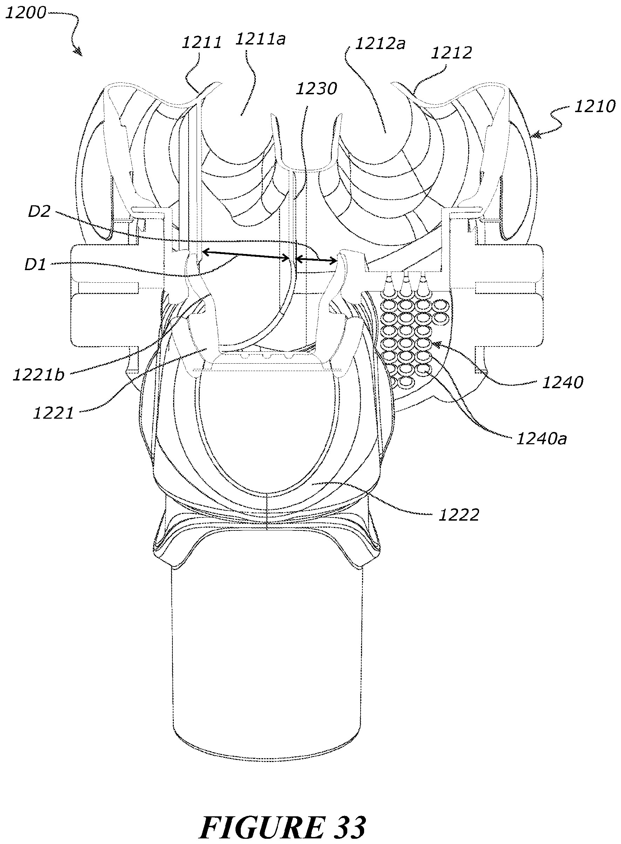

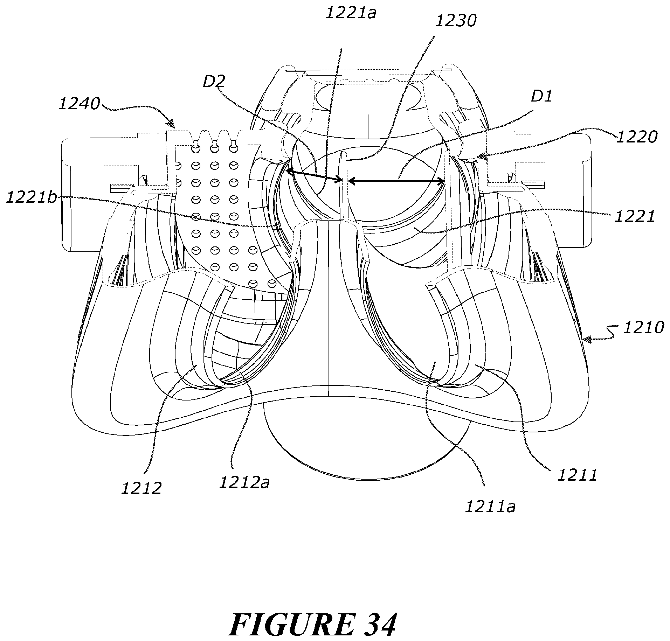

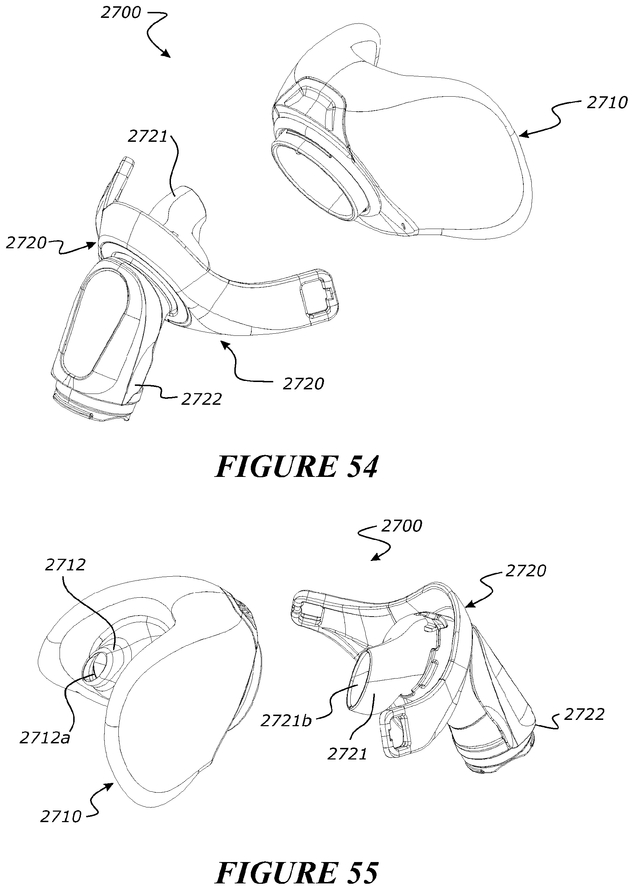

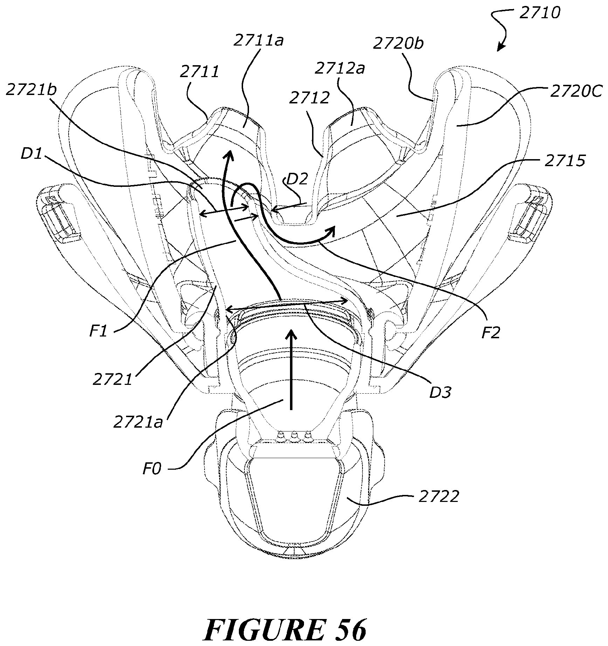

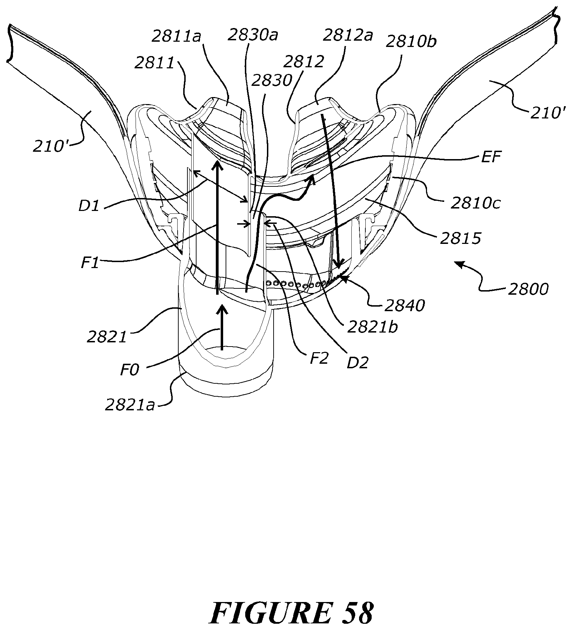

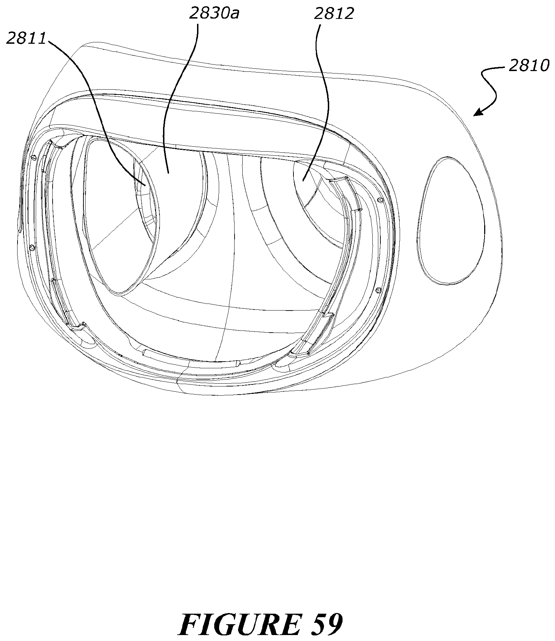

Figure 29 schematically shows an alternative configuration nasal interface 1100 for use in the patient interface 1. Unless described as being different below, the features, functionality, alternatives, and uses of the nasal interface 1100 are as described for nasal interface 100. Like reference numbers indicate like parts with the addition of 1000. Exemplary configurations of the nasal interface are described in more detail below with reference to Figures 30-59.

The nasal interface 1100 comprises an interface body 1110 configured to substantially form a seal with a patient's nasal airways. The interface body 1110 is configured to deliver gases to a first naris of the patient and to a second naris of the patient.

The nasal interface 1100 comprises a gases inlet 1121 for delivery of respiratory gases into the nasal interface 1100. The gases inlet 1121 is in fluid communication with the interface body 1110 to deliver the respiratory gases from the gases inlet 1121 through the interface body 1110 to the first naris and second naris of the patient in use.

The nasal interface 1100 is configured to receive incoming gases F0 from the gases inlet 1121 and to provide, from the incoming gases F0, a first flow stream of gases F1 configured to be substantially provided to the first naris of the patient in use and a second flow stream of gases F2 configured to be substantially provided to the second naris of the patient in use.

The nasal interface 1100 is configured to direct more of the incoming gases to the first flow stream of gases F1 than to the second flow stream of gases F2, to create an asymmetric flow of gases at a patient's nasal airways throughout a respiratory cycle of a patient.

Flow generated by respiratory therapy depends on flow through the nasal interface 1100. The flow through the nasal interface 1100 is related to pressure at each outlet 1111a, 1112a of the nasal interface. If the pressure is different at each outlet 1111a, 1112a, an asymmetric flow of gases will be generated.

The nasal interface may comprise distinct outlets 1111a, 1112a for delivery of the respiratory gases to the respective nares of the patient. Alternatively, the nasal interface may comprise a single outlet that defines a first outlet portion and a second outlet portion for delivery of the respiratory gases to the respective nares of the patient. Therefore, reference herein to "first outlet" and "second outlet" can instead be considered references to "first outlet portion" and "second outlet portion" respectively. Some of the possible exemplary configurations are described in more detail below.

Asymmetric flow is provided by the nasal interface 1100 by more flow being directed to the first naris/first outlet 1111a than to the second naris/second outlet 1112a. That may be considered flow directionality.

The nasal interface 1100 may be structured and configured to provide the flow directionality in different ways. For example, the nasal interface 1100 may comprise a flow director and/or a flow splitter and/or at least partial alignment of a gases inlet with the first outlet 1111a to provide the flow directionality. Some of the possible exemplary configurations are described in more detail below.

In some configurations, the nasal interface 1100 may comprise an interface body 1110 and a gases manifold 1120.

The interface body 1110 and gases manifold 1120 may cooperate to define a gases plenum 1115 therein. In some alternative configurations, the gases plenum 1115 may instead be defined substantially or solely by the interface body 1110. Instead of having a gases manifold 1120, the nasal interface 1100 may comprise a frame component to support the interface body and/or one or more other components (such as the gases inlet 1121, headgear 200, and/or the interface body 1110). Therefore, reference herein to "gases manifold" can instead be considered references to "frame".

The interface body 1110 may be configured to contact and seal internally in the nares of the patient, may be configured to contact and seal at the entrance to the nares of the patient, and/or may be configured to seal around the exterior surface of the nose, e.g. the alar and pronasale.

In some configurations, the interface body 1110 comprises a first outlet 1111a configured to substantially deliver gases to the first naris of the patient, and comprises a second outlet 1112a configured to substantially deliver gases to the second naris of the patient.

In some configurations, the interface body 1110 comprises first and second nasal delivery elements 1111, 1112 that are configured to engage with respective nares of the patient.

In some configurations, the interface body 1110 is a nasal cushion. The nasal cushion may comprise a single outlet that provides a first outlet portion and a second outlet portion. Alternatively, the nasal cushion may comprise first and second nasal delivery elements 1111, 1112 that are each configured to engage with a respective naris of the patient.

The nasal interface 1110 is structured and configured to create the asymmetric flow of gases at a patient's nasal airways throughout a respiratory cycle of a patient.

In the configuration shown in Figure 29, the gases inlet 1121 is at least partly aligned with the first outlet 1111a and is less aligned or is not aligned with the second outlet 1112a.

This configuration provides a substantially direct flow of gases from the gases inlet 1121 to the first outlet 1111a. The alignment of the gases inlet 1121 with the first outlet 1111a may act as a flow director.

The flow path for the flow of gases from the gases inlet 1121 to the second outlet 1112a is more tortuous than the flow path for the flow of gases from the gases inlet 1121 to the first outlet 1111a. In addition or alternatively, the flow path flow of gases from the gases inlet 1121 to the second outlet 1112a may be longer than the flow path for the flow of gases from the gases inlet 1121 to the first outlet 1111a.

The gases inlet 1121 is offset from a central axis C-A of the nasal interface 1100.

In some configurations, the gases inlet 1121 is substantially axially aligned with the first outlet 1111a.

In some configurations, at least half of a transverse cross-sectional area A0 of the gases inlet 1121 is axially aligned with at least half of a transverse cross-sectional area A1 of the first outlet 1111a.

The gases inlet comprises an outer portion 1121a for connecting to a respiratory conduit 300 or other gases supply tube 16 to provide a flow of gases from a gases source to the interface body 1110, and further comprises an inner portion 1121b in fluid communication with the interface body 1110.

The inner portion 1121b of the gases inlet 1121 is at least partly aligned with the first outlet 1111a or first outlet portion.

The inner portion 1121b and outer portion 1121a may be aligned with each other, or may be angled relative to each other.

In some configurations, the first outlet 1111a and the second outlet 1112a comprise substantially the same cross-sectional areas. That is, the flow asymmetry is caused by other features in the nasal interface 1100 rather than differing outlet sizes.

In some configurations, the first outlet 1111a and the second outlet 1112a may be symmetrically and structurally identical.

In some configurations, the nasal interface 1100 is configured to deliver a lower velocity of gases flow through the first outlet 1111a than a velocity of gases flow through the second outlet 1112a during an inhalation phase of a respiratory cycle.

Due to the less restricted flow path to the first outlet 1111a, the first flow stream of gases F1 has a lower velocity and higher pressure than the second flow stream of gases F2 along its more restricted flow path to the second outlet 1112a.

The nasal interface 1100 may comprise a restriction to restrict flow to the second outlet 1112a. The restriction may be provided by one or more of a flow director, a flow splitter, or any other suitable feature. The restriction may comprise a bypass restriction.

In some configurations, the nasal interface 1100 is configured to deliver a higher pressure of gases flow through the first outlet 1111a than a pressure of gases flow through the second outlet 1112a during an inhalation phase of a respiratory cycle.

In the configuration shown in Figure 29, the interface body 1110 comprises a first nasal delivery element 1111 comprising the first outlet 1111a and a second nasal delivery element 1112 comprising the second outlet 1112a, wherein the nasal interface 1100 is configured such that the first flow stream of gases F1 is configured to be substantially delivered to the first nasal delivery element 1111 and the second flow stream of gases F2 is configured to be substantially delivered to the second nasal delivery element 1112, and wherein the first nasal delivery element and the second nasal delivery element are each configured to seal with a respective naris of a patient.

In some configurations, the nasal interface comprises a flow director that is configured to direct more of the incoming gases F0 from the gases inlet 1121 to the first flow stream of gases F1 than to the second flow stream of gases F2.

Figure 29 shows a first exemplary configuration of the flow director. In this configuration, the flow director comprises the inner portion 1121a of the gases inlet 1121. Because the gases inlet is more aligned with the first outlet 1111a than the second outlet 1112a, the flow director directs more of the incoming gases F0 to the first flow stream of gases F1 than to the second flow stream of gases F2.

In some configurations, and as described below, the nasal interface comprises a connector or elbow for connecting a respiratory conduit 300 to the patient interface.

The connector or elbow may comprise or may be the flow director. That is, the connector or elbow may be the component that directs the flow more towards the first outlet 1111a than towards the second outlet 1112a.

In some configurations, the nasal interface 1100 is configured to direct more of the incoming gases to the first flow stream of gases F1 than to the second flow stream of gases F2 during an inhalation phase of the respiratory cycle. Additionally, this may also occur during an exhalation phase of the respiratory cycle. The inhalation phase and exhalation phase may define a respiratory cycle.

In some configurations, the flow path F1 to the first naris comprises a converging flow path. Additionally, or alternatively, the flow path F2 to the second naris comprises a diverging flow path.

In some configurations, the flow director comprises a nozzle that is configured to accelerate flow towards the first outlet 1111a.

In such a configuration, a first portion of the nozzle proximal to the gases inlet 1121 or proximal to an entry 1121a into the gases inlet may have a relatively large cross-sectional dimension, and a second portion of the nozzle distal from the gases inlet 1121 or distal from the entry 1121a into the gases inlet (and proximal to the gases plenum in the interface body 1110 and/or gases manifold 1120) may have a relatively small cross-sectional dimension. Due to the reduction in cross-sectional area, the nozzle will cause the gases to accelerate through the nozzle towards the first outlet 1111a.

In some configurations, the reduction in cross-sectional area of the nozzle may be a gradual reduction in cross-sectional area between the first portion and the second portion of the nozzle, such as a tapering. In another configuration, the reduction in cross-sectional area of the nozzle may be one or more substantially abrupt reductions in cross-sectional area provided between the first portion and the second portion of the nozzle, such as one or more step changes.

The nozzle may comprise part of the gases inlet 1121, or may be coupled or in fluid communication with the gases inlet 1121.

In some configurations, the nozzle may be provided in combination with an additional flow director. Alternatively, the nozzle may act as the flow director.

The nasal interface 1100 will be configured to simultaneously deliver the respiratory gases from the gases inlet 1121 through the interface body 1110 to both the first naris and second naris of the patient in use.

The nasal interface 1100 comprises a bias flow restriction 1140 comprising at least one aperture 1140a, and optionally a plurality of apertures 1140a, for the flow of gases from the nasal interface 1100 to an ambient environment.

The bias flow restriction 1140 may provide the functionality described above for the bias flow restriction 140.

There will typically be a positive flow of gases out of the patient interface 1100 through the bias flow restriction 1140, during respiratory therapy.

The bias flow restriction 1140 may comprise a filter and/or a diffuser to filter or diffuse gases flowing through the aperture(s) 1140a. When a filter is used, in some configurations the filter may additionally act as a diffuser.

In the configuration shown, the bias flow restriction 1140 is provided in the gases manifold 1120.

In some configurations, the bias flow restriction is positioned closer to the second nasal delivery element 1112 and second outlet 1112a than to the first nasal delivery element 1111 and first outlet 1111a. This encourages exhaled gases to pass through the second nasal delivery element 1112 and out of the nasal interface through the bias flow restriction 1140.

The bias flow restriction 1140 could be positioned elsewhere rather than the gases manifold 1120.

There will be an asymmetric flow of gases through the first outlet 1111a and the second outlet 1112a when inlet gases are being delivered to the nasal interface 1100 and the nasal interface is not mounted on a patient and the outlets are free of restrictions. The pressures at the outlets 1111a, 1111b could be checked to determine that there is an asymmetric flow.

The nasal interface 1100 is configured to provide a larger dynamic pressure at the first naris of the patient in use and to provide a smaller dynamic pressure at the second naris of the patient in use.

Therefore, the nasal interface 1100 may be considered to comprise an interface body 1110 configured to substantially form a seal with a patient's nasal airways, the interface body 1110 configured to deliver gases to a first naris of the patient and to a second naris of the patient.

The nasal interface 1100 comprises a gases inlet 1121 for delivery of respiratory gases into the nasal interface, wherein the gases inlet 1121 is in fluid communication with the interface body 1110 to deliver the respiratory gases from the gases inlet 1121 through the interface body 1110 to the first naris and second naris of the patient in use.

The nasal interface 1100 is configured to provide a larger dynamic pressure at the first naris of the patient in use and to provide a smaller dynamic pressure at the second naris of the patient in use. The larger dynamic pressure at the first naris compared to the smaller dynamic pressure at the second naris of the patient in use creates an asymmetric flow of gases at a patient's nasal airways. The asymmetric flow of gases at the patient's nasal airways may be created during an inhalation phase of the respiratory cycle. Additionally, this may also occur during an exhalation phase of the respiratory cycle. The inhalation phase and exhalation phase may define a respiratory cycle. As such, an asymmetric flow may be provided at the nasal airways of the patient by the nasal interface 1100 throughout a respiratory cycle of a patient.

The nasal interface 1100 provides an asymmetric flow. In use of the nasal interface, there is a net flow from the first naris of the patient to the second naris of the patient throughout a respiratory or breath cycle when flow or pressure therapy is delivered to the patient, such as CPAP or BiPAP.

In some configurations, the patient may be spontaneously breathing.

The breath cycle could be described to have an inspiration phase, an inflection phase where the patient is neither inspiring or expiring (this phase could also be known as a breathing holding phase), and an expiration phase. The inflection phase may occur over a significantly shorter time period than the inspiration and/or expiration phase.

Flow enters the nasal interface 1100 from the gases inlet 1121 at some total pressure delivered by the breathing assistance apparatus. The flow has a static component of pressure and a dynamic component of pressure, where the dynamic component of pressure refers to the flow component.

The gases flow from the gases inlet gets divided in the nasal interface and the resulting first flow stream of gases F1, which may have a larger cross-sectional area (referred to here as A-1), is directed towards the first naris and the second flow stream of gases F2, which may have a smaller cross-sectional area (referred to here as A-2), is directed towards the second naris and the bias flow restriction 1140.

This division of flow may create a bias toward the first naris.

In some configurations, area A-1 is larger than Area A-2, and the pressure drop, or flow restriction for gases flow to pass through area A-2 is greater than through area A-1.

In some configurations, area A-1 is larger than Area A-2 and the majority of the gases flow is directed in the direction of the first naris.

In some configurations, Area A-1 may not be larger than area A-2, but incoming gases flow may be directed more toward the first naris than toward the second naris and/or the flow path for the second flow stream of gases F2 to the second naris may be more tortuous than the flow path for the first flow stream of gases F1 to the first naris.

In such configurations, at least a portion of the area A-2 may comprise a filter or a diffuser to filter or diffuse gases flowing through the second flow stream of gases F2 to the second naris. The filter or a diffuser provided in at least a portion of the area A-2 may act to create a bias toward the first naris.

The bias of flow toward the first naris results in the dynamic pressure at the first naris being larger than the dynamic pressure at the second naris. That dynamic pressure is the flow component of the total pressure since the flow is coming in at a direction with its energy directed towards the first naris.

During the inspiration phase of respiration, flow from the gases inlet 1121 enters both nares in different proportions with more flow entering the first naris than the second naris because of the bias created as described above. Flow that does not enter the first and/or second naris may exit the patient interface to atmosphere through the bias flow restriction 1140. In some configurations, it is possible that flow leaves the second naris, rather than entering the second naris during some or all of inspiration. Such flow may be a portion of the flow from the gases inlet 1121, or a flow exiting the patient's airway via the second naris, or a combination thereof.

During the breath holding phase, the flow from the gases inlet 1121 is divided in the nasal interface, and some of that divided flow enters the first naris and exits the second naris via the patient's airways. Flows (or portions thereof) from the divided flow and/or flow exiting the patient's airways via the first and/or second naris leave to atmosphere through the bias flow restriction 1140.

During the expiration phase of respiration, flow either leaves both nares, or flow may enter the first naris and exit the second naris depending on the configuration exiting through the bias flow restriction 1140. Some flow may flow back to the gases inlet 1121 through the nasal interface. If flow exits the first naris, then incoming gases stagnate and travel along with the flow from the first naris through the gases plenum 1115 toward the second naris and out to atmosphere via the bias flow restriction 1140. Since the total pressure at the second naris is less than at the first naris, if there is a net flow out of the lungs then flow will exit out of the second naris.

The nasal interface provides a pressure differential between the flow paths for the first flow stream of gases F1 and the second flow stream of gases F2.

In at least some configurations, the respiratory gases from the gases inlet 1121 are more likely to enter the first naris, as they are directed towards that naris, and there is a resistance to movement towards the second naris (e.g. in the form of a tortuous and/or reduced flow path). The second flow stream of gases F2 may need to backtrack or pass through a restriction to enter the second naris, but the first flow stream of gases F1 will not need to do so, causing more gas to flow to the first naris. Similarly, at the second naris, exhaled gases from the patient are more likely to exit via the second naris, as the exhaled gases are directed towards the bias flow restriction 1140, and there is a resistance to movement back towards the first naris (again by the tortuous and/or restricted flow path). As such, there is a dynamic pressure differential in both directions (into the first naris, and out of the second naris).

The nasal interface 1100 has a single gases inlet 1121. Therefore, the first and second outlets 1111a, 1112a or outlet portions receive their gases flow from the respiratory gases from the inlet.

The nasal interface 1100 may be used with a single gases source, such as a single flow generator for example.

Exemplary configurations of nasal interfaces that provide the functionality described for the nasal interface of Figure 29 are described below with reference to Figures 30-59. Unless described as being different below, the features, functionality, alternatives, and uses of the nasal interfaces are as described for nasal interface 1100 or any of the other described nasal interfaces. Like reference numbers indicate like parts with the addition of 100 for each exemplary configuration.

Figures 30-36 show an exemplary configuration of the nasal interface 1200.

The nasal interface 1200 comprises the interface body 1210 and a gases manifold 1220.

The gases manifold 1220 and interface body 1210 are coupled together to define a gases plenum 1215 therein. The gases plenum 1215 provides fluid communication between the gases inlet 1221 and the first and second outlets 1211a, 1212a.

The nasal interface 1200 comprises a bias flow restriction 1240 comprising at least one aperture for the flow of gases from the nasal interface 1100 to an ambient environment.

The bias flow restriction 1240 is at least partly aligned with the second outlet 1212a and is less aligned or is not aligned with the first outlet 1211a.

In the configuration shown, the bias flow restriction is substantially axially aligned with the second outlet 1212a.

The gases inlet 1221 is provided as part of, or is coupled to, a connector or elbow 1222 for connecting a respiratory conduit 300 to the patient interface 1200.

The connector or elbow 1222 enters the gases manifold 1220 from a front of the gases manifold. Alternatively, the connector or elbow 1222 may enter the gases manifold 1220 from a different position; for example a side of the gases manifold 1220 or beneath the gases manifold.

The nasal interface 1200 comprises connector portions 1213, 1214 for connecting headgear 200 to the gases manifold 1220 and/or interface body 1210.

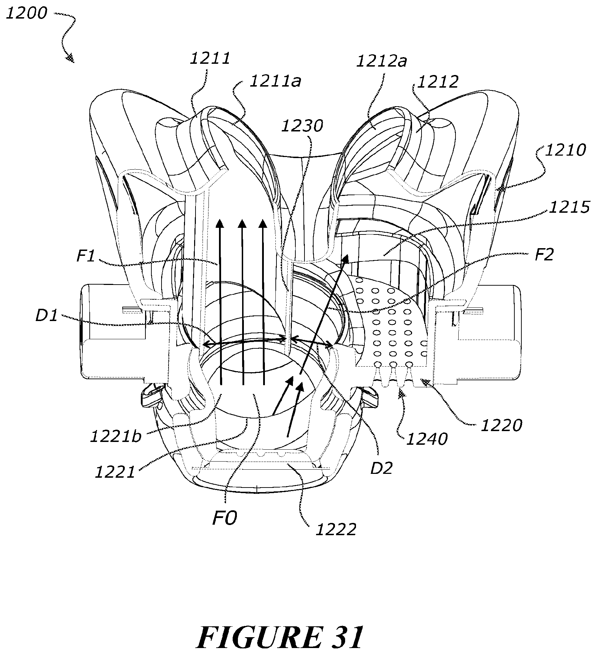

As shown in Figure 31, the incoming flow F0 is divided into two flow streams of gases F1, F2 along respective flow paths, which each lead to a respective outlet 1211a, 1212a and a respective naris.

As shown in Figure 31, the first flow stream of gases F1 has at least one dimension D1 that is larger than a corresponding dimension D2 of the second flow stream of gases F2. The same may apply to the other configurations of nasal interrace described herein.

The at least one dimension D1 may comprise a lateral dimension of the first flow stream of gases F1, and the corresponding dimension D2 may comprise a lateral dimension of the second flow stream of gases F2.

For example, the first flow stream of gases F1 may have a larger diameter, cross-sectional area, and/or volume than a corresponding diameter, cross-sectional area, and/or volume of the second flow stream of gases F2.

In some configurations, a ratio of the cross-sectional area (in the direction of dimension D1) of the first flow stream of gases F1 to the corresponding cross-sectional area (in the direction of dimension D2) of the second flow stream of gases F2 is between about 2:1 and about 5:1, optionally between about 2:1 and about 4:1, optionally between about 2.5:1 and about 3.5: 1, optionally about 3: 1.

In some configurations, the ratio of the cross-sectional area of the first flow stream of gases F1 to the corresponding cross-sectional area of the second flow stream of gases F2 is about 2:1, 2.25:1, 2.5:1, 2.75:1, 3:1, 3.25:1, 3.5:1, 3.75:1, 4:1, 4.25:1, 4.5:1, 4.75:1, 5:1, or is any value between any two of those values.

By way of example only, the combined cross-sectional areas of the first flow stream of gases F1 and the second flow stream of gases F2 at or adjacent the gases inlet 1221 may be about 200 mm², the cross-sectional area of the first flow stream of gases F1 may be about 150 mm² and the cross-sectional area of the second flow stream of gases F2 may be about 50 mm².

Although the above relationships of the at least one dimension D1 of the first flow stream of gases F1 and the corresponding dimension D2 of the second flow stream of gases F2 is described in relation to the configuration of Figures 30-36, the same relationships could be used in any of the configurations of Figures 29-59.

The flow stream relationships may differ slightly between inhalation and exhalation and/or may differ slightly as different pressures/flows are delivered by the flow generator.

In some configurations, the nasal interface 1200 is configured to provide less asymmetry during an inhalation phase of a respiratory cycle and more asymmetry during an exhalation phase of the respiratory cycle, but is configured to provide an asymmetric flow throughout the respiratory cycle. That is, a pressure differential of gases flow through the first outlet 1211a and the second outlet 1212a is higher during an expiration phase than during an inspiration phase.

Due to the directing of the flow, more flow is provided to the first outlet 1211a, and less flow is provided to the second outlet 1212a.

As such, the first nasal delivery element 1211 and first outlet 1211a is provided with a higher pressure than the second nasal delivery element 1212 and second outlet 1212a.

This creates a pressure differential between the nares, which provides for an asymmetric flow of gases at the patient's airways.

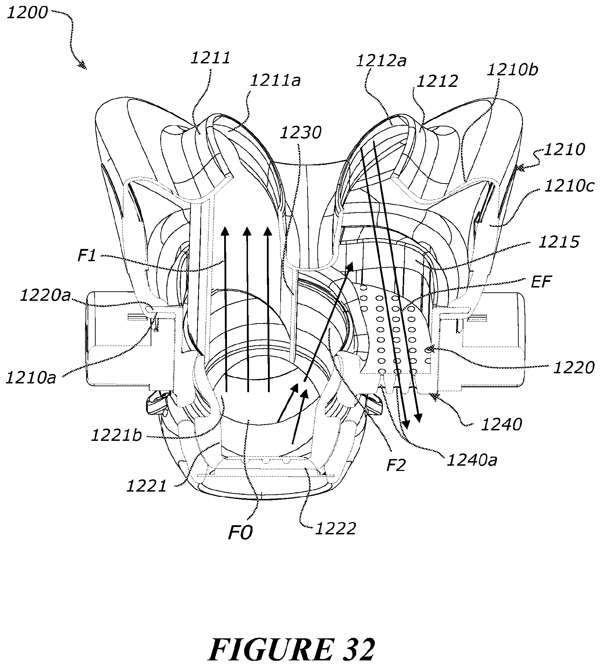

As more flow enters one naris (a first naris associated with the first outlet 1211a) than the other naris (a second naris associated with the second outlet 1212a), this means that the other naris can be used to exhale. This is represented graphically in Figure 32 which shows the predominant expiratory flow EF. The naris associated with the first outlet 1211a can also exhale, but the exhalation flow from that naris will take a longer and more tortuous flow path towards the bias flow restriction 1240.

This flushes dead space as the flow predominantly enters the first naris and exits the other naris.

As described elsewhere herein, the nasal interfaces can be used with pressure controlled therapies (i.e. CPAP, BiPAP). The asymmetrical flow in the nasal interfaces is a result of differential pressure.

In use of the nasal interfaces, if one naris is completely blocked, the pressure controlled therapy (i.e. CPAP, BiPAP) would be provided at the unblocked naris, without asymmetry. The work of rebreathing may increase. The nasal interfaces provide for this functionality by not directing 100% of the incoming gases flow to one naris by default.

In the configuration shown, the nasal interface 1200 has two flow director features. Different configurations of the nasal interface 1200 may have a single one of the flow director features or both flow director features.

The first flow director feature is the positioning of the gases inlet 1221 more towards the first outlet 1211a than to the second outlet 1212a, as described above in relation to Figure 29.

The second flow director feature comprises a flow splitter 1230 that is configured to unevenly split the flow F0 from the gases inlet 1221 into a first flow stream of gases F1 configured to be substantially provided to the first naris of the patient in use and a second flow stream of gases F2 configured to be substantially provided to the second naris of the patient in use. The first flow stream of gases F1 is configured to deliver a greater flow of gases along the first flow stream of gases F1 than a flow of gases along the second flow stream of gases F2, to create an asymmetric flow of gases at a patient's airways in use.

In some configurations, the nasal interface 1200 comprises: an interface body 1210 configured to substantially form a seal with a patient's nasal airways, the interface body 1210 configured to deliver gases to a first naris of the patient and to a second naris of the patient, and a gases inlet 1221 for delivery of respiratory gases into the nasal interface, wherein the gases inlet 1221 is in fluid communication with the interface body 1210 to deliver the respiratory gases from the gases inlet 1221 through the interface body 1210 to the first naris and second naris of the patient in use, and a flow splitter 1230 configured to unevenly split the flow from the gases inlet 1221 into a first flow stream of gases F1 configured to be substantially provided to the first naris of the patient in use and a second flow stream of gases F2 configured to be substantially provided to the second naris of the patient in use, wherein the first flow stream of gases F1 is configured to deliver a greater flow of gases along the first flow stream of gases F1 than a flow of gases along the second flow stream of gases F2, to create an asymmetric flow of gases at a patient's nasal airways throughout a respiratory cycle of a patient.

In some configurations, the nasal interface 1200 comprises: an interface body 1210 comprising a first nasal delivery element 1211 comprising a first outlet 1211a configured to deliver gases to a first naris of a patient and a second nasal delivery element 1212 comprising a second outlet 1212a configured to deliver gases to a second naris of a patient, wherein the first nasal delivery element 1211 and the second nasal delivery element 1212 are each configured to seal with a respective naris of a patient, and a gases inlet 1221 for delivery of respiratory gases into the nasal interface 1200, wherein the gases inlet 1221 is in fluid communication with the interface body 1210 to deliver the respiratory gases from the gases inlet 1221 through the first nasal delivery element 1211 and through the second nasal delivery element 1212, and a flow splitter 1230 to unevenly split the flow from the gases inlet 1221 into a first flow stream of gases F1 configured to be substantially provided to the first nasal delivery element 1211 and a second flow stream of gases F2 configured to be substantially provided to the second nasal delivery element 1212, wherein the first flow stream of gases F1 is configured to deliver a greater flow of gases along the first flow stream of gases F1 than a flow of gases along the second flow stream of gases F2, to create an asymmetric flow of gases at a patient's nasal airways throughout a respiratory cycle of a patient.

The flow splitter 1230 may be provided in the interface body 1210, the gases manifold 1220, and/or the gases inlet 1221. The gases inlet 1221 may be part of the elbow/connector 1222, or may be a separate component.

The flow splitter 1230 may be integrally formed with one or more of those components or may be separately formed and connected to one or more of those components.

The flow splitter 1230 may be a removable insert that is arranged to be connected to one or more of those components. The removable insert may be used to convert an existing nasal interface to an asymmetric nasal interface.

In the configuration shown, the flow splitter 1230 comprises a wall portion that extends towards or into the gases inlet 1221, wherein the first flow stream of gases F1 is located on one side of the wall portion and the second flow stream of gases F2 is located on an opposite side of the wall portion.

In some configurations, the flow splitter 1230 extends into the gases inlet, and splits the gases inlet 1221 into a first gases flow stream portion on said one first side of the flow splitter 1230 and a second gases flow stream portion on an opposite side of the flow splitter 1230.

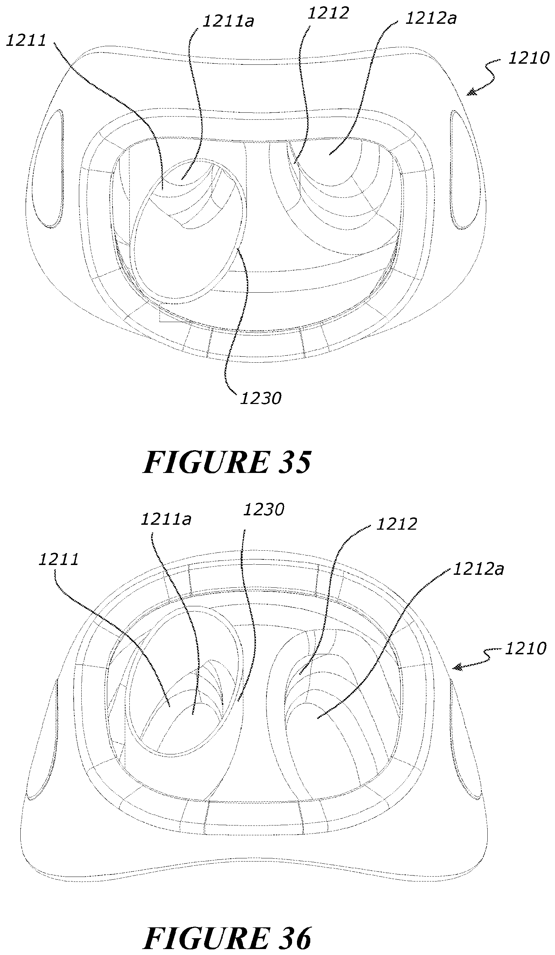

As shown in Figures 31-34 and in Figures 35 and 36 for example, the flow splitter 1230 may comprise a cylindrical wall that is aligned with the first outlet 1211a and that extends in a direction away from the first outlet 1211a (and towards or into the gases inlet 1221). Alternatively, the flow splitter 1230 may have a different configuration. For example, the flow splitter 1230 may comprise a wall (planar or another shape) positioned between the first nasal delivery element 1211 and the second nasal delivery element 1212.

The flow splitter 1230 may be a substantially rigid portion so as to provide a substantially constant relationship between the first flow stream of gases F1 and the second flow stream of gases F2.

In some configurations, the nasal interface may comprise a flow director that is configured to direct more of the incoming gases from the gases inlet 1221 to the first flow stream F1 than to the second flow stream F2. The flow director may be provided in addition to the flow splitter 1230.

In some configurations, the flow director may comprise a nozzle that is configured to accelerate flow towards the first outlet 1211a.

As shown in Figure 32, the body portion 1210 is provided with a coupling feature 1210a for engaging with a complementary coupling feature 1220a on the gases manifold 1220.

In the configuration shown, the coupling feature 1210a comprises an inwardly open recess and the complementary coupling feature 1220a comprises a radially outwardly extending flange that is received in the recess. Alternatively, the coupling feature 1210a may comprise a radially inwardly directed flange and the complementary coupling feature may comprise an outwardly open recess.

In the configuration shown, the interface body 1210 is a nasal cushion.

The nasal cushion is made of one or more compliant materials such as thermoplastic elastomer, latex, vinyl, silicone, or polyurethane for example.

Still referring to Figure 32, in some configurations, an inner portion 1210b of the nasal cushion that is configured to contact a user's face is more flexible than an outer portion 1210c of the nasal cushion that is not configured to contact a user's face. The outer portion 1210c is more rigid or stiff than the more flexible or supple inner portion 1210b. The inner portion 1210b comprises the first and second nasal delivery elements 1211, 1212 and/or the first outlet 1211a and the second outlet 1212a.

The more rigid outer portion 1210c supports the general shape of the nasal cushion. The more flexible inner portion 1210b enhances sealing against a patient's face and also enhances patient comfort.

In the configuration shown, at least part of the more rigid outer portion 1210c has a thicker wall than the more flexible inner portion 1210b. Additionally or alternatively, the more rigid outer portion 1210c may comprise one or more features to enhance its rigidity, such as one or more ribs for example.

The nasal cushion and nasal interface may have any one or more features outlined in US patent number 10,792,451 or US patent application publication number 2020/0046928.

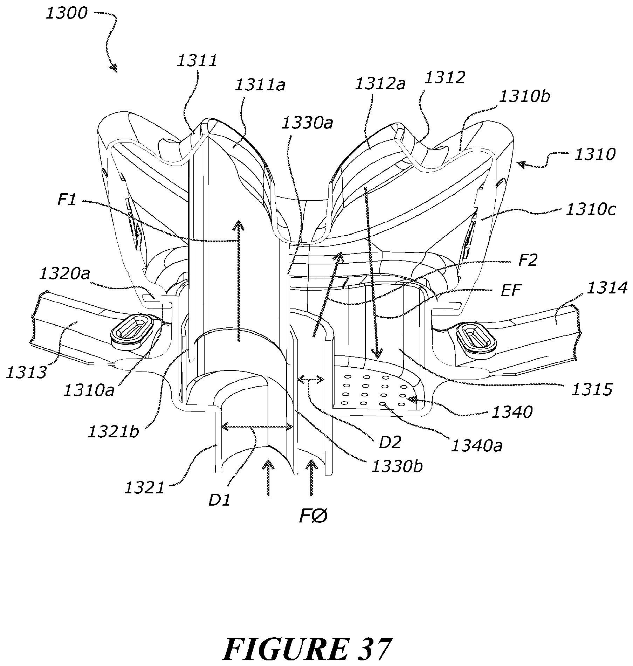

Figure 37 shows another exemplary configuration of the nasal interface 1300.

In this configuration, the flow splitter 1330 is provided in the interface body 1310 and the gases manifold 1320.

The flow splitter comprises a first splitter portion 1330a in the interface body 1310 and a second splitter portion 1330b in the gases manifold 1320.

The first splitter portion 1330a comprises a wall portion that extends towards or into the gases inlet 1321. The second splitter portion 1330b comprises a wall portion in the gases inlet 1321.

The second splitter portion 1330b splits the gases inlet 1321 into a first gases flow stream portion on one side of the second splitter portion 1330b and a second gases flow stream portion on an opposite side of the second splitter portion 1330b.

The first splitter portion 1330a and the second splitter portion 1330b are configured to be in close proximity to each other, and may contact each other or at least partly overlap.

The first flow stream of gases F1 is located on one side of the first splitter portion 1330a and the second splitter portion 1330b, and the second flow stream of gases F2 is located on an opposite side of the first splitter portion 1330a and second splitter portion 1330b.

The second splitter portion 1330b splits the gases inlet 1321 into a first gases flow stream portion on one side of the second splitter portion 1330b and a second gases flow stream portion on an opposite side of the second splitter portion 1330b.

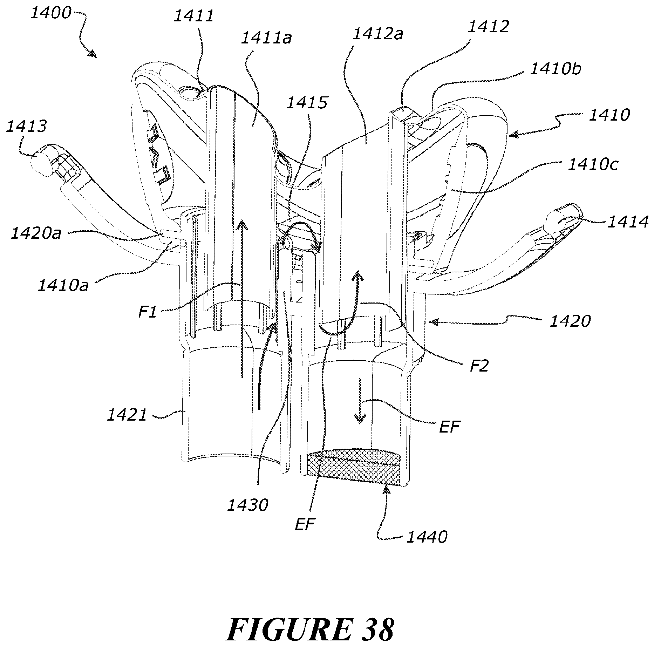

Figure 38 shows another exemplary configuration of the nasal interface 1400.

This configuration uses gases inlet alignment and a tortuous flow path to create an asymmetric flow of gases at a patient's nasal airways throughout a respiratory cycle of a patient.

In this configuration, the gases inlet 1421 is substantially aligned with the first outlet 1411a or first outlet portion. This provides a substantially direct flow path for first flow stream of gases F1 to the first outlet 1411a.

The flow splitter 1430 provides a restricted tortuous flow path (indicated by the arrows near reference number 1415 in Figure 38) for the second flow stream of gases F2 to the second outlet 1412a.

The tortuous flow path increases flow velocity and thus reduces pressure at the second outlet 1412a.

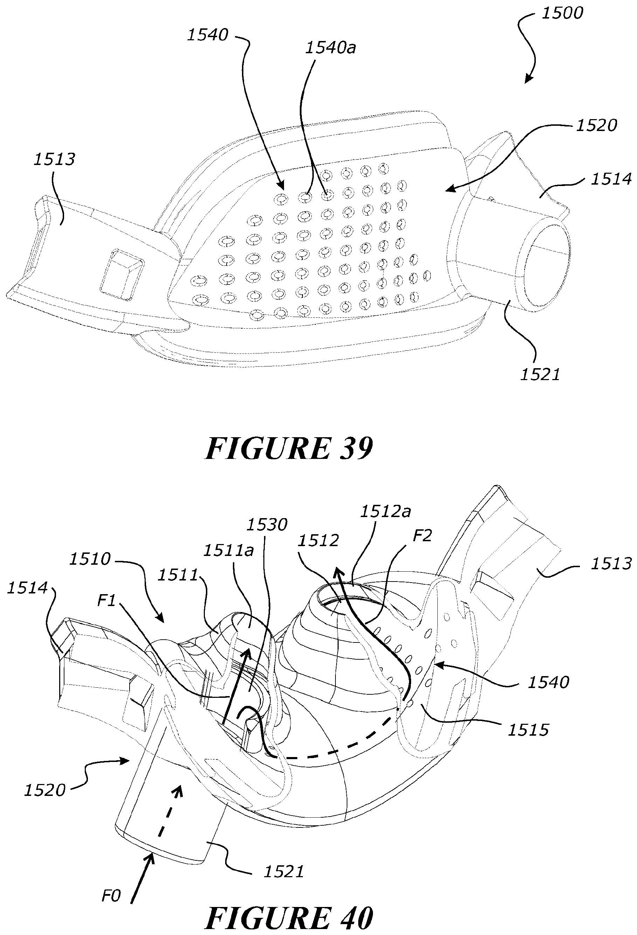

Figures 39 and 40 show another exemplary configuration of the nasal interface 1500.

This configuration uses gases inlet alignment and a tortuous flow path to create an asymmetric flow of gases at a patient's nasal airways. The asymmetric flow of gases at the patient's nasal airways may be created during an inhalation phase of the respiratory cycle. Additionally, this may also occur during an exhalation phase of the respiratory cycle. The inhalation phase and exhalation phase may define a respiratory cycle. As such, an asymmetric flow may be provided at the nasal airways of the patient by the nasal interface 1100 throughout a respiratory cycle of a patient.

In this configuration, the gases inlet 1521 is substantially aligned with the first outlet 1511a or first outlet portion. This provides a substantially direct flow path for the first flow stream of gases F1 to the first outlet 1511a.

A restriction 1530 is provided in the gases plenum 1515 that is formed between the gases manifold 1520 and interface body 1510.

The restriction provides a restricted tortuous flow path for the second flow stream of gases F2 to the second outlet 1512a.

The tortuous flow path increases flow velocity and thus reduces pressure at the second outlet 1512a.

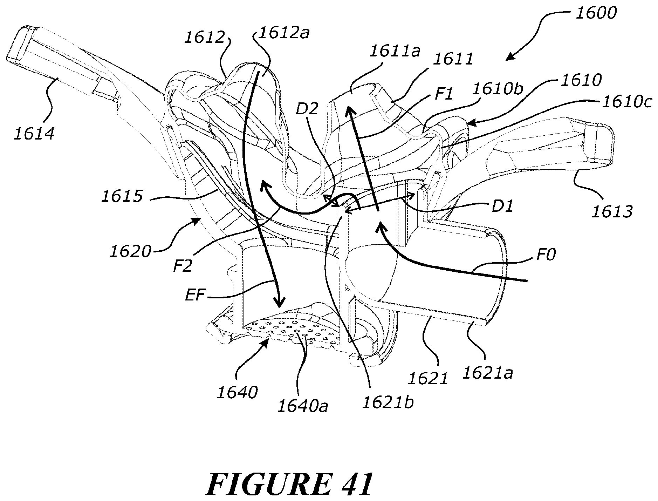

Figure 41 shows another exemplary configuration of the nasal interface 1600.

In this configuration, the gases inlet 1621 enters the gases manifold 1620 from the side rather than the front.

The gases inlet 1621 comprises an outer portion 1621a for connecting to a respiratory conduit 300 to provide a flow of gases for a gases source to the interface body 1610, and further comprises an inner portion 1621b in fluid communication with the interface body.

The inner portion 1621b of the gases inlet 1621 is at least partly aligned with the first outlet 1611a or first outlet portion.

The gases inlet 1621 comprises a directional change between the outer portion 1621a and the inner portion 1621b.

The directional change may be any suitable angle. In some configurations, the directional change may be between about 30 degrees and about 100 degrees, optionally between about 45 degrees and about 100 degrees, optionally between about 60 degrees and about 100 degrees, and optionally about 90 degrees.

Again, the nasal interface 1600 provides a restricted tortuous flow path for the second flow stream of gases F2.

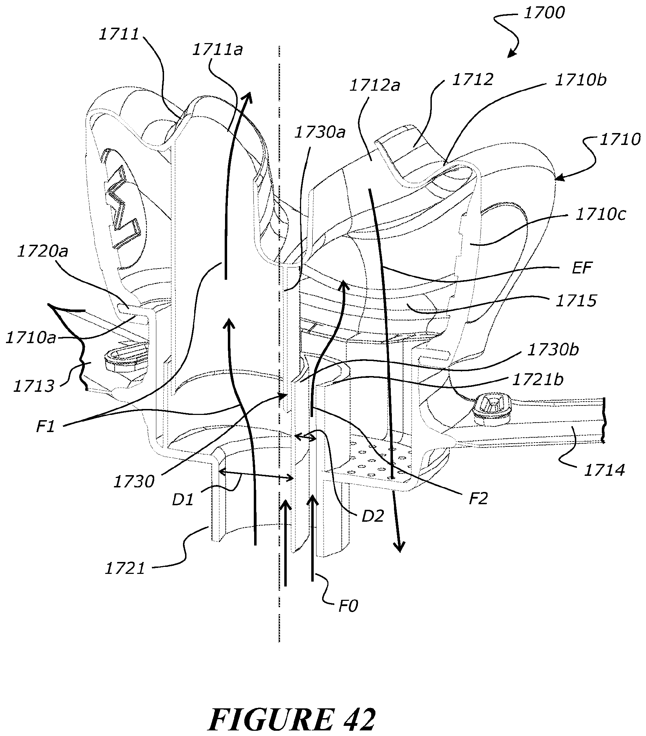

Figure 42 show another exemplary configuration of the nasal interface 1700.

The flow splitter 1730 of this configuration is similar to that of Figure 37.

This configuration differs in that rather than being aligned with the first outlet 1711a, the gases inlet 1721 enters the nasal interface more centrally. That is, an axis along the centre of the gases inlet would be relatively centrally located between axes that extend through the outlets 1711a, 1712a.

In some configurations, the gases inlet 1721 is centrally located on the nasal interface.

In the configuration of Figure 42, the flow splitter 1730 alone may provide the flow directionality.

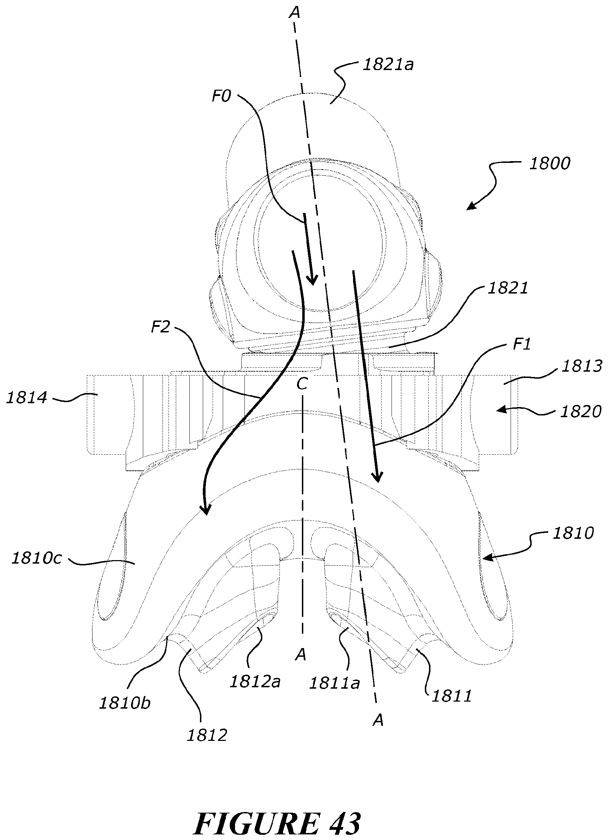

Figure 43 shows another exemplary configuration of the nasal interface 1800.

In this configuration, the gases inlet 1821 is offset from, but angled towards, the first outlet 1811a or first outlet portion. That directs the gases flow more towards the first outlet 1811a or first outlet portion than to the second outlet 1812a or second outlet portion.

Due to the angling of the gases inlet 1821, an axis A-A extending through the gases inlet 1821 is at a non-parallel angle relative to a central axis C-A through the nasal interface 1800.

The angle will depend on the amount of offset between the gases inlet 1821 entry into the gases manifold 1820 and the first outlet 1811a.

In some configurations, the angle is more than 0 degrees and up to about 30 degrees, optionally up to about 20 degrees, optionally up to about 15 degrees, and optionally up to about 10 degrees.

The angled gases inlet 1821 configuration may be used in a nasal interface with a centrally located gases inlet, or in a nasal interface with a gases inlet that is offset towards the first outlet 1811a or first outlet portion.

This angled gases inlet 1821 configuration may be the only feature of the nasal interface 1800 that directs the flow towards the first outlet 1811a or first outlet portion. Alternatively, the nasal interface 1800 may have one or more of the other flow directing features described herein, such as the flow splitter for example.

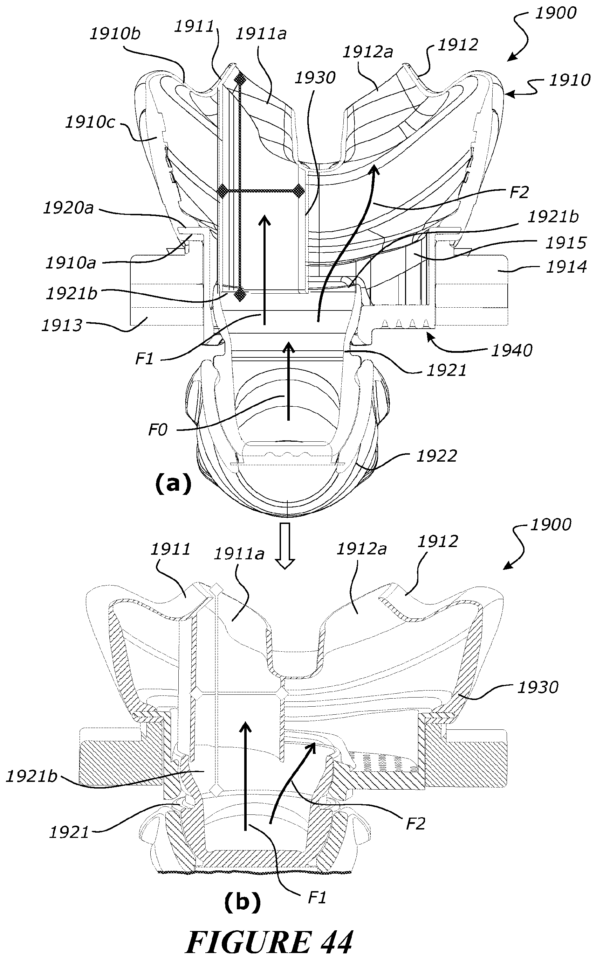

Figure 44 shows another exemplary configuration of the nasal interface 1800.

In this configuration, the interface body 1910 is a nasal cushion.

In order to properly seal with a patient's face, and to provide a comfortable experience for the patient, the nasal cushion is flexible and designed to compress/deform against the patient's face. The cushion may compress/deform in multiple directions as it is positioned on and contacts the face.

The nasal cushion comprises the flow splitter 1930. The flow splitter 1930 in this configuration is configured to move and/or deform upon compression of the nasal cushion.

The compression/deformation of the nasal cushion can cause the ratio or proportion of flow directing or splitting to be varied between patients, depending on the level of compression/flexing in the nasal cushion.

The shape of the flow split may be modified by the movement and/or deformation of the flow splitter 1930, causing the ratio between the first flow stream of gases F1 and second flow stream of gases F2 to change.

The flow splitter 1930 may be flexible, and may be configured to deform upon compression/deformation of the nasal cushion. Alternatively, the flow splitter 1930 may be more rigid.

In the configuration shown, the flow splitter 1930 is configured to move more towards or into the gases inlet 1921 upon compression/deformation of the nasal cushion, as shown in Figure 44(b). This may cause more flow to be directed along the first flow stream of gases F1 than the second flow stream of gases F2, compared to an at-rest position of the nasal cushion (shown in Figure 44(a)).

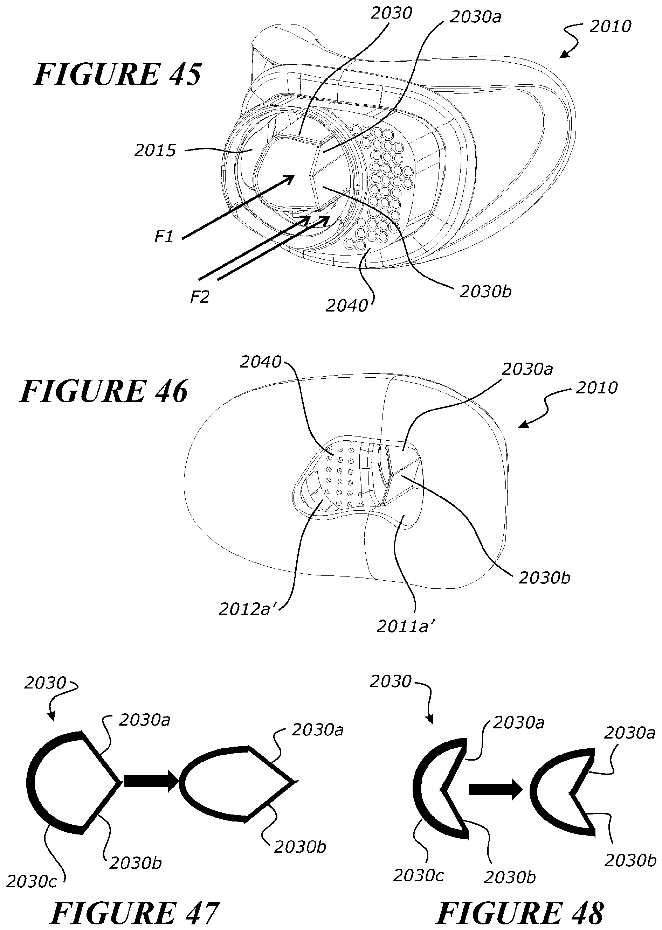

Figures 45 and 46 show an alternative exemplary configuration of a nasal cushion 2010 that may be used as the interface body in any of the nasal interfaces disclosed herein.

In this configuration, the nasal cushion 2010 comprises a single outlet for delivering gases to the first naris and second naris of the patient. The single outlet comprises the first outlet portion 2011a' and the second outlet portion 2012a'. The nasal cushion 2010, and thereby the nasal interface, is configured such that the first flow stream of gases F1 is configured to be substantially delivered to the first outlet portion 2011a' and the second flow stream of gases F2 is configured to be substantially delivered to the second outlet portion 2012a'.

In the configuration shown, the nasal cushion 2010 comprises a flow splitter 2030 with a first wall portion 2030a and a second wall portion 2030b. The first wall portion 2030a and the second wall portion 2030b are hingedly connected to each other. Relative angles of the first and second wall portions 2030a, 2030b are configured to change upon deformation/compression of the nasal cushion 2010.

The deformation in the flow splitter 2030 may be configured to maintain a substantially constant ratio between the first flow stream of gases F1 and the second flow stream of gases F2 as the nasal cushion 2010 is deformed or compressed. Alternatively, the deformation in the flow splitter 2030 may be configured to change the ratio between the first flow stream of gases F1 and the second flow stream of gases F2 as the nasal cushion 2010 is deformed or compressed.

Figures 47 and 48 show exemplary deformations of the flow splitter 2030 as the nasal cushion 2010 is compressed.

As shown in these figures, in some configurations an outer peripheral wall 2030c that is opposite to the first wall portion 2030a and the second wall portion 2030b may also deform upon deformation/compression of the nasal cushion 2010.

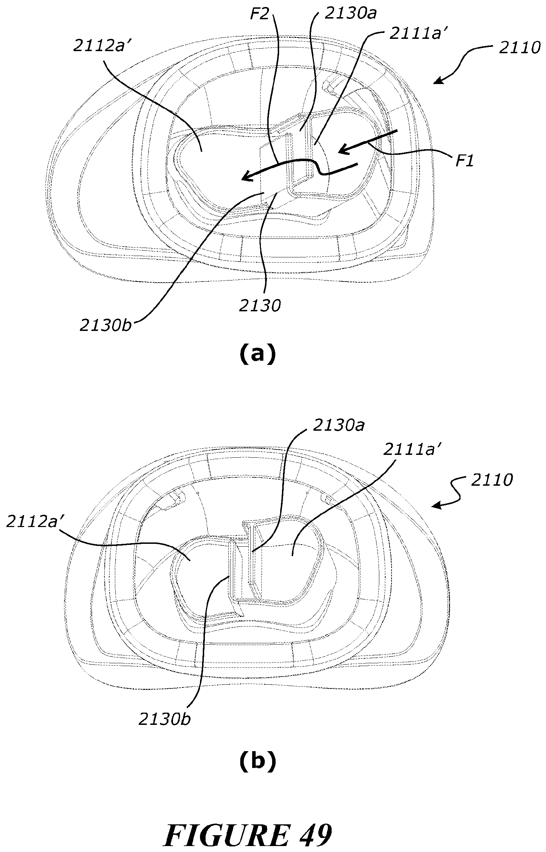

Figure 49 shows an alternative exemplary configuration of a nasal cushion 2110 that may be used as the interface body in any of the nasal interfaces disclosed herein.

Again, in this configuration, the flow splitter 2130 comprises a first wall portion 2130a and a second wall portion 2130b.

The first wall portion 2130a and the second wall portion 2130b overlap each other in a relaxed state of the nasal cushion 2110. The extent of overlap of the wall portions 2130a, 2130b increases upon compression of the nasal cushion 2110.

The configurations of Figures 47-49 may be used in a nasal cushion or interface body that has first and second nasal delivery elements with respective outlets, rather than with a single outlet that has first and second outlet portions.

As outlined above, any of the nasal interfaces disclosed herein could use a nasal cushion that has a single outlet for delivering gases to the first naris and second naris of the patient. The single outlet comprises the first outlet portion and the second outlet portion. The single outlet may be free of a distinct septum split between the nares, which can be more comfortable for a patient by not having a septum contacting portion.

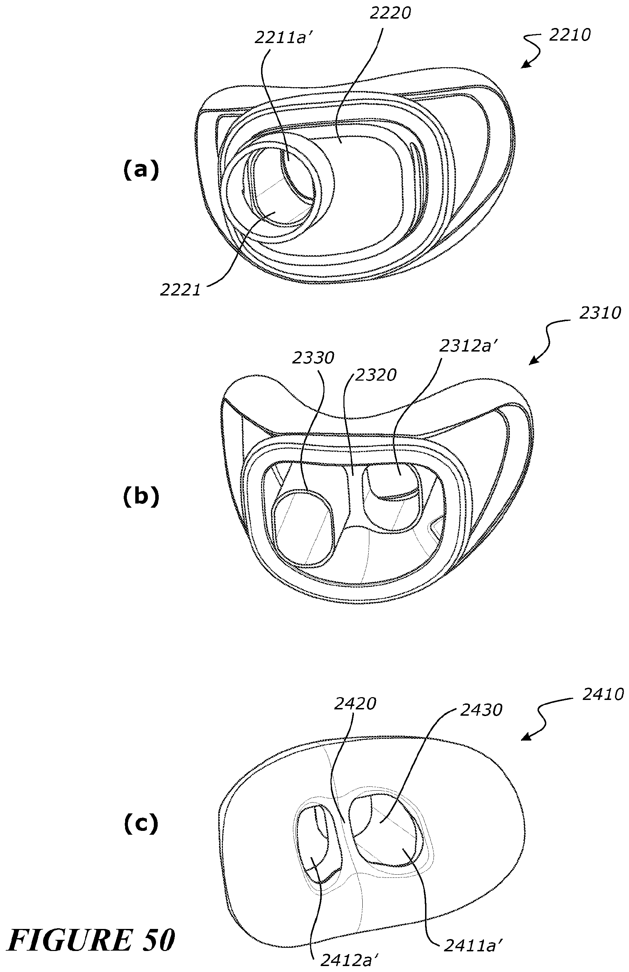

In such a configuration, the flow directing/flow split will occur before the outlet portions. Figure 50 shows three exemplary configurations of such nasal cushions 2210, 2310, 2410 in which the flow directing features or flow splitters 2330, 2430 are provided by a gases manifold portion 2220, 2320, 2420.

Alternatively, the flow directing feature may be provided by the nasal cushion, as described for the configuration of Figures 45-48 for example.

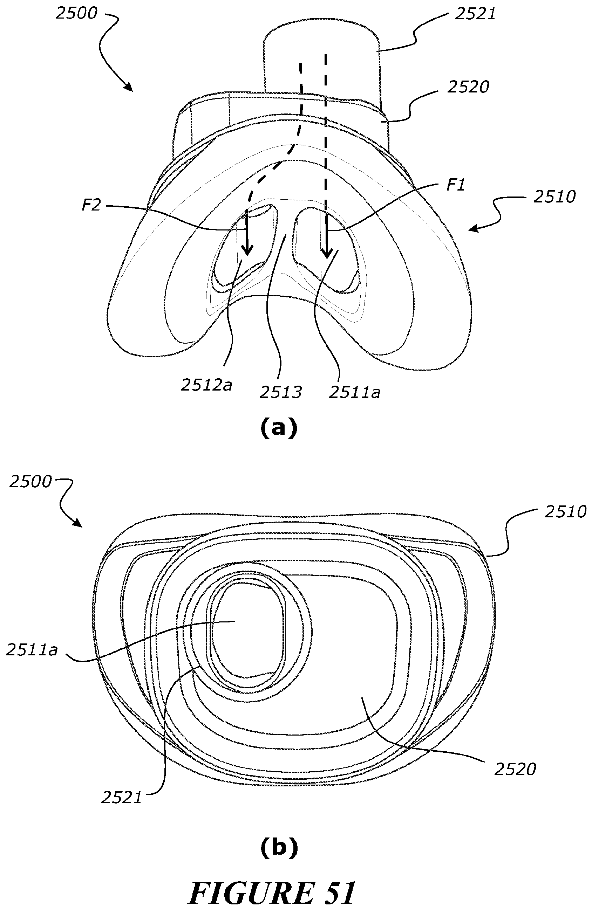

In an alternative configuration, the nasal cushion may have a septum contacting portion. An exemplary configuration of such a nasal cushion 2510 is shown in Figure 51.

The septum contacting portion 2513 forms a first outlet 2511a and a second outlet 2512a for delivering gases to respective nares of the patient.

The septum contacting portion 2513 may provide greater separation of flows between the first flow stream of gases F1 and second flow stream of gases F2 and reduce mixing of the flow streams before their delivery through the first and second outlets 2511a, 2512a.

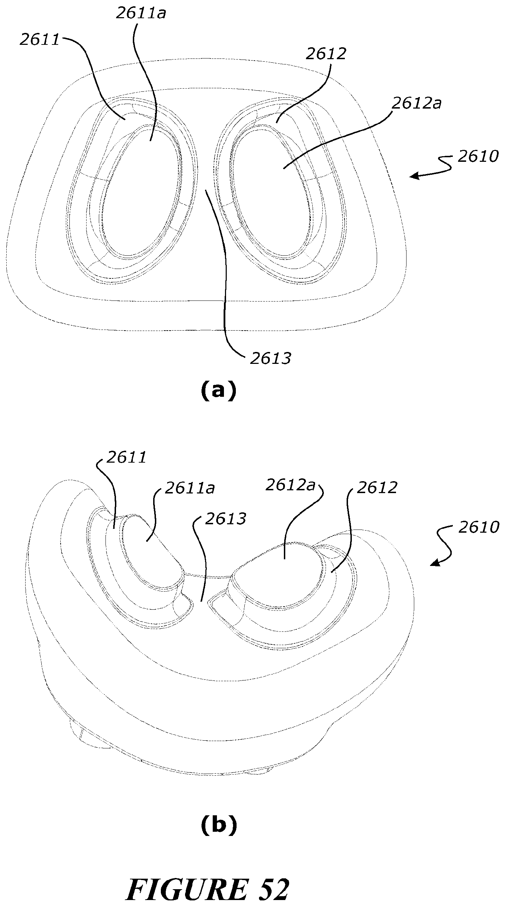

Figure 52 shows an alternative exemplary nasal cushion 2610.

This configuration comprises short first and second nasal delivery elements 2611, 2612. The nasal delivery elements are shorter than those shown in the embodiment of Figure 31 for example.

By having shorter nasal delivery elements, the first and second openings 2611a, 2612a may be larger than with longer nasal delivery elements.

The nasal delivery elements 2611a, 2612a form locating features to help locate the nasal delivery elements 2611a,2612a in the nares, and may assist with keeping the nares open.

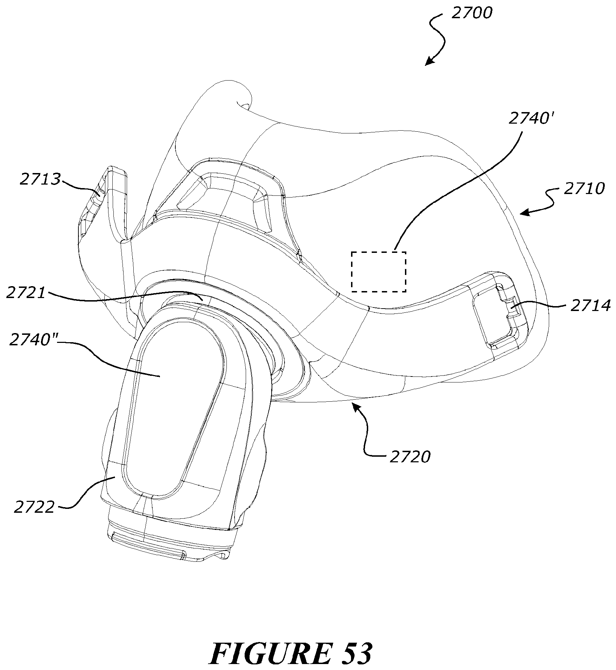

Figures 53-56 show another exemplary configuration of the nasal interface 2700.

In this configuration, the flow director comprises the gases inlet 2721 that is angled towards the first outlet 2711a. That directs the gases flow more towards the first outlet 2711a than to the second outlet 2712a.

The gases inlet 2721 comprises a nozzle that is configured to accelerate flow towards the first outlet 2711a or first outlet portion.

In the configuration shown, a first portion of the nozzle proximal to the entry 2721a to the gases inlet has a relatively large transverse cross-sectional dimension D3, and a second portion of the nozzle distal from the entry 2721a into the gases inlet (and proximal to the gases plenum 2715 in the interface body 2710 and/or gases manifold 2720) has a relatively transverse small cross-sectional dimension. The nozzle will cause the gases to accelerate through the nozzle towards the first outlet 2711a or outlet portion.

In some configurations, the outlet at the second portion of the nozzle has a cross-sectional area of between about 15 mm² and about 150 mm².

The nozzle may comprise part of the gases inlet 2721, or may be coupled or in fluid communication with the gases inlet.

In the configuration shown, the gases inlet 2721 is part of the connector or elbow 2722. Therefore, the connector or elbow 2722 is configured to direct the gases flow more towards the first outlet 2711a than to the second outlet 2712a.

The gases inlet 2721 and connector or elbow 2722 may be integrally formed or may be coupled to each other.

In the configuration shown, the nozzle acts as the flow director. In an alternative configuration, the nozzle may be provided in combination with an additional flow director. For example, the nozzle may be shorter than what is shown, and an additional flow director in the interface body 2710 and/or gases manifold 2720 may direct the flow from the nozzle more towards the first outlet 2711a or first outlet portion than towards the second outlet 2712a or second outlet portion.

The second flow stream of gases F2 has a more restricted tortuous flow path than the first flow stream of gases F1.

The bias flow restriction may be provided in any suitable position on the nasal interface 2700.

In one configuration, the bias flow restriction 2740' is provided in the interface body 2710/nasal cushion. In another alternative, the bias flow restriction 2740" may be provided in the gases manifold 2720.

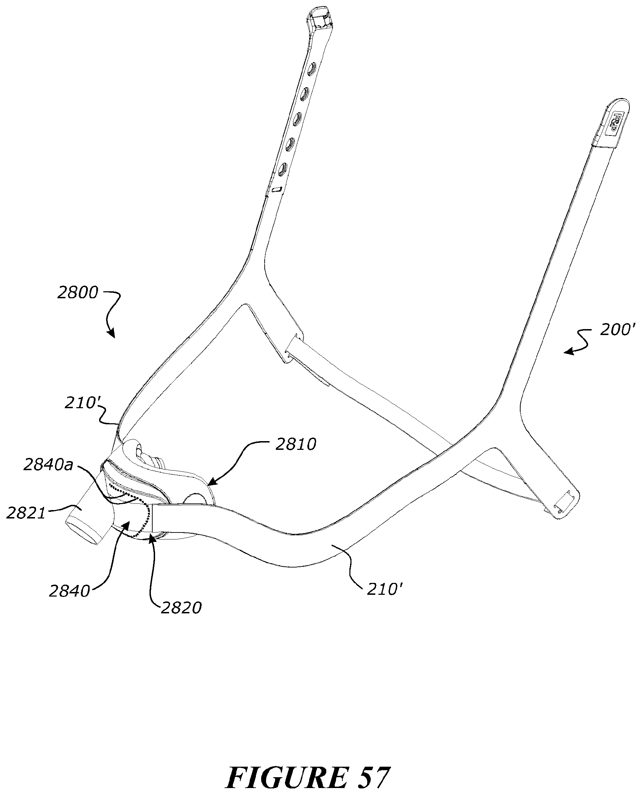

Figures 57-59 show another exemplary configuration of the patient interface 2800.

In this configuration, the flow splitter 2830 is provided in the interface body 2810 and the gases manifold 2820.

The flow splitter comprises a first splitter portion 2830a in the interface body 2810 and a second splitter portion 2830b in the gases manifold 2820.

The first splitter portion 2830a comprises a wall portion that extends towards or into the gases inlet 2821. The second splitter portion 2830b comprises a wall portion of the gases inlet 2821.

The first splitter portion 2830a is received in the second splitter portion 2830b of the gases inlet 2821.

The first splitter portion 2830a splits the gases flow F0 from the gases inlet into a first gases flow stream portion on one side of the first splitter portion 2830a and a second gases flow stream portion on an opposite side of the first splitter portion 2830a.

The first splitter portion 2830a and the second splitter portion 2830b are configured to be in close proximity to each other, and partly overlap.

A gap is provided between the outside of the first splitter portion 2830a and the inside of the second splitter portion. That gap provides a flow path for the second flow stream of gases F2, which travels on a restricted tortuous path to be delivered to the second outlet 2812a or second outlet portion.

In the configuration shown, the bias flow restriction 2840 comprises an array of apertures 2840a on the front of the gases manifold 2820.

In some configurations, the straps 210' of the headgear 200' may be integrally formed with gases manifold 2820. For example, the straps 210' may be overmoulded with the gases manifold 2820.

As outlined above for the nasal interfaces 100, in some configurations of the nasal interface 1100, 1100, 1200, 1300, 1400, 1500, 1600, 1700, 1800, 1900, 2000, 2500, 2700, 2800, the nasal interface is configured to achieve a patient pressure at the first outlet or first outlet portion and the second outlet or second outlet portion of between about 2 cmH2O and about 30 cmH2O in use, optionally between about 2 cmH2O and about 25 cmH2O in use, optionally between about 2 cmH2O and about 20 cmH2O in use, optionally between about 2 cmH2O and about 15 cmH2O in use, optionally between about 2 cmH2O and about 14 cmH2O in use, optionally between about 2 cmH2O and about 13 cmH2O in use, optionally between about 2 cmH2O and about 12 cmH2O in use, optionally between about 2 cmH2O and about 11 cmH2O in use, optionally between about 2 cmH2O and about 10 cmH2O in use.

As outlined above for the nasal interfaces 100, in some configurations of the nasal interface 1100, 1100, 1200, 1300, 1400, 1500, 1600, 1700, 1800, 1900, 2000, 2500, 2700, 2800, a pressure differential between the first outlet 1111a or first outlet portion and the second outlet 1112a or second outlet portion is configured to provide an asymmetric flow through upper airways of a patient of at least about 1 liter per minute (lpm), optionally between about 1 lpm and about 5 lpm.

The asymmetric flow provided by the nasal interfaces 100, 1100, 1100, 1200, 1300, 1400, 1500, 1600, 1700, 1800, 1900, 2000, 2500, 2700, 2800 promotes clearing of CO₂ from anatomical dead space of the patient.

The nasal interfaces 100, 1100, 1100, 1200, 1300, 1400, 1500, 1600, 1700, 1800, 1900, 2000, 2500, 2700, 2800 disclosed herein are configured to simultaneously deliver the respiratory gases from the gases inlet through the interface body to both the first naris and second naris of the patient in use.

In some configurations, during an expiration phase, flow may leave one or both of the first and/or second nares as the patient exhales. Some of the exhaled gases from the first and/or second nares may then enter the first and/or second nares as the patient then inhales.

Patient interfaces 1 with nasal interfaces 100, 1100, 1200, 1300, 1400, 1500, 1600, 1700, 1800, 1900, 2000, 2500, 2700, 2800 according to the configurations described herein may be employed in a method of delivering gas to the airway of a patient in need thereof, improving the ventilation of a patient in need thereof, reducing the volume of anatomical dead space within the volume of the airway of a patient in need thereof, and/or treating a respiratory condition in a patient in need thereof, as described above.

Patient interfaces 1 comprising nasal interfaces 100, 1100, 1200, 1300, 1400, 1500, 1600, 1700, 1800, 1900, 2000, 2500, 2700, 2800 of the type disclosed herein may be used in a respiratory therapy system for delivering gases to a patient.

Because the patient interfaces 1 can comprise any of the nasal interfaces (or nasal interface components such as the interface bodies 2110, 2210, 2310, 2410, 2610) of the type disclosed herein, references herein and below to nasal interface 100 can instead be considered references to any of the other nasal interfaces 1100, 1200, 1300, 1400, 1500, 1600, 1700, 1800, 1900, 2000, 2500, 2700, 2800.

In some configurations, the respiratory therapy system 1000 comprises a respiratory therapy apparatus 1100 and a patient interface 1 comprising a nasal interface 100.

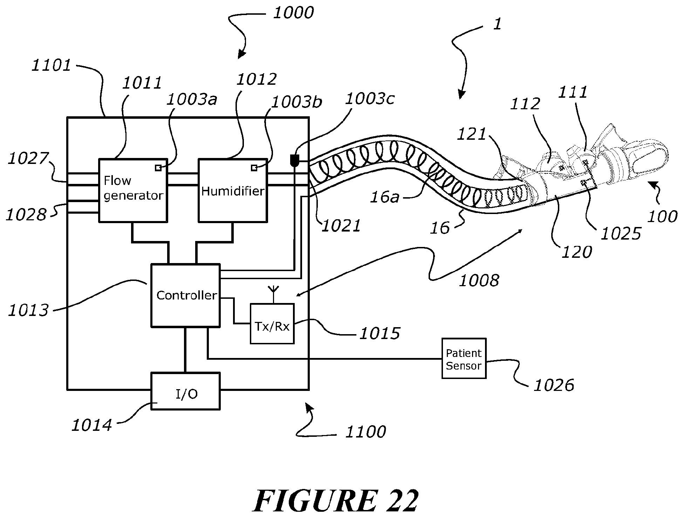

An exemplary respiratory therapy apparatus 1100 is shown in Figure 22.

The respiratory therapy apparatus 1100 comprises a main housing 1101 that contains a flow generator 1011 in the form of a motor/impeller arrangement (for example, a blower), an optional humidifier 1012, a controller 1013, and a user interface 1014 (comprising, for example, a display and input device(s) such as button(s), a touch screen, or the like).

The controller 1013 can be configured or programmed to control the operation of the apparatus. For example, the controller can control components of the apparatus, including but not limited to: operating the flow generator 1011 to create a flow of gas (gases flow) for delivery to a patient, operating the humidifier 1012 (if present) to humidify and/or heat the generated gases flow, control a flow of oxygen into the flow generator blower, receiving user input from the user interface 1014 for reconfiguration and/or user-defined operation of the apparatus 1000, and outputting information (for example on the display) to the user.

The user can be a patient, healthcare professional, or anyone else interested in using the apparatus. As used herein, a "gases flow" can refer to any flow of gases that may be used in the breathing assistance or respiratory device, such as a flow of ambient air, a flow comprising substantially 100% oxygen, a flow comprising some combination of ambient air and oxygen, and/or the like.

The controller 1013 may be implemented as a purely hardware controller, as a software regime running on controller hardware, or as software operational on other non-dedicated controller hardware of the device. Alternatively, it may be implemented as any number of combinations of the foregoing implementation examples.

In various forms the controller 1013 may include a processor and a memory.

It will be understood that the various detection, operation, sensing, comparison, enabling or disabling, triggering, pulsing, monitoring, receiving, determining and such like steps by the controller 1013 or as part of a method of operation of the device may be carried out autonomously, automatically, or dynamically. For example, the steps or various of the steps may be conducted by the controller, whether as a hardware, hardware and software, or internal or external system implementation, independent of any other input or control signal. As a further example, they may be conducted automatically in response to one or more precursor steps or preconditions.

Where a method or control step or structural element is described to be associated with another method or control step or structural element, this will generally be understood to indicate a relationship between the two features. In particular, relation to method or control steps, it may indicate a relationship of antecedence, dependence, subsequency, or general connection to the method or controls steps. In relation to structural elements, it may indicate a functional association between the structural elements, such as a working relationship to provide a particular result or a connection or synergy. Alternatively, in relation to structural elements it may indicate a direct physical relationship between the two structural elements. Where appropriate, further meaning of the term "associated" in relation to such elements or steps is to be understood from the surrounding context.

A breathing tube 16 is coupled at one end to a gases flow outlet 1021 in the housing 1100 of the respiratory therapy apparatus 1100. The breathing tube 16 is coupled at another end to the nasal interface 100 with the gases manifold 120 and nasal prongs 111, 112. The connection to the nasal interface 100 may be a direct connection to the nasal interface, or may be via a respiratory conduit 300 and optionally a filter 500.

The gases flow that is generated by the respiratory therapy apparatus 1100 may be humidified, and delivered to the patient via the breathing tube 16 through the nasal interface 100. The breathing tube 16 can have a heater to heat gases flow passing through to the patient. For example, the breathing tube 16 can have a heater wire 16a to heat gases flow passing through to the patient. The heater wire 16a can be under the control of the controller 1013. The breathing tube 16a, respiratory conduit 300 (when provided), and/or nasal interface 100 can be considered part of the respiratory therapy apparatus 1100, or alternatively peripheral to it. The respiratory therapy apparatus 1100, breathing tube 16, respiratory conduit 300 (when provided), and patient interface 1 comprising a nasal interface 100 together can form a respiratory therapy system 1000.

The controller 1013 can control the flow generator 1011 to generate a gases flow of the desired flow rate. The controller 1013 can also control a supplemental oxygen inlet to allow for delivery of supplemental oxygen, the humidifier 1012 (if present) can humidify the gases flow and/or heat the gases flow to an appropriate level, and/or the like. The gases flow is directed out through the breathing tube 16, respiratory conduit 300, and nasal interface 100 to the patient. The controller 1013 can also control a heating element in the humidifier 1012 and/or the heating element 16a in the patient conduit 16 to heat the gas to a desired temperature for a desired level of therapy and/or level of comfort for the patient. The controller 1013 can be programmed with or can determine a suitable target temperature of the gases flow. In some configurations, gas mixture compositions including supplemental oxygen and/or administration of therapeutic medicaments may be provided through the supplemental oxygen inlet. The gas mixtures compositions may comprise oxygen, heliox, nitrogen, nitric oxide, carbon dioxide, argon, helium, methane, sulfur hexafluoride, and combinations thereof, and/or the supplemental gas can comprise an aerosolized medicament.

Operation sensors 1003a, 1003b, 1003c, such as flow, temperature, humidity, and/or pressure sensors can be placed in various locations in the respiratory therapy apparatus 1100. Additional sensors (for example, sensors 1020, 1025) may be placed in various locations on the breathing tube 16, respiratory conduit 300, and/or nasal interface 100 (for example, there may be a temperature sensor 1029 at or near the end of the inspiratory tube). Output from the sensors can be received by the controller 1013, to assist the controller in operating the respiratory therapy apparatus 1100 in a manner that provides suitable therapy. In some configurations, providing suitable therapy includes meeting a patient's peak inspiratory demand. The apparatus 1100 may have a transmitter and/or receiver 1015 to enable the controller 1013 to receive signals 1008 from the sensors and/or to control the various components of the respiratory therapy apparatus 1100, including but not limited to the flow generator 1011, humidifier 1012, and heater wire 16, or accessories or peripherals associated with the respiratory therapy apparatus 1100. Additionally, or alternatively, the transmitter and/or receiver 1015 may deliver data to a remote server or enable remote control of the apparatus 1100.

In some configurations, the respiratory therapy system 1000 comprises a gases source 1011 for respiratory gases and configured to provide pressure controlled respiratory gases, a breathing tube 16 to receive the pressure controlled respiratory gases, and a nasal interface.

In some configurations of the respiratory therapy system 1000, the nasal interface 100 includes any one or more of the features outlined herein in fluid communication with the breathing tube 16 to deliver the pressure controlled respiratory gases to a patient.

In some configurations of the respiratory therapy system 1000, the nasal interface 100 has a gases inlet 121 in fluid communication with the breathing tube 16 to deliver the respiratory gases to a patient, the nasal interface comprising a first nasal delivery element 111 and a second nasal delivery element 112, wherein the first nasal delivery element 111 and the second nasal delivery element 112 are each configured to seal with a respective naris of a patient, wherein the nasal interface 100 is configured to create a pressure differential between the first nasal delivery element 111 and the second nasal delivery element 112 when gases are delivered from the gases inlet 121 to both the first nasal delivery element 111 and the second nasal delivery element 112 such that pressure at the first nasal delivery element 111 is higher than pressure at the second nasal delivery element 112.

In some configurations of the respiratory therapy system, the nasal interface 1100 used in the respiratory therapy system comprises an interface body 1110 configured to substantially form a seal with a patient's nasal airways. The interface body 1110 is configured to deliver gases to a first naris of the patient and to a second naris of the patient. The nasal interface 1100 comprises a gases inlet 1121 for delivery of respiratory gases into the nasal interface 1100. The gases inlet 1121 is in fluid communication with the interface body 1110 to deliver the respiratory gases from the gases inlet 1121 through the interface body 1110 to the first naris and second naris of the patient in use. The nasal interface 1100 is configured to receive incoming gases F0 from the gases inlet 1121 and to provide, from the incoming gases F0, a first flow stream of gases F1 configured to be substantially provided to the first naris of the patient in use and a second flow stream of gases F2 configured to be substantially provided to the second naris of the patient in use. The nasal interface 1100 is configured to direct more of the incoming gases to the first flow stream of gases F1 than to the second flow stream of gases F2, to create an asymmetric flow of gases at a patient's nasal airways throughout a respiratory cycle of a patient.

In some configurations of the respiratory therapy system, the nasal interface 1100 used in the respiratory therapy system comprises an interface body 1110 configured to substantially form a seal with a patient's nasal airways, the interface body 1110 configured to deliver gases to a first naris of the patient and to a second naris of the patient. The nasal interface 1100 comprises a gases inlet 1121 for delivery of respiratory gases into the nasal interface, wherein the gases inlet 1121 is in fluid communication with the interface body 1110 to deliver the respiratory gases from the gases inlet 1121 through the interface body 1110 to the first naris and second naris of the patient in use. The nasal interface 1100 is configured to provide a larger dynamic pressure at the first naris of the patient in use and to provide a smaller dynamic pressure at the second naris of the patient in use, to create an asymmetric flow of gases at a patient's nasal airways.

The asymmetric flow of gases at the patient's nasal airways may be created during an inhalation phase of the respiratory cycle. Additionally, this may also occur during an exhalation phase of the respiratory cycle. The inhalation phase and exhalation phase may define a respiratory cycle. As such, an asymmetric flow may be provided at the nasal airways of the patient by the nasal interface 1100 throughout a respiratory cycle of a patient.

In some configurations, the interface body 1100 comprises a first outlet 1111a or first outlet portion configured to deliver gases to the first naris of the patient, and comprises a second outlet 1112a or second outlet portion configured to deliver gases to the second naris of the patient, and wherein the nasal interface 1100 is configured to create a pressure differential between the first outlet 1111a or first outlet portion and the second outlet 1112a or second outlet portion when gases are delivered from the gases inlet 1221 to both the first outlet 1111a or first outlet portion and the second outlet 1112a or second outlet portion such that pressure at the first outlet 1111a or first outlet portion is higher than pressure at the second outlet 1112a or outlet portion.

In some configurations, the respiratory therapy system 1000 comprises a respiratory conduit 300 to receive the pressure controlled respiratory gases from the breathing tube 16, wherein the respiratory conduit 300 is in fluid communication with the breathing tube 16 and the gases inlet 121 of the nasal interface 100, 1100.

In some configurations, the respiratory therapy system 1000 comprises a respiratory gases filter 500.

In some configurations, the respiratory gases filter 500 is located between the heated breathing tube 16 and the respiratory conduit 300. In an additional or alternative configuration, and as shown in Figure 17(b) for example, a respiratory gases filter 500' may be located between the gases manifold 120 and the bias flow restriction 140. For example, in the configuration of Figure 18, the respiratory gases filter 500' may be located between the expiratory gases conduit 160 and the bias flow restriction 140.

In some configurations, the respiratory therapy system comprises a humidifier 1012 configured to humidify said pressure controlled respiratory gases prior to their delivery to the nasal interface 100, 1100.

In some configurations, the breathing tube 16 is a heated breathing tube, and is configured to receive the pressure controlled respiratory gases from the humidifier 1012.

In some configurations, the temperature of gases flow exiting the first and second nasal delivery elements 111, 112, or exiting the nasal interface 1100 for delivery to the patient's nasal airways, may be between about 31°C and about 41°C, optionally more than about 31°C and up to about 41°C, optionally between about 36°C and about 39°C, optionally about 37°C. For example, the temperature may be about 31°C, about 32°C, about 33°C, about 34°C, about 35°C, about 36°C, about 37°C, about 38°C, about 39°C, about 40°C, or about 41°C, or may be any value between any two of those values.

The respiratory therapy system may have any one or more of the features and functionality described in PCT publication no. WO 2021/048744 and U.S. provisional application numbers 62/897,899 and 63/025,151 and/or in PCT publication no. WO 2021/049954 and U.S. provisional application no. 62/898,464.

The patient interface 1 and nasal interface 100 used in the respiratory therapy system 1000 may have any one or more of the features and/or functionality described herein for nasal interface 100 or any of the other nasal interfaces disclosed herein.

In some configurations, the nasal interface 100, patient interface 1, and/or respiratory therapy system 1000 may be used in a method of providing respiratory support to a patient.

In some configurations, the method of providing respiratory support to a patient comprises:
providing a respiratory therapy system 1000 comprising:
a gases source 1011 for respiratory gases and configured to provide pressure controlled respiratory gases;
a breathing tube 16 to receive the pressure controlled respiratory gases; and
a nasal interface 100 having a gases inlet 121 in fluid communication with the breathing tube 16 to deliver the respiratory gases to a patient, the nasal interface 100 comprising a first nasal delivery element 111 and a second nasal delivery element 112;
sealing each of the first nasal delivery element 111 and the second nasal delivery element 112 with a respective naris of a patient;
operating the respiratory therapy apparatus 1000 to provide a flow of gases to the nasal interface 100; and
delivering an asymmetrical flow of gases from the respiratory therapy apparatus 1000 through the first nasal delivery element 111 and the second nasal delivery element 112 at a patient's nares.

In some configurations, the nasal delivery elements 111, 112 are in fluid communication with the gases inlet 121 via a gases flow channel 125, wherein the first nasal delivery element 111 is proximal to the gases inlet 121 and the second nasal delivery element 112 is distal from the gases inlet 121, and wherein the nasal interface comprises a bypass restriction 130 that provides a cross-sectional area A₂ of a portion of the gases flow channel 125, wherein each of the first nasal delivery element 111 and the second nasal delivery element 112 comprises an inner cross-sectional area A₃, A₄, wherein the inner cross-sectional areas together provide a combined cross-sectional area A₃ + A₄ of the nasal delivery elements 111, 112, and wherein the cross-sectional area A₂ of the portion of the gases flow channel 125 is more than 0 to about 1.5 times the combined cross-sectional area A₃ + A₄ of the nasal delivery elements 111, 112.

In some configurations, the cross-sectional area A₂ of the portion of the gases flow channel 125 is up to about 1 times, optionally up to about 2/3 times, the combined cross-sectional area A₃ + A₄ of the nasal delivery elements 111, 112, and wherein the method comprises providing a pressure of 4 cmH2O to the gases inlet 121 such that there is a bias flow through a bias flow restriction 140 of 20 Ipm.

In some configurations, the cross-sectional area A₂ of the portion of the gases flow channel 125 is up to about 1 times, optionally up to about 2/3 times, the combined cross-sectional area A₃ + A₄ of the nasal delivery elements 111, 112, and wherein the method comprises providing a pressure of 8 cmH2O to the gases inlet 121 such that there is a bias flow through a bias flow restriction 140 of 32 Ipm.

In some configurations, the cross-sectional area A₂ of the portion of the gases flow channel 125 is up to about 2/3 times the combined cross-sectional area A₃ + A₄ of the nasal delivery elements 111, 112, and wherein the method comprises providing a pressure of 4 cmH2O to the gases inlet 121 such that there is a bias flow through the bias flow restriction 140 of 20 Ipm, or wherein the method comprises providing a pressure of 8 cmH2O to the gases inlet 121 such that there is a bias flow through the bias flow restriction 140 of 32 Ipm, or wherein the method comprises providing a pressure of 12 cmH2O to the gases inlet 121 such that there is a bias flow through the bias flow restriction 140 of 41 lpm, or wherein the method comprises providing a pressure of 16 cmH2O to the gases inlet 121 such that there is a bias flow through the bias flow restriction 140 of 48 Ipm, or wherein the method comprises providing a pressure of 20 cmH2O to the gases inlet 121 such that there is a bias flow through the bias flow restriction 140 of 53 lpm.

In some configurations, the cross-sectional area A₂ of the portion of the gases flow channel 125 is up to about 2/3 times the combined cross-sectional area A₃ + A₄ of the nasal delivery elements 111, 112, and wherein the method comprises providing a pressure of 8 cmH2O to the gases inlet 121 such that there is a bias flow through the bias flow restriction 140 of 32 lpm or higher.

In some configurations, the cross-sectional area A₂ of the portion of the gases flow channel 125 is up to about 1/3 times the combined cross-sectional area A₃ + A₄ of the nasal delivery elements 111, 112 and wherein the method comprises providing a pressure of 8 cmH2O to the gases inlet 121 such that there is a bias flow through the bias flow restriction 140 of 32 lpm or higher, or wherein the cross-sectional area A₂ of the portion of the gases flow channel 125 is up to about 2/5 times the combined cross-sectional area A₃ + A₄ of the nasal delivery elements 111, 112 and wherein the method comprises providing a pressure of 12 cmH2O to the gases inlet 121 such that there is a bias flow through the bias flow restriction 140 of 41 lpm or higher, or wherein the cross-sectional area A₂ of the portion of the gases flow channel 125 is up to about 2/3 times the combined cross-sectional area A₃ + A₄ of the nasal delivery elements 111, 112 and wherein the method comprises providing a pressure of 16 cmH2O to the gases inlet 121 such that there is a bias flow through the bias flow restriction 140 of 48 lpm or higher.

In some configurations, a temperature of gases flow exiting the first and second nasal delivery elements 111, 112 is between about 31°C and about 41°C, optionally more than 31°C and up to about 41°C, optionally between about 36°C and about 39°C, optionally about 37°C.

In some configurations, the method of providing respiratory support to a patient comprises:
providing a respiratory therapy system 1000 comprising:
a gases source 1011 for respiratory gases and configured to provide pressure controlled respiratory gases;
a breathing tube 16 to receive the pressure controlled respiratory gases; and
a nasal interface 1100 in fluid communication with the breathing tube 16 to deliver the respiratory gases to a patient;
sealing the nasal interface 1100 with a patient's nasal airways;
operating the respiratory therapy apparatus 1000 to provide a flow of gases to the nasal interface 1100; and
receiving incoming gases at a gases inlet 1221 of the nasal interface and creating an asymmetric flow of gases at a patient's nasal airways.

The asymmetric flow of gases at the patient's nasal airways may be created during an inhalation phase of the respiratory cycle. Additionally, this may also occur during an exhalation phase of the respiratory cycle. The inhalation phase and exhalation phase may define a respiratory cycle. As such, an asymmetric flow may be provided at the nasal airways of the patient by the nasal interface 1100 throughout a respiratory cycle of a patient.

The nasal interface may be any one of the nasal interfaces 1100, 1100, 1200, 1300, 1400, 1500, 1600, 1700, 1800, 1900, 2000, 2500, 2700, 2800 disclosed herein.

In some configurations, the system is configured to deliver gases through the nasal interface 100, 1100 with a relative humidity of up to 100%.

In some configurations, the system is configured to deliver gases through the nasal interface 100, 1100 with a relative humidity of between about 14 mg/l and about 34 mg/l.

In some configurations, the temperature of gases flow exiting the first and second nasal delivery elements 111, 112 and/or exiting the nasal interface 1100 for delivery to the nares of the patient, is between about 16°C and about 32°C.

In some configurations, the system is configured to deliver gases through the nasal interface 100, 1100 with an absolute humidity of greater than about 33 mg/l. In some configurations, the system is configured to deliver gases through the nasal interface 100 with an absolute humidity of up to about 44 mg/l.

In some configurations, the system is configured to deliver gases through the nasal interface 100, 1100 with an absolute humidity of up to about 54 mg/l.

In some configurations, the temperature of gases flow exiting the first and second nasal delivery elements 111, 112 and/or or exiting the nasal interface 1100 for delivery to the nares of the patient is up to about 41°C.

The patient interface 1 and nasal interface 100, 1100 used in the method may have any one or more of the features and/or functionality described herein for nasal interfaces 100, 1100, 1200, 1300, 1400, 1500, 1600, 1700, 1800, 1900, 2000, 2500, 2700, 2800.

The respiratory therapy system 1000 used in the method may have nay one or more of the features and/or functionality described herein for the respiratory therapy system 1000.

In use of the patient interface 1 and nasal interface 100, 1100, 1200, 1300, 1400, 1500, 1600, 1700, 1800, 1900, 2000, 2500, 2700, 2800 of the present disclosure for a CPAP-style therapy, compared to a high flow therapy the CPAP-style therapy may provide one or more of: a quieter therapy, increased therapy pressure, easy detection of breath rate, nasal interface removal, and/or leaking (due to control of pressure).

The nasal interfaces 100, 1100, 1200, 1300, 1400, 1500, 1600, 1700, 1800, 1900, 2000, 2500, 2700, 2800 disclosed herein could be used in a medical care facility, home environment, emergency vehicle, or any other suitable environment. Therefore, references herein to "patient" should be interpreted to be any suitable subject that the nasal interfaces are used for or by.

Although the present disclosure has been described in terms of certain embodiments, other embodiments apparent to those of ordinary skill in the art also are within the scope of this disclosure. Thus, various changes and modifications may be made without departing from the scope of the disclosure. For instance, various components may be repositioned as desired. Features from any of the described embodiments may be combined with each other and/or an apparatus may comprise one, more, or all of the features of the above-described embodiments. Moreover, not all of the features, aspects and advantages are necessarily required to practice the present disclosure. Accordingly, the scope of the present disclosure is intended to be defined only by the claims that follow.

## Claims

1. A nasal interface (100, 1100, 1200, 1300, 1400, 1500, 1600, 1700, 1800, 1900, 2000, 2500, 2700, 2800) comprising:
an interface body (110, 1110, 1210, 1310, 1510, 1610, 1910, 2710, 2810), configured to substantially form a seal with a patient's nasal airways, the interface body (110, 1110, 1210, 1310, 1510, 1610, 1910, 2710, 2810) configured to deliver gases to a first naris of the patient and to a second naris of the patient,
and a gases inlet (121, 1121, 1221, 1321, 1421, 1521, 1621, 1721, 1821, 1921, 2721, 2821) for delivery of respiratory gases into the nasal interface (100, 1100, 1200, 1300, 1400, 1500, 1600, 1700, 1800, 1900, 2000, 2500, 2700, 2800), wherein the gases inlet (121, 1121, 1221, 1321, 1421, 1521, 1621, 1721, 1821, 1921, 2721, 2821) is in fluid communication with the interface body (110, 1110, 1210, 1310, 1510, 1610, 1910, 2710, 2810) to deliver the respiratory gases from the gases inlet (121, 1121, 1221, 1321, 1421, 1521, 1621, 1721, 1821, 1921, 2721, 2821) through the interface body (110, 1110, 1210, 1310, 1510, 1610, 1910, 2710, 2810) to the first naris and second naris of the patient in use, and
wherein the nasal interface (100, 1100, 1200, 1300, 1400, 1500, 1600, 1700, 1800, 1900, 2000, 2500, 2700, 2800) is configured to receive incoming gases from the gases inlet (121, 1121, 1221, 1321, 1421, 1521, 1621, 1721, 1821, 1921, 2721, 2821) and to provide, from the incoming gases, a first flow stream of gases (F1) configured to be substantially provided to the first naris of the patient in use and a second flow stream of gases (F2) configured to be substantially provided to the second naris of the patient in use, and is configured to direct more of the incoming gases to the first flow stream of gases (F1) than to the second flow stream of gases (F2), to create an asymmetric flow of gases at a patient's nasal airways throughout a respiratory cycle of a patient, and
wherein the interface body (110, 1110, 1210, 1310, 1510, 1610, 1910, 2710, 2810) comprises a first outlet (111a, 1111a, 1211a, 1411a, 1511a, 1611a, 1711a, 1811a, 2511a, 2711a, 2811a) configured to substantially deliver gases to the first naris of the patient, and comprises a second outlet (112a, 1112a, 1212a, 1412a, 1512a, 1612a, 1712a, 1812a, 2512a, 2712a, 2812a) configured to substantially deliver gases to the second naris of the patient,
wherein the interface body (110, 1110, 1210, 1310, 1510, 1610, 1910, 2710, 2810) comprises a first nasal delivery element (111, 1111, 1211, 1411, 1511, 1611, 1711, 1811, 2711) comprising the first outlet (111a, 1111a, 1211a, 1411a, 1511a, 1611a, 1711a, 1811a, 2511a, 2711a) and a second nasal delivery element (112, 1112, 1212, 1412, 1512, 1612, 1712, 1812, 2712) comprising the second outlet (112a, 1112a, 1212a, 1412a, 1512a, 1612a, 1712a, 1812a, 2512a, 2712a, 2812a),
wherein the nasal interface (100, 1100, 1200, 1300, 1400, 1500, 1600, 1700, 1800, 1900, 2000, 2500, 2700, 2800) is configured such that the first flow stream of gases (F1) is configured to be substantially delivered to the first nasal delivery element (111, 1111, 1211, 1411, 1511, 1611, 1711, 1811, 2711) and the second flow stream of gases (F2) is configured to be substantially delivered to the second nasal delivery element (112, 1112, 1212, 1412, 1512, 1612, 1712, 1812, 2712), and
wherein the first nasal delivery element (111, 1111, 1211, 1411, 1511, 1611, 1711, 1811, 2711) and the second nasal delivery element (112, 1112, 1212, 1412, 1512, 1612, 1712, 1812, 2712) are each configured to seal with a respective naris of a patient,
wherein the nasal interface (100, 1100, 1200, 1300, 1400, 1500, 1600, 1700, 1800, 1900, 2000, 2500, 2700, 2800) further comprises a bias flow restriction (140, 1140, 1240, 2740', 2740", 2840) for a flow of gases out of the nasal interface (100, 1100, 1200, 1300, 1400, 1500, 1600, 1700, 1800, 1900, 2000, 2500, 2700, 2800), and
wherein either:
the nasal interface (100, 1100, 1200, 1300, 1400, 1500, 1600, 1700, 1800, 1900, 2000, 2500, 2700, 2800) comprises a flow director that is configured to direct more of the incoming gases from the gases inlet (121, 1121, 1221, 1321, 1421, 1521, 1621, 1721, 1821, 1921, 2721, 2821) to the first flow stream of gases (F1) than to the second flow stream of gases (F2), or
the nasal interface (100, 1100, 1200, 1300, 1400, 1500, 1600, 1700, 1800, 1900, 2000, 2500, 2700, 2800) comprises a bypass restriction (130) to provide a pressure drop through the nasal interface (100, 1100, 1200, 1300, 1400, 1500, 1600, 1700, 1800, 1900, 2000, 2500, 2700, 2800) between the first nasal delivery element (111, 1111, 1211, 1411, 1511, 1611, 1711, 1811, 2711) and the second nasal delivery element (112, 1112, 1212, 1412, 1512, 1612, 1712, 1812, 2712) when gases are delivered from the gases inlet (121, 1121, 1221, 1321, 1421, 1521, 1621, 1721, 1821, 1921, 2721, 2821) to the first nasal delivery element (111, 1111, 1211, 1411, 1511, 1611, 1711, 1811, 2711) and the second nasal delivery element (112, 1112, 1212, 1412, 1512, 1612, 1712, 1812, 2712) such that pressure at the first nasal delivery element (111, 1111, 1211, 1411, 1511, 1611, 1711, 1811, 2711) is higher than pressure at the second nasal delivery element (112, 1112, 1212, 1412, 1512, 1612, 1712, 1812, 2712).

2. The nasal interface (100, 1100, 1200, 1300, 1400, 1500, 1600, 1700, 1800, 1900, 2000, 2500, 2700, 2800) according to claim 1, wherein the gases inlet (121, 1121, 1221, 1321, 1421, 1521, 1621, 1721, 1821, 1921, 2721, 2821) is at least partly aligned with the first outlet (111a, 1111a, 1211a, 1411a, 1511a, 1611a, 1711a, 1811a, 2511a, 2711a) and is less aligned or is not aligned with the second outlet (112a, 1112a, 1212a, 1412a, 1512a, 1612a, 1712a, 1812a, 2512a, 2712a, 2812a).

3. The nasal interface (100, 1100, 1200, 1300, 1400, 1500, 1600, 1700, 1800, 1900, 2000, 2500, 2700, 2800) according to claim 2, wherein the gases inlet (121, 1121, 1221, 1321, 1421, 1521, 1621, 1721, 1821, 1921, 2721, 2821) is substantially axially aligned with the first outlet (111a, 1111a, 1211a, 1411a, 1511a, 1611a, 1711a, 1811a, 2511a, 2711a).

4. The nasal interface (100, 1100, 1200, 1300, 1400, 1500, 1600, 1700, 1800, 1900, 2000, 2500, 2700, 2800) according to claim 3, wherein at least half of a transverse cross-sectional area of the gases inlet (121, 1121, 1221, 1321, 1421, 1521, 1621, 1721, 1821, 1921, 2721, 2821) is axially aligned with at least half of a transverse cross-sectional area (A₃) of the first outlet (111a, 1111a, 1211a, 1411a, 1511a, 1611a, 1711a, 1811a, 2511a, 2711a).

5. The nasal interface (100, 1100, 1200, 1300, 1400, 1500, 1600, 1700, 1800, 1900, 2000, 2500, 2700, 2800) according to claim 1, wherein the gases inlet (121, 1121, 1221, 1321, 1421, 1521, 1621, 1721, 1821, 1921, 2721, 2821) is angled toward the first outlet (111a, 1111a, 1211a, 1411a, 1511a, 1611a, 1711a, 1811a, 2511a, 2711a).

6. The nasal interface (100, 1100, 1200, 1300, 1400, 1500, 1600, 1700, 1800, 1900, 2000, 2500, 2700, 2800) according to any one of claims 1 to 5, wherein the first flow stream of gases (F1) has at least one dimension (D1) that is larger than a corresponding dimension (D2) of the second flow stream of gases (F2).

7. The nasal interface (100, 1100, 1200, 1300, 1400, 1500, 1600, 1700, 1800, 1900, 2000, 2500, 2700, 2800) according to claim 6, wherein the at least one dimension (D1) comprises a lateral dimension of the first flow stream of gases (F1), and wherein the corresponding dimension (D2) comprises a lateral dimension of the second flow stream of gases (F2).

8. The nasal interface (100, 1100, 1200, 1300, 1400, 1500, 1600, 1700, 1800, 1900, 2000, 2500, 2700, 2800) according to claim 6 or 7, wherein the first flow stream of gases (F1) has a larger diameter, cross-sectional area, and/or volume than a corresponding diameter, cross-sectional area, and/or volume of the second flow stream of gases (F2).

9. The nasal interface (100, 1100, 1200, 1300, 1400, 1500, 1600, 1700, 1800, 1900, 2000, 2500, 2700, 2800) according to any one of claims 1 to 8, wherein the nasal interface (100, 1100, 1100, 1200, 1300, 1400, 1500, 1600, 1700, 1800, 1900, 2000, 2500, 2700, 2800) is configured to deliver a lower velocity of gases flow through the first outlet (111a, 1111a, 1211a, 1411a, 1511a, 1611a, 1711a, 1811a, 2511a, 2711a) than a velocity of gases flow through the second outlet (112a, 1112a, 1212a, 1412a, 1512a, 1612a, 1712a, 1812a, 2512a, 2712a, 2812a) during an inhalation phase of a respiratory cycle.

10. The nasal interface (100, 1100, 1200, 1300, 1400, 1500, 1600, 1700, 1800, 1900, 2000, 2500, 2700, 2800) according to any one of claims 1 to 9, wherein the nasal interface (100, 1100, 1200, 1300, 1400, 1500, 1600, 1700, 1800, 1900, 2000, 2500, 2700, 2800) is configured to deliver a higher pressure of gases flow through the first outlet (111a, 1111a, 1211a, 1411a, 1511a, 1611a, 1711a, 1811a, 2511a, 2711a) than a pressure of gases flow through the second outlet (112a, 1112a, 1212a, 1412a, 1512a, 1612a, 1712a, 1812a, 2512a, 2712a, 2812a) during an inhalation phase of a respiratory cycle.

11. The nasal interface (100, 1100, 1200, 1300, 1400, 1500, 1600, 1700, 1800, 1900, 2000, 2500, 2700, 2800) according to any one of claims 1 to 10, further comprising a connector or elbow (1222, 2722) for connecting a respiratory conduit (300) to the nasal interface (100, 1100, 1200, 1300, 1400, 1500, 1600, 1700, 1800, 1900, 2000, 2500, 2700, 2800).

12. The nasal interface (100, 1100, 1200, 1300, 1400, 1500, 1600, 1700, 1800, 1900, 2000, 2500, 2700, 2800) according to any one of claims 1 to 11, wherein the nasal interface (100, 1100, 1200, 1300, 1400, 1500, 1600, 1700, 1800, 1900, 2000, 2500, 2700, 2800) is configured to direct more of the incoming gases to the first flow stream of gases (F1) than to the second flow stream of gases (F2) during an inhalation phase of the respiratory cycle, and/or
wherein the interface body (110, 1110, 1210, 1310, 1510, 1610, 1910, 2710, 2810) is a nasal cushion (1110, 1210, 1910, 2110, 2510, 2610, 2710) and/or
wherein the nasal interface (100, 1100, 1200, 1300, 1400, 1500, 1600, 1700, 1800, 1900, 2000, 2500, 2700, 2800) is configured to simultaneously deliver the respiratory gases from the gases inlet (121, 1121, 1221, 1321, 1421, 1521, 1621, 1721, 1821, 1921, 2721, 2821) through the interface body (110, 1110, 1210, 1310, 1510, 1610, 1910, 2710, 2810) to both the first naris and second naris of the patient in use.

13. The nasal interface of according to any one of claims 1 to 12, wherein the gases inlet (121, 1121, 1221, 1321, 1421, 1521, 1621, 1721, 1821, 1921, 2721, 2821) is positioned at a side of a gases manifold (120, 1120, 1220, 1320, 1520, 1620, 1820, 2220, 2320, 2420, 2720, 2820) of the nasal interface (100, 1100, 1200, 1300, 1400, 1500, 1600, 1700, 1800, 1900, 2000, 2500, 2700, 2800), and the nasal interface (100, 1100, 1200, 1300, 1400, 1500, 1600, 1700, 1800, 1900, 2000, 2500, 2700, 2800) comprises a respiratory conduit (300) connected to the gases inlet (121, 1121, 1221, 1321, 1421, 1521, 1621, 1721, 1821, 1921, 2721, 2821) and the respiratory conduit (300) extending laterally from the side of the gases manifold (120, 1120, 1220, 1320, 1520, 1620, 1820, 2220, 2320, 2420, 2720, 2820).

14. The nasal interface (100, 1100, 1200, 1300, 1400, 1500, 1600, 1700, 1800, 1900, 2000, 2500, 2700, 2800) according to any one of claims 1 to 13, wherein the gases manifold (120) comprises side ports (121, 122) on both left and right sides to selectively receive the respiratory conduit (300).

## Patentansprüche

1. Nasenschnittstelle (100, 1100, 1200, 1300, 1400, 1500, 1600, 1700, 1800, 1900, 2000, 2500, 2700, 2800), umfassend:
einen Schnittstellenkörper (110, 1110, 1210, 1310, 1510, 1610, 1910, 2710, 2810), der dazu ausgelegt ist, im Wesentlichen eine Abdichtung mit den Nasenatmungswegen eines Patienten zu bilden, wobei der Schnittstellenkörper (110, 1110, 1210, 1310, 1510, 1610, 1910, 2710, 2810) dazu ausgelegt ist, Gase an ein erstes Nasenloch des Patienten und an ein zweites Nasenloch des Patienten zu leiten,
und einen Gaseinlass (121, 1121, 1221, 1321, 1421, 1521, 1621, 1721, 1821, 1921, 2721, 2821) zur Abgabe von Atemgasen in die Nasenschnittstelle (100, 1100, 1200, 1300, 1400, 1500, 1600, 1700, 1800, 1900, 2000, 2500, 2700, 2800), wobei der Gaseinlass (121, 1121, 1221, 1321, 1421, 1521, 1621, 1721, 1821, 1921, 2721, 2821) in Fluidverbindung mit dem Schnittstellenkörper (110, 1110, 1210, 1310, 1510, 1610, 1910, 2710, 2810) steht, um die Atemgase von dem Gaseinlass (121, 1121, 1221, 1321, 1421, 1521, 1621, 1721, 1821, 1921, 2721, 2821) durch den Schnittstellenkörper (110, 1110, 1210, 1310, 1510, 1610, 1910, 2710, 2810) an das erste Nasenloch und das zweite Nasenloch des Patienten bei Anwendung zu leiten, und
wobei die Nasenschnittstelle (100, 1100, 1200, 1300, 1400, 1500, 1600, 1700, 1800, 1900, 2000, 2500, 2700, 2800) dazu ausgelegt ist, einströmende Gase von dem Gaseinlass (121, 1121, 1221, 1321, 1421, 1521, 1621, 1721, 1821, 1921, 2721, 2821) aufzunehmen und aus den einströmenden Gasen einen ersten Gasstrom (F1) bereitzustellen, der dazu ausgelegt ist, im Einsatz im Wesentlichen dem ersten Nasenloch des Patienten zugeführt zu werden, und einen zweiten Gasstrom (F2) bereitzustellen, der dazu ausgelegt ist, im Einsatz im Wesentlichen dem zweiten Nasenloch des Patienten zugeführt zu werden, und dazu ausgelegt ist, mehr von den einströmenden Gasen zu dem ersten Gasstrom (F1) als zu dem zweiten Gasstrom (F2) zu leiten, um einen asymmetrischen Gasstrom an den nasalen Atemwegen eines Patienten während eines gesamten Atemzyklus eines Patienten zu erzeugen, und
wobei der Schnittstellenkörper (110, 1110, 1210, 1310, 1510, 1610, 1910, 2710, 2810) einen ersten Auslass (111a, 1111a, 1211a, 1411a, 1511a, 1611a, 1711a, 1811a, 2511a, 2711a, 2811a) umfasst, der dazu ausgelegt ist, im Wesentlichen Gase an das erste Nasenloch des Patienten zu leiten, und einen zweiten Auslass (112a, 1112a, 1212a, 1412a, 1512a, 1612a, 1712a, 1812a, 2512a, 2712a, 2812a) umfasst, der dazu ausgelegt ist, im Wesentlichen Gase an das zweite Nasenloch des Patienten zu leiten,
wobei der Schnittstellenkörper (110, 1110, 1210, 1310, 1510, 1610, 1910, 2710, 2810) ein erstes nasales Abgabeelement (111, 1111, 1211, 1411, 1511, 1611, 1711, 1811, 2711), umfassend den ersten Auslass (111a, 1111a, 1211a, 1411a, 1511a, 1611a, 1711a, 1811a, 2511a, 2711a), und ein zweites nasales Abgabeelement (112, 1112, 1212, 1412, 1512, 1612, 1712, 1812, 2712), umfassend den zweiten Auslass (112a, 1112a, 1212a, 1412a, 1512a, 1612a, 1712a, 1812a, 2512a, 2712a, 2812a), umfasst,
wobei die Nasenschnittstelle (100, 1100, 1200, 1300, 1400, 1500, 1600, 1700, 1800, 1900, 2000, 2500, 2700, 2800) so ausgelegt ist, dass der erste Gasstrom (F1) dazu ausgelegt ist, im Wesentlichen an das erste nasale Abgabeelement (111, 1111, 1211, 1411, 1511, 1611, 1711, 1811, 2711) geleitet zu werden, und der zweite Gasstrom (F2) dazu ausgelegt ist, im Wesentlichen an das zweite nasale Abgabeelement (112, 1112, 1212, 1412, 1512, 1612, 1712, 1812, 2712) geleitet zu werden, und
wobei das erste nasale Abgabeelement (111, 1111, 1211, 1411, 1511, 1611, 1711, 1811, 2711) und das zweite nasale Abgabeelement (112, 1112, 1212, 1412, 1512, 1612, 1712, 1812, 2712) jeweils so ausgelegt sind, dass sie dicht an einem jeweiligen Nasenloch eines Patienten anliegen,
wobei die Nasenschnittstelle (100, 1100, 1200, 1300, 1400, 1500, 1600, 1700, 1800, 1900, 2000, 2500, 2700, 2800) ferner eine Vorspannungs-Durchflussbegrenzung (140, 1140, 1240, 2740', 2740", 2840) für einen Strom von Gasen aus der Nasenschnittstelle (100, 1100, 1200, 1300, 1400, 1500, 1600, 1700, 1800, 1900, 2000, 2500, 2700, 2800) heraus umfasst, und
wobei entweder:
die Nasenschnittstelle (100, 1100, 1200, 1300, 1400, 1500, 1600, 1700, 1800, 1900, 2000, 2500, 2700, 2800) ein Strömungsleitelement umfasst, das dazu ausgelegt ist, mehr der einströmenden Gase aus dem Gaseinlass (121, 1121, 1221, 1321, 1421, 1521, 1621, 1721, 1821, 1921, 2721, 2821) in den ersten Gasstrom (F1) zu leiten als in den zweiten Gasstrom (F2), oder
die Nasenschnittstelle (100, 1100, 1200, 1300, 1400, 1500, 1600, 1700, 1800, 1900, 2000, 2500, 2700, 2800) eine Bypass-Verengung (130) umfasst, um einen Druckabfall durch die Nasenschnittstelle (100, 1100, 1200, 1300, 1400, 1500, 1600, 1700, 1800, 1900, 2000, 2500, 2700, 2800) zwischen dem ersten nasalen Abgabeelement (111, 1111, 1211, 1411, 1511, 1611, 1711, 1811, 2711) und dem zweiten nasalen Abgabeelement (112, 1112, 1212, 1412, 1512, 1612, 1712, 1812, 2712) bereitzustellen, wenn Gase von dem Gaseinlass (121, 1121, 1221, 1321, 1421, 1521, 1621, 1721, 1821, 1921, 2721, 2821) zu dem ersten nasalen Abgabeelement (111, 1111, 1211, 1411, 1511, 1611, 1711, 1811, 2711) und dem zweiten nasalen Abgabeelement (112, 1112, 1212, 1412, 1512, 1612, 1712, 1812, 2712) derart zugeführt werden, dass der Druck an dem ersten nasalen Abgabeelement (111, 1111, 1211, 1411, 1511, 1611, 1711, 1811, 2711) höher ist als der Druck an dem zweiten nasalen Abgabeelement (112, 1112, 1212, 1412, 1512, 1612, 1712, 1812, 2712).

2. Nasenschnittstelle (100, 1100, 1200, 1300, 1400, 1500, 1600, 1700, 1800, 1900, 2000, 2500, 2700, 2800) nach Anspruch 1, wobei der Gaseinlass (121, 1121, 1221, 1321, 1421, 1521, 1621, 1721, 1821, 1921, 2721, 2821) mindestens teilweise auf den ersten Auslass (111a, 1111a, 1211a, 1411a, 1511a, 1611a, 1711a, 1811a, 2511a, 2711a) ausgerichtet ist und weniger oder gar nicht auf den zweiten Auslass (112a, 1112a, 1212a, 1412a, 1512a, 1612a, 1712a, 1812a, 2512a, 2712a, 2812a) ausgerichtet ist.

3. Nasenschnittstelle (100, 1100, 1200, 1300, 1400, 1500, 1600, 1700, 1800, 1900, 2000, 2500, 2700, 2800) nach Anspruch 2, wobei der Gaseinlass (121, 1121, 1221, 1321, 1421, 1521, 1621, 1721, 1821, 1921, 2721, 2821) im Wesentlichen axial auf den ersten Auslass (111a, 1111a, 1211a, 1411a, 1511a, 1611a, 1711a, 1811a, 2511a, 2711a) ausgerichtet ist.

4. Nasenschnittstelle (100, 1100, 1200, 1300, 1400, 1500, 1600, 1700, 1800, 1900, 2000, 2500, 2700, 2800) nach Anspruch 3, wobei mindestens die Hälfte einer transversalen Querschnittsfläche des Gaseinlasses (121, 1121, 1221, 1321, 1421, 1521, 1621, 1721, 1821, 1921, 2721, 2821) axial auf mindestens die Hälfte einer transversalen Querschnittsfläche (A₃) des ersten Auslasses (111a, 1111a, 1211a, 1411a, 1511a, 1611a, 1711a, 1811a, 2511a, 2711a) ausgerichtet ist.

5. Nasenschnittstelle (100, 1100, 1200, 1300, 1400, 1500, 1600, 1700, 1800, 1900, 2000, 2500, 2700, 2800) nach Anspruch 1, wobei der Gaseinlass (121, 1121, 1221, 1321, 1421, 1521, 1621, 1721, 1821, 1921, 2721, 2821) schräg in Richtung des ersten Auslasses (111a, 1111a, 1211a, 1411a, 1511a, 1611a, 1711a, 1811a, 2511a, 2711a) verläuft.

6. Nasenschnittstelle (100, 1100, 1200, 1300, 1400, 1500, 1600, 1700, 1800, 1900, 2000, 2500, 2700, 2800) nach einem der Ansprüche 1 bis 5, wobei der erste Gasstrom (F1) mindestens eine Abmessung (D1) aufweist, die größer als eine entsprechende Abmessung (D2) des zweiten Gasstroms (F2) ist.

7. Nasenschnittstelle (100, 1100, 1200, 1300, 1400, 1500, 1600, 1700, 1800, 1900, 2000, 2500, 2700, 2800) nach Anspruch 6, wobei die mindestens eine Abmessung (D1) eine laterale Abmessung des ersten Gasstroms (F1) umfasst, und wobei die entsprechende Abmessung (D2) eine laterale Abmessung des zweiten Gasstroms (F2) umfasst.

8. Nasenschnittstelle (100, 1100, 1200, 1300, 1400, 1500, 1600, 1700, 1800, 1900, 2000, 2500, 2700, 2800) nach Anspruch 6 oder 7, wobei der erste Gasstrom (F1) einen größeren Durchmesser, eine größere Querschnittsfläche und/oder ein größeres Volumen aufweist als ein entsprechender Durchmesser, eine entsprechende Querschnittsfläche und/oder ein entsprechendes Volumen des zweiten Gasstroms (F2).

9. Nasenschnittstelle (100, 1100, 1200, 1300, 1400, 1500, 1600, 1700, 1800, 1900, 2000, 2500, 2700, 2800) nach einem der Ansprüche 1 bis 8, wobei die Nasenschnittstelle (100, 1100, 1100, 1200, 1300, 1400, 1500, 1600, 1700, 1800, 1900, 2000, 2500, 2700, 2800) dazu ausgelegt ist, während einer Einatmungsphase eines Atemzyklus eine niedrigere Geschwindigkeit des Gasstroms durch den ersten Auslass (111a, 1111a, 1211a, 1411a, 1511a, 1611a, 1711a, 1811a, 2511a, 2711a) als die Geschwindigkeit des Gasstroms durch den zweiten Auslass (112a, 1112a, 1212a, 1412a, 1512a, 1612a, 1712a, 1812a, 2512a, 2712a, 2812a) zu erzeugen.

10. Nasenschnittstelle (100, 1100, 1200, 1300, 1400, 1500, 1600, 1700, 1800, 1900, 2000, 2500, 2700, 2800) nach einem der Ansprüche 1 bis 9, wobei die Nasenschnittstelle (100, 1100, 1200, 1300, 1400, 1500, 1600, 1700, 1800, 1900, 2000, 2500, 2700, 2800) dazu ausgelegt ist, während einer Einatmungsphase eines Atemzyklus einen höheren Druck des Gasstroms durch den ersten Auslass (111a, 1111a, 1211a, 1411a, 1511a, 1611a, 1711a, 1811a, 2511a, 2711a) als den Druck des Gasstroms durch den zweiten Auslass (112a, 1112a, 1212a, 1412a, 1512a, 1612a, 1712a, 1812a, 2512a, 2712a, 2812a) zu erzeugen.

11. Nasenschnittstelle (100, 1100, 1200, 1300, 1400, 1500, 1600, 1700, 1800, 1900, 2000, 2500, 2700, 2800) nach einem der Ansprüche 1 bis 10, ferner umfassend ein Verbindungsstück oder ein Kniestück (1222, 2722) zum Verbinden einer Atemleitung (300) mit der Nasenschnittstelle (100, 1100, 1200, 1300, 1400, 1500, 1600, 1700, 1800, 1900, 2000, 2500, 2700, 2800).

12. Nasenschnittstelle (100, 1100, 1200, 1300, 1400, 1500, 1600, 1700, 1800, 1900, 2000, 2500, 2700, 2800) nach einem der Ansprüche 1 bis 11, wobei die Nasenschnittstelle (100, 1100, 1200, 1300, 1400, 1500, 1600, 1700, 1800, 1900, 2000, 2500, 2700, 2800) dazu ausgelegt ist, während einer Einatmungsphase des Atemzyklus mehr von den einströmenden Gasen zu dem ersten Gasstrom (F1) als zu dem zweiten Gasstrom (F2) zu leiten, und/oder wobei der Schnittstellenkörper (110, 1110, 1210, 1310, 1510, 1610, 1910, 2710, 2810) ein Nasenpolster (1110, 1210, 1910, 2110, 2510, 2610, 2710) ist und/oder wobei die Nasenschnittstelle (100, 1100, 1200, 1300, 1400, 1500, 1600, 1700, 1800, 1900, 2000, 2500, 2700, 2800) dazu ausgelegt ist, die Atemgase gleichzeitig aus dem Gaseinlass (121, 1121, 1221, 1321, 1421, 1521, 1621, 1721, 1821, 1921, 2721, 2821) durch den Schnittstellenkörper (110, 1110, 1210, 1310, 1510, 1610, 1910, 2710, 2810) sowohl an das erste Nasenloch als auch an das zweite Nasenloch des Patienten während der Anwendung zu leiten.

13. Nasenschnittstelle nach einem der Ansprüche 1 bis 12, wobei der Gaseinlass (121, 1121, 1221, 1321, 1421, 1521, 1621, 1721, 1821, 1921, 2721, 2821) an einer Seite eines Gasverteilers (120, 1120, 1220, 1320, 1520, 1620, 1820, 2220, 2320, 2420, 2720, 2820) der Nasenschnittstelle (100, 1100, 1200, 1300, 1400, 1500, 1600, 1700, 1800, 1900, 2000, 2500, 2700, 2800) angeordnet ist, und die Nasenschnittstelle (100, 1100, 1200, 1300, 1400, 1500, 1600, 1700, 1800, 1900, 2000, 2500, 2700, 2800) eine Atemleitung (300) umfasst, die mit dem Gaseinlass (121, 1121, 1221, 1321, 1421, 1521, 1621, 1721, 1821, 1921, 2721, 2821) verbunden ist, und sich die Atemleitung (300) lateral von der Seite des Gasverteilers (120, 1120, 1220, 1320, 1520, 1620, 1820, 2220, 2320, 2420, 2720, 2820) erstreckt.

14. Nasenschnittstelle (100, 1100, 1200, 1300, 1400, 1500, 1600, 1700, 1800, 1900, 2000, 2500, 2700, 2800) nach einem der Ansprüche 1 bis 13, wobei der Gasverteiler (120) Seitenanschlüsse (121, 122) sowohl auf der linken als auch auf der rechten Seite umfasst, um die Atemleitung (300) selektiv aufzunehmen.

## Revendications

1. Interface nasale (100, 1100, 1200, 1300, 1400, 1500, 1600, 1700, 1800, 1900, 2000, 2500, 2700, 2800) comprenant :
un corps d'interface (110, 1110, 1210, 1310, 1510, 1610, 1910, 2710, 2810), conçu pour former sensiblement un joint d'étanchéité avec les voies respiratoires nasales d'un patient, le corps d'interface (110, 1110, 1210, 1310, 1510, 1610, 1910, 2710, 2810) étant conçu pour administrer des gaz à une première narine du patient et à une seconde narine du patient,
et une entrée de gaz (121, 1121, 1221, 1321, 1421, 1521, 1621, 1721, 1821, 1921, 2721, 2821) pour l'administration de gaz respiratoires dans l'interface nasale (100, 1100, 1200, 1300, 1400, 1500, 1600, 1700, 1800, 1900, 2000, 2500, 2700, 2800), dans laquelle l'entrée de gaz (121, 1121, 1221, 1321, 1421, 1521, 1621, 1721, 1821, 1921, 2721, 2821) est en communication fluidique avec le corps d'interface (110, 1110, 1210, 1310, 1510, 1610, 1910, 2710, 2810) pour administrer les gaz respiratoires de l'entrée de gaz (121, 1121, 1221, 1321, 1421, 1521, 1621, 1721, 1821, 1921, 2721, 2821) à travers le corps d'interface (110, 1110, 1210, 1310, 1510, 1610, 1910, 2710, 2810) à la première narine et à la seconde narine du patient lors de l'utilisation, et
dans laquelle l'interface nasale (100, 1100, 1200, 1300, 1400, 1500, 1600, 1700, 1800, 1900, 2000, 2500, 2700, 2800) est conçue pour recevoir des gaz entrants à partir de l'entrée de gaz (121, 1121, 1221, 1321, 1421, 1521, 1621, 1721, 1821, 1921, 2721, 2821) et pour fournir, à partir des gaz entrants, un premier flux d'écoulement de gaz (F1) conçu pour être sensiblement fourni à la première narine du patient lors de l'utilisation et un second flux d'écoulement de gaz (F2) conçu pour être sensiblement fourni à la seconde narine du patient lors de l'utilisation, et est conçue pour diriger plus de gaz entrants vers le premier flux d'écoulement de gaz (F1) que vers le second flux d'écoulement de gaz (F2), afin de créer un écoulement asymétrique de gaz au niveau des voies respiratoires nasales d'un patient tout au long d'un cycle respiratoire d'un patient, et
dans laquelle le corps d'interface (110, 1110, 1210, 1310, 1510, 1610, 1910, 2710, 2810) comprend une première sortie (111a, 1111a, 1211a, 1411a, 1511a, 1611a, 1711a, 1811a, 2511a, 2711a, 2811a) conçue pour administrer sensiblement des gaz à la première narine du patient, et comprend une seconde sortie (112a, 1112a, 1212a, 1412a, 1512a, 1612a, 1712a, 1812a, 2512a, 2712a, 2812a) conçue pour administrer sensiblement des gaz à la seconde narine du patient,
dans laquelle le corps d'interface (110, 1110, 1210, 1310, 1510, 1610, 1910, 2710, 2810) comprend un premier élément d'administration nasale (111, 1111, 1211, 1411, 1511, 1611, 1711, 1811, 2711) comprenant la première sortie (111a, 1111a, 1211a, 1411a, 1511a, 1611a, 1711a, 1811a, 2511a, 2711a) et un second élément d'administration nasale (112, 1112, 1212, 1412, 1512, 1612, 1712, 1812, 2712) comprenant la seconde sortie (112a, 1112a, 1212a, 1412a, 1512a, 1612a, 1712a, 1812a, 2512a, 2712a, 2812a),
dans laquelle l'interface nasale (100, 1100, 1200, 1300, 1400, 1500, 1600, 1700, 1800, 1900, 2000, 2500, 2700, 2800) est conçue de telle sorte que le premier flux d'écoulement de gaz (F1) est conçu pour être sensiblement administré au premier élément d'administration nasale (111, 1111, 1211, 1411, 1511, 1611, 1711, 1811, 2711) et le second flux d'écoulement de gaz (F2) est conçu pour être sensiblement administré au second élément d'administration nasale (112, 1112, 1212, 1412, 1512, 1612, 1712, 1812, 2712), et
dans laquelle le premier élément d'administration nasale (111, 1111, 1211, 1411, 1511, 1611, 1711, 1811, 2711) et le second élément d'administration nasale (112, 1112, 1212, 1412, 1512, 1612, 1712, 1812, 2712) sont chacun conçus pour assurer l'étanchéité avec une narine respective d'un patient,
dans laquelle l'interface nasale (100, 1100, 1200, 1300, 1400, 1500, 1600, 1700, 1800, 1900, 2000, 2500, 2700, 2800) comprend en outre une restriction d'écoulement de base (140, 1140, 1240, 2740', 2740", 2840) pour un écoulement de gaz hors de l'interface nasale (100, 1100, 1200, 1300, 1400, 1500, 1600, 1700, 1800, 1900, 2000, 2500, 2700, 2800), et
dans laquelle soit :
l'interface nasale (100, 1100, 1200, 1300, 1400, 1500, 1600, 1700, 1800, 1900, 2000, 2500, 2700, 2800) comprend un directeur d'écoulement qui est configuré pour diriger plus de gaz entrants de l'entrée de gaz (121, 1121, 1221, 1321, 1421, 1521, 1621, 1721, 1821, 1921, 2721, 2821) vers le premier flux d'écoulement de gaz (F1) que vers le second flux d'écoulement de gaz (F2), soit
l'interface nasale (100, 1100, 1200, 1300, 1400, 1500, 1600, 1700, 1800, 1900, 2000, 2500, 2700, 2800) comprend une restriction de dérivation (130) pour fournir une chute de pression à travers l'interface nasale (100, 1100, 1200, 1300, 1400, 1500, 1600, 1700, 1800, 1900, 2000, 2500, 2700, 2800) entre le premier élément d'administration nasale (111, 1111, 1211, 1411, 1511, 1611, 1711, 1811, 2711) et le second élément d'administration nasale (112, 1112, 1212, 1412, 1512, 1612, 1712, 1812, 2712) lorsque des gaz sont administrés de l'entrée de gaz (121, 1121, 1221, 1321, 1421, 1521, 1621, 1721, 1821, 1921, 2721, 2821) au premier élément d'administration nasale (111, 1111, 1211, 1411, 1511, 1611, 1711, 1811, 2711) et au second élément d'administration nasale (112, 1112, 1212, 1412, 1512, 1612, 1712, 1812, 2712) de telle sorte que la pression au niveau du premier élément d'administration nasale (111, 1111, 1211, 1411, 1511, 1611, 1711, 1811, 2711) est supérieure à la pression au niveau du second élément d'administration nasale (112, 1112, 1212, 1412, 1512, 1612, 1712, 1812, 2712).

2. Interface nasale (100, 1100, 1200, 1300, 1400, 1500, 1600, 1700, 1800, 1900, 2000, 2500, 2700, 2800) selon la revendication 1, dans laquelle l'entrée de gaz (121, 1121, 1221, 1321, 1421, 1521, 1621, 1721, 1821, 1921, 2721, 2821) est au moins partiellement alignée sur la première sortie (111a, 1111a, 1211a, 1411a, 1511a, 1611a, 1711a, 1811a, 2511a, 2711a) et est moins alignée ou n'est pas alignée sur la seconde sortie (112a, 1112a, 1212a, 1412a, 1512a, 1612a, 1712a, 1812a, 2512a, 2712a, 2812a).

3. Interface nasale (100, 1100, 1200, 1300, 1400, 1500, 1600, 1700, 1800, 1900, 2000, 2500, 2700, 2800) selon la revendication 2, dans laquelle l'entrée de gaz (121, 1121, 1221, 1321, 1421, 1521, 1621, 1721, 1821, 1921, 2721, 2821) est sensiblement alignée axialement sur la première sortie (111a, 1111a, 1211a, 1411a, 1511a, 1611a, 1711a, 1811a, 2511a, 2711a).

4. Interface nasale (100, 1100, 1200, 1300, 1400, 1500, 1600, 1700, 1800, 1900, 2000, 2500, 2700, 2800) selon la revendication 3, dans laquelle au moins la moitié d'une surface de section transversale de l'entrée de gaz (121, 1121, 1221, 1321, 1421, 1521, 1621, 1721, 1821, 1921, 2721, 2821) est alignée axialement sur au moins la moitié d'une surface de section transversale (A₃) de la première sortie (111a, 1111a, 1211a, 1411a, 1511a, 1611a, 1711a, 1811a, 2511a, 2711a).

5. Interface nasale (100, 1100, 1200, 1300, 1400, 1500, 1600, 1700, 1800, 1900, 2000, 2500, 2700, 2800) selon la revendication 1, dans laquelle l'entrée de gaz (121, 1121, 1221, 1321, 1421, 1521, 1621, 1721, 1821, 1921, 2721, 2821) est inclinée vers la première sortie (111a, 1111a, 1211a, 1411a, 1511a, 1611a, 1711a, 1811a, 2511a, 2711a).

6. Interface nasale (100, 1100, 1200, 1300, 1400, 1500, 1600, 1700, 1800, 1900, 2000, 2500, 2700, 2800) selon l'une quelconque des revendications 1 à 5, dans laquelle le premier flux d'écoulement de gaz (F1) a au moins une dimension (D1) qui est supérieure à une dimension correspondante (D2) du second flux d'écoulement de gaz (F2).

7. Interface nasale (100, 1100, 1200, 1300, 1400, 1500, 1600, 1700, 1800, 1900, 2000, 2500, 2700, 2800) selon la revendication 6, dans laquelle l'au moins une dimension (D1) comprend une dimension latérale du premier flux d'écoulement de gaz (F1), et dans laquelle la dimension correspondante (D2) comprend une dimension latérale du second flux d'écoulement de gaz (F2).

8. Interface nasale (100, 1100, 1200, 1300, 1400, 1500, 1600, 1700, 1800, 1900, 2000, 2500, 2700, 2800) selon la revendication 6 ou 7, dans laquelle le premier flux d'écoulement de gaz (F1) a un diamètre, une surface de section transversale et/ou un volume supérieurs à un diamètre, une surface transversale et/ou un volume correspondants du second flux d'écoulement de gaz (F2).

9. Interface nasale (100, 1100, 1200, 1300, 1400, 1500, 1600, 1700, 1800, 1900, 2000, 2500, 2700, 2800) selon l'une quelconque des revendications 1 à 8, dans laquelle l'interface nasale (100, 1100, 1100, 1200, 1300, 1400, 1500, 1600, 1700, 1800, 1900, 2000, 2500, 2700, 2800) est conçue pour administrer une vitesse d'écoulement de gaz à travers la première sortie (111a, 1111a, 1211a, 1411a, 1511a, 1611a, 1711a, 1811a, 2511a, 2711a) inférieure à une vitesse d'écoulement de gaz à travers la seconde sortie (112a, 1112a, 1212a, 1412a, 1512a, 1612a, 1712a, 1812a, 2512a, 2712a, 2812a) pendant une phase d'inhalation d'un cycle respiratoire.

10. Interface nasale (100, 1100, 1200, 1300, 1400, 1500, 1600, 1700, 1800, 1900, 2000, 2500, 2700, 2800) selon l'une quelconque des revendications 1 à 9, dans laquelle l'interface nasale (100, 1100, 1200, 1300, 1400, 1500, 1600, 1700, 1800, 1900, 2000, 2500, 2700, 2800) est conçue pour administrer une pression d'écoulement de gaz à travers la première sortie (111a, 1111a, 1211a, 1411a, 1511a, 1611a, 1711a, 1811a, 2511a, 2711a) supérieure à une pression d'écoulement de gaz à travers la seconde sortie (112a, 1112a, 1212a, 1412a, 1512a, 1612a, 1712a, 1812a, 2512a, 2712a, 2812a) pendant une phase d'inhalation d'un cycle respiratoire.

11. Interface nasale (100, 1100, 1200, 1300, 1400, 1500, 1600, 1700, 1800, 1900, 2000, 2500, 2700, 2800) selon l'une quelconque des revendications 1 à 10, comprenant en outre un raccord ou un coude (1222, 2722) pour raccorder un conduit respiratoire (300) à l'interface nasale (100, 1100, 1200, 1300, 1400, 1500, 1600, 1700, 1800, 1900, 2000, 2500, 2700, 2800).

12. Interface nasale (100, 1100, 1200, 1300, 1400, 1500, 1600, 1700, 1800, 1900, 2000, 2500, 2700, 2800) selon l'une quelconque des revendications 1 à 11, dans laquelle l'interface nasale (100, 1100, 1200, 1300, 1400, 1500, 1600, 1700, 1800, 1900, 2000, 2500, 2700, 2800) est conçue pour diriger plus de gaz entrants vers le premier flux d'écoulement de gaz (F1) que vers le second flux d'écoulement de gaz (F2) pendant une phase d'inhalation du cycle respiratoire, et/ou dans laquelle le corps d'interface (110, 1110, 1210, 1310, 1510, 1610, 1910, 2710, 2810) est un coussinet nasal (1110, 1210, 1910, 2110, 2510, 2610, 2710) et/ou dans laquelle l'interface nasale (100, 1100, 1200, 1300, 1400, 1500, 1600, 1700, 1800, 1900, 2000, 2500, 2700, 2800) est conçue pour administrer simultanément les gaz respiratoires de l'entrée de gaz (121, 1121, 1221, 1321, 1421, 1521, 1621, 1721, 1821, 1921, 2721, 2821) à travers le corps d'interface (110, 1110, 1210, 1310, 1510, 1610, 1910, 2710, 2810) à la fois vers la première narine et la seconde narine du patient lors de l'utilisation.

13. Interface nasale selon l'une quelconque des revendications 1 à 12, dans laquelle l'entrée de gaz (121, 1121, 1221, 1321, 1421, 1521, 1621, 1721, 1821, 1921, 2721, 2821) est positionnée sur un côté d'un collecteur de gaz (120, 1120, 1220, 1320, 1520, 1620, 1820, 2220, 2320, 2420, 2720, 2820) de l'interface nasale (100, 1100, 1200, 1300, 1400, 1500, 1600, 1700, 1800, 1900, 2000, 2500, 2700, 2800), et l'interface nasale (100, 1100, 1200, 1300, 1400, 1500, 1600, 1700, 1800, 1900, 2000, 2500, 2700, 2800) comprend un conduit respiratoire (300) raccordé à l'entrée de gaz (121, 1121, 1221, 1321, 1421, 1521, 1621, 1721, 1821, 1921, 2721, 2821) et le conduit respiratoire (300) s'étendant latéralement depuis le côté du collecteur de gaz (120, 1120, 1220, 1320, 1520, 1620, 1820, 2220, 2320, 2420, 2720, 2820).

14. Interface nasale (100, 1100, 1200, 1300, 1400, 1500, 1600, 1700, 1800, 1900, 2000, 2500, 2700, 2800) selon l'une quelconque des revendications 1 à 13, dans laquelle le collecteur de gaz (120) comprend des orifices latéraux (121, 122) sur les deux côtés gauche et droit pour recevoir sélectivement le conduit respiratoire (300).
